(19) Europäisches Patentamt
European Patent Office
Office européen des brevets

(11) EP 1 707 632 A1

(12) EUROPÄISCHE PATENTANMELDUNG

(43) Veröffentlichungstag:
**04.10.2006 Patentblatt 2006/40**

(21) Anmeldenummer: **05090085.1**

(22) Anmeldetag: **01.04.2005**

(51) Int Cl.:
***C12N 15/82*** *(2006.01)* ***C12N 9/10*** *(2006.01)*
***C08B 30/00*** *(2006.01)*

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

(71) Anmelder: **Bayer CropScience GmbH**
**65929 Frankfurt/Main (DE)**

(72) Erfinder:
- **Soyka, Stephan**
  **9051 Sint-Denijs-Western (BE)**
- **Pilling, Jens**
  **14149 Berlin (DE)**
- **Frohberg, Claus**
  **14532 Kleinmachnow (DE)**

(74) Vertreter: **Kossmann, Jochen**
**Bayer BioScience GmbH**
**Hermannswerder 20a**
**14473 Potsdam (DE)**

Bemerkungen:
The sequence listing, which is published as annex to the application documents, was filed after the date of filing. The applicant has declared that it does not include matter which goes beyond the content of the application as filed.

(54) **Phosphorylierte waxy-Kartoffelstärke**

(57) Die Erfindung betrifft Kartoffelstärken mit einem Amylosegehalt von weniger als 10 Gew.-%, einem Phosphatgehalt in C6-Position zwischen 35 und 100 nmol Phosphat pro Milligramm Stärke und einem erhöhten Gehalt an Seitenketten mit einem DP von 12 bis 19 im Vergleich zu Kartoffelstärke aus entsprechenden Kartoffel-Wildtyp-Pflanzen.

EP 1 707 632 A1

## Beschreibung

Die vorliegende Erfindung betrifft Kartoffelstärken mit einem Amylosegehalt von weniger als 10 Gew.-%, einem Phosphatgehalt in C6-Position zwischen 35 und 100 nmol Phosphat pro Milligramm Stärke und einem erhöhten Gehalt an Seitenketten mit einem DP von 12 bis 19 im Vergleich zu Kartoffelstärke aus entsprechenden Kartoffel-Wildtyp-Pflanzen.

Im Hinblick auf die zunehmende Bedeutung, die pflanzlichen Inhaltsstoffen als emeuerbaren Rohstoffquellen zur Zeit beigemessen wird, ist es eine der Aufgaben der biotechnologischen Forschung, sich um eine Anpassung dieser pflanzlichen Rohstoffe an die Anforderungen der verarbeitenden Industrie zu bemühen. Um eine Anwendung von nachwachsenden Rohstoffen in möglichst vielen Einsatzgebieten zu ermöglichen, ist es darüber hinaus erforderlich, eine große Stoffvielfalt zu erreichen.

Das Polysaccharid Stärke ist aus chemisch einheitlichen Grundbausteinen, den Glucosemolekülen, aufgebaut, stellt jedoch ein komplexes Gemisch unterschiedlicher Molekülformen dar, die Unterschiede hinsichtlich des Polymerisations- und des Verzweigungsgrades aufweisen und sich somit in ihren physikalisch-chemischen Eigenschaften stark voneinander unterscheiden. Man differenziert zwischen Amylosestärke, einem im Wesentlichen unverzweigten Polymer aus alpha-1,4-glycosidisch verknüpften Glucoseeinheiten, und der Amylopektinstärke, einem verzweigten Polymer, bei dem die Verzweigungen durch das Auftreten zusätzlicher alpha-1,6-glycosidischer Verknüpfungen zustande kommen. Ein weiterer wesentlicher Unterschied zwischen Amylose und Amylopektin liegt im Molekulargewicht. Während Amylose, je nach Herkunft der Stärke, ein Molekulargewicht von $5x10^5$ - $10^6$ Da besitzt, liegt das Molekulargewicht des Amylopektins zwischen $10^7$ und $10^8$ Da. Die beiden Makromoleküle können durch ihr Molekulargewicht und ihre unterschiedlichen physikochemischen Eigenschaften differenziert werden, was am einfachsten durch ihre unterschiedlichen Jodbindungseigenschaften sichtbar gemacht werden kann.

Amylose wurde lange als lineares Polymer, bestehend aus alpha-1,4-glycosidisch verknüpften alpha-D-Glucose-Monomeren, angesehen. In neueren Studien wurde jedoch die Anwesenheit eines geringen Anteils alpha-1,6-glycosidischer Verzweigungspunkte (ca. 0,1%) nachgewiesen (Hizukuri und Takagi, Carbohydr. Res. 134, (1984), 1-10; Takeda et al., Carbohydr. Res. 132, (1984), 83-92).

Amylopektin stellt ein komplexes Gemisch aus unterschiedlich verzweigten Glukoseketten dar. Im Gegensatz zur Amylose ist das Amylopektin stärker verzweigt. Mit der aus α-1,4-glycosidisch verknüpften α-D-Glukosemonomeren bestehenden Hauptkette sind Seitenketten über α-1,6-glykosidische Bindungen verknüpft. Nach Lehrbuchangaben (Voet and Voet, Biochemistry, John Wiley & Sons, 1990) treten die α-1,6-Verzweigungen durchschnittlich alle 24 bis 30 Glukosereste auf. Dies entspricht einem Verzweigungsgrad von ca. 3% - 4%. Die Angaben zum Verzweigungsgrad sind variabel und abhängig von der Herkunft (z.B. Pflanzenspezies, Pflanzensorte usw.) der jeweiligen Stärke. In typischen für die industrielle Stärkeproduktion verwendeten Pflanzen, wie z.B. Mais, Weizen oder Kartoffel, besteht die synthetisierte Stärke zu ca. 20% - 30% aus Amylose-Stärke und zu ca. 70% - 80% aus Amylopektin-Stärke.

Die funktionellen Eigenschaften, wie z.B. die Löslichkeit, das Retrogradationsverhalten, das Wasserbindevermögen, die Filmbildungseigenschaften, die Viskosität, die Verkleisterungseigenschaften, die Gefrier-Tau-Stabilität, die Säurestabilität, die Gelfestigkeit, die Stärkekomgröße von Stärken werden u.a. durch das Amylose/Amylopektin-Verhältnis, das Molekulargewicht, das Muster der Seitenkettenverteilung des Amylopektins, den Gehalt an Ionen, den Lipid- und Proteingehalt, die mittlere Stärkekomgröße die Stärkekornmorphologie etc. beeinflusst. Die funktionellen Eigenschaften von Stärke werden auch vom Phosphatgehalt der Stärke beeinflusst. Dabei ist zwischen Phosphat, welches in Form von Monoestern kovalent an die Glucosemoleküle der Stärke gebunden ist (im folgenden auch als Stärkephosphat bezeichnet) und Phosphat in Form von mit der Stärke assoziierten Phospholipiden zu unterscheiden.

Der Gehalt an Stärkephosphat variiert je nach Pflanzenspezies. So synthetisieren z.B. bestimmte Maismutanten eine Stärke mit erhöhtem Gehalt an Stärkephosphat (waxy-Mais 0,002% und Hoch-Amylose-Mais 0,013%), während herkömmliche Mais-Sorten nur Spuren von Stärkephosphat aufweisen. Ebenfalls geringe Mengen an Stärkephosphat findet man in Weizen (0,001 %) während in Hafer und Sorghum kein Stärkephosphat nachgewiesen werden konnte. Größere Mengen von Stärkephosphat wurden bisher in Knollen- oder Wurzelspeicherstärke wie z.B. Tapioca (0,008%), Süßkartoffel (0,011%), Pfeilwurz (0,021%) oder Kartoffel (0,089%) nachgewiesen. Die im Vorangegangenen zitierten prozentualen Werte für den Stärkephosphatgehalt beziehen sich jeweils auf das Trockengewicht der Stärke und sind von Jane et al. (1996, Cereal Foods World 41 (11), 827-832) ermittelt worden. Stärkephosphat kann in Form von Monoestern an der C-2, C-3 oder C-6 Position der polymerisierten Glucosemonomere vorliegen (Takeda und Hizukuri, 1971, Starch/Stärke 23, 267-272). Die kovalent gebundenen Stärkephosphatgruppen finden sich im allgemeinen zu etwa 30% bis 40% in C-3-Position und zu etwa 60% bis 70% in C-6-Position der Glucosemonomere (Blennow et al., 2000, Int. J. of Biological Macromolecules 27, 211-218).

Kartoffel-Amylopektinstärken, d.h. Stärken mit einem Amylopektinanteil von mehr als 90% und einem Amyloseanteil von weniger als 10%, sind erhältlich aus Kartoffelpflanzen, bei welchen die Aktivität der Stärkekorn-gebundenen Stärkesynthase GBSSI ("Granule-Bound Starch Synthase I") reduziert ist (Shure et al., 1983, Cell 35, 225-233; Hovenkamp-Hermelink et al., 1987, Theoretical and Applied Genetics 75, 217-221; Visser et al., 1991, Mol. Gen. Genet.

225, 289-296). Die GBSSI ist an der Bildung von Amylose beteiligt. Die Hemmung der GBSSI-Aktivität führt zu einer Synthese von Stärke, die fast ausschließlich aus Amylopektin besteht. Das entsprechende GBSSI-Gen aus Mais ist unter der Bezeichnung "waxy" bekannt. Die Amylopektinstärken werden auch als waxy-Stärken bezeichnet.

Weiterhin sind Pflanzen beschrieben, bei welchen die Aktivität der löslichen Stärkesynthase III (SSIII) reduziert ist (Abel et al., 1996, The Plant Journal 10(6), 981-991; Lloyd et al., 1999, Biochemical Journal 338, 515-521). Stärke aus solchen Pflanzen weist, verglichen mit aus entsprechenden Wildtyp-Pflanzen isolierter Stärke, eine relative Verschiebung der Seitenketten des Amylopektins von längeren Ketten zu kurzen Ketten (Lloyd et al., 1999, Biochemical Journal 338, 515-521), einen erhöhten Phosphatgehalt, keine Veränderung des Amylosegehaltes (Abel et al., 1996, The Plant Journal 10(6), 9891-9991) und eine erniedrigte Endviskosität in der RVA-Analyse (Abel, 1995, Dissertation, Freie Universität Berlin) auf.

Weiterhin sind Pflanzen beschrieben, bei welchen die Aktivität des Verzweigungsenzyms I (= Branching Enzyme I = BEI) reduziert ist (Kossmann et al., 1991, Mol. Gen. Genet. 230, 39-44; Safford et al., 1998, Carbohydrate Polymers 35, 155-168; WO 92/14827). Safford et al. (1998, supra) beschreiben, dass entsprechende Kartoffeln eine Stärke mit einem leicht verändertem Amylose/Amylopektin-Verhältnis herstellen. Auch der Verzweigungsgrad des Amylopektins unterscheidet sich nicht signifikant von demjenigen einer aus Wildtyp-Kartoffeln isolierten Stärke. Der Stärke-gebundene Phosphatgehalt ist jedoch leicht erhöht.

In WO 01/19975 sind Pflanzen beschrieben, bei welchen sowohl die GBSSI- als auch die SSII-und/oder SSIII-Aktivität reduziert ist. Stärke aus Kartoffeln mit verminderten GBSSI-, SSII- <u>und</u> SSIII-Aktivitäten weist im Vergleich zu Stärke aus Wildtyp-Kartoffeln einen niedrigeren Amylosegehalt, veränderte Quellfähigkeits- und Verkleisterungseigenschaften, und eine erhöhte Gefrier/Tau-Stabilität auf.

In WO 01/12782 sind Pflanzen beschrieben, bei welchen sowohl die GBSSI- als auch die BEI-Aktivität reduziert ist. Stärke aus solchen Kartoffel-Pflanzen weist im Vergleich zu Kartoffelstärke aus Wildtyp-Pflanzen einen verminderten Amylosegehalt und im Vergleich zu Kartoffelstärke aus Pflanzen des waxy-Phänotyps einen erhöhten Phosphatgehalt und/oder eine verringerte Verkleisterungstemperatur in der RVA-Analyse auf.

In WO 00/08184 sind unter anderem Pflanzen beschrieben, bei welchen sowohl die SSIII- als auch die BEI-Aktivität reduziert ist. Stärke aus solchen Pflanzen weist im Vergleich zu Stärke aus Wildtyp-Pflanzen einen deutlich erhöhten Phosphatgehalt auf.

Der vorliegenden Erfindung liegt die Aufgabe zu Grunde, Kartoffel-Amylopektin-Stärken mit neuen Eigenschaften, neue Pflanzenzellen und/oder Pflanzen, die solche Stärken herstellen, sowie Mittel und Verfahren zur Erzeugung besagter Pflanzenzellen und/oder Pflanzen zur Verfügung zu stellen.

Diese Aufgabe wird durch die Bereitstellung der in den Patentansprüchen bezeichneten Ausführungsformen gelöst.

Gegenstand der vorliegenden Erfindung sind Kartoffelstärken, dadurch gekennzeichnet, dass sie einen Amylosegehalt gemessen nach der Methode von Hovenkamp-Hermelink et al. (1988, Potato Research 31, 241-246) von weniger als 10 Gew.-% und einen Phosphatgehalt in C6-Position zwischen 35 und 100 nmol Phosphat pro mg Stärke (Trockengewicht) und einen erhöhten Gehalt an Seitenketten mit einem DP von 12 bis 19 im Vergleich zu Kartoffelstärke aus entsprechenden Kartoffel-Wildtyp-Pflanzen aufweisen.

Ein weiterer Gegenstand der vorliegenden Erfindung betrifft Kartoffelstärken, dadurch gekennzeichnet, dass sie einen Amylosegehalt gemessen nach der Methode ("Allgemeine Methoden") von Hovenkamp-Hermelink et al. (1988, Potato Research 31, 241-246) von weniger als 10 Gew.-%, einen Phosphatgehalt in C6-Position zwischen 35 und 100 nmol Phosphat pro mg Stärke (Trockengewicht) und ein Verhältnis von Gesamtphosphatgehalt zu Phosphatgehalt in C6-Position von 1.10-1.60 aufweisen.

Der Amylosegehalt wird im Zusammenhang mit der vorliegenden Erfindung nach der weiter unten für Kartoffelstärke beschriebenen Methode von Hovenkamp-Hermelink et al. (1988, Potato Research 31, 241-246) bestimmt. Diese Methode ist auch auf isolierte Stärken anderer Pflanzenspezies anwendbar. Verfahren zur Isolierung von Stärken sind dem Fachmann bekannt und werden weiter unten im Abschnitt "Allgemeine Methoden" im Detail beschrieben.

Unter dem Begriff "Phosphatgehalt in C6-Position" ist im Zusammenhang mit der vorliegenden Erfindung der Gehalt an Phosphatgruppen zu verstehen, die an Kohlenstoffatomposition "6" der Glukosemonomere der Stärke kovalent gebunden sind. Grundsätzlich können in der Stärke in vivo die Positionen C2, C3 und C6 der Glukoseeinheiten phosphoryliert sein. Die Bestimmung des Phosphatgehaltes in C6-Position (= C6-P-Gehalt) erfolgt im Zusammenhang mit der vorliegenden Erfindung über eine Glukose-6-phosphat-Bestimmung mittels des weiter unten ("Allgemeine Methoden: Bestimmung des Phosphatgehaltes in C6-Position") beschriebenen optisch-enzymatischen Tests.

Unter dem Begriff "Gesamtphosphatgehalt" soll im Zusammenhang mit der vorliegenden Erfindung die Menge an Stärkephosphat verstanden werden, die insgesamt an Glucosemoleküle der Stärke kovalent gebunden ist. Die Bestimmung des Gesamtphosphatgehaltes erfolgt im Zusammenhang mit der vorliegenden Erfindung über die weiter unten beschriebene Methode ("Allgemeine Methoden: Bestimmung des Gesamtphosphatgehaltes").

Der Begriff "erhöhter Gehalt an Seitenketten mit einem DP von 12 bis 19" bedeutet im Zusammenhang mit der vorliegenden Erfindung eine Erhöhung des Anteils der Summe an Seitenketten der Stärke mit einem DP (= Degree of Polymerisation) von 12 bis 19 auf 125%-200%, bevorzugt auf 130%-180% und besonders bevorzugt auf 140%-160% im Vergleich zum Anteil an Seitenketten mit einem DP von 12 bis 19 von Kartoffelstärke aus entsprechenden Kartoffel-Wildtyp-Pflanzen (100%).

Die Bestimmung der Seitenkettenverteilung der Stärke erfolgt im Zusammenhang mit der vorliegenden Erfindung wie weiter unten im Abschnitt "Allgemeine Methoden: Analyse der Seitenkettenverteilung von Gesamtstärke mittels Gelpermeationschromatographie" beschrieben.

Der Begriff "Kartoffel-Wildtyp-Pflanzenzelle" bedeutet im Zusammenhang mit der vorliegenden Erfindung, dass es sich um Kartoffel-Pflanzenzellen handelt, die als Ausgangsmaterial für die Herstellung der erfindungsgemäßen Pflanzenzellen dienten, d.h. deren genetische Information, abgesehen von der eingeführten genetischen Modifikation, derjenigen einer erfindungsgemäßen Pflanzenzelle entspricht.

Der Begriff "Kartoffel-Wildtyp-Pflanze" bedeutet im Zusammenhang mit der vorliegenden Erfindung, dass es sich um Pflanzen handelt, die als Ausgangsmaterial für die Herstellung der weiter unten beschriebenen erfindungsgemäßen Pflanzen dienten, d.h. deren genetische Information, abgesehen von der eingeführten genetischen Modifikation, der einer erfindungsgemäßen Pflanze entspricht.

Der Begriff "entsprechend" bedeutet im Zusammenhang mit der vorliegenden Erfindung, dass beim Vergleich von mehreren Gegenständen die betreffenden Gegenstände, die miteinander verglichen werden, unter gleichen Bedingungen gehalten wurden. Im Zusammenhang mit der vorliegenden Erfindung bedeutet der Begriff "entsprechend" in bezug auf Wildtyp-Pflanzenzellen oder Wildtyp-Pflanzen insbesondere, dass die Pflanzenzellen oder Pflanzen, die miteinander verglichen werden, unter gleichen Kulturbedingungen aufgezogen wurden, und dass sie das gleiche (Kultur-) Alter aufweisen.

In einer bevorzugten Ausführungsform der Erfindung weisen die erfindungsgemäßen Kartoffelstärken einen Amylosegehalt gemessen nach der Methode von Hovenkamp-Hermelink et al. (1988, Potato Research 31, 241-246) von weniger als 5 Gew.-%, besonders bevorzugt von weniger als 3 Gew.-% auf.

In einer weiteren bevorzugten Ausführungsform der Erfindung weist die von den erfindungsgemäße Kartoffelstärke einen Phosphatgehalt in C6-Position von 40 - 85 nmol C6-P pro mg Stärke, besonders bevorzugt von 45 - 70 nmol C6-P pro mg Stärke, ganz besonders bevorzugt von 50 - 65 nmol C6-P pro mg Stärke auf.

In einer weiteren bevorzugten Ausführungsform der Erfindung weist die erfindungsgemäße Kartoffelstärke einen erhöhten Phosphatgehalt im Vergleich zu Kartoffelstärke aus entsprechenden Kartoffel-Wildtyp-Pflanzen auf. Der Begriff "erhöhter Phosphatgehalt" bedeutet im Zusammenhang mit der vorliegenden Erfindung, dass der Phosphatgehalt in C-6-Position der erfindungsgemäßen Stärke erhöht ist, insbesondere um 415% - 520%, bevorzugt um 430% - 500% und besonders bevorzugt um 440% - 490% erhöht im Vergleich zu Stärke aus entsprechenden Wildtyp-Pflanzenzellen bzw. -Pflanzen.

In einer weiteren Ausführungsform der Erfindung weist die erfindungsgemäße Kartoffelstärke einen "erhöhten Gehalt an Seitenketten mit einem DP<12" auf. Dies bedeutet im Zusammenhang mit der vorliegenden Erfindung eine Erhöhung des Anteils der Summe an Seitenketten der Stärke mit einem DP (= Degree of Polymerisation) kleiner 12 auf 130%-170%, bevorzugt auf 140%-160% und besonders bevorzugt auf 145%-155% im Vergleich zum Anteil an Seitenketten mit einem DP kleiner 12 von Kartoffelstärke aus entsprechenden Kartoffel-Wildtyp-Pflanzen (100%).

In einer weiteren Ausführungsform der Erfindung weist die erfindungsgemäße Kartoffelstärke einen "erhöhten Gehalt an Seitenketten mit einem DP von 20-25" auf. Dies bedeutet im Zusammenhang mit der vorliegenden Erfindung eine Erhöhung des Anteils der Summe an Seitenketten der Stärke mit einem DP (= Degree of Polymerisation) von 20-25 auf 132%-160%, bevorzugt auf 136%-150% und besonders bevorzugt auf 139%-148% im Vergleich zum Anteil an Seitenketten mit einem DP von 20-25 von Kartoffelstärke aus entsprechenden Kartoffel-Wildtyp-Pflanzen (100%).

In einer weiteren Ausführungsform der Erfindung weist die erfindungsgemäße Kartoffelstärke einen "verringerten Gehalt an Seitenketten mit einem DP von 63-123" auf. Dies bedeutet im Zusammenhang mit der vorliegenden Erfindung eine Verringerung des Anteils der Summe an Seitenketten der Stärke mit einem DP (= Degree of Polymerisation) von 63-123 auf 50%-95%, bevorzugt auf 65%-90% und besonders bevorzugt auf 73%-85% im Vergleich zum Anteil an Seitenketten mit einem DP von 63-123 von Kartoffelstärke aus entsprechenden Kartoffel-Wildtyp-Pflanzen (100%).

In einer weiteren Ausführungsform der Erfindung weisen die erfindungsgemäßen Kartoffelstärken einen "verringerten Gehalt an Seitenketten mit einem DP von >123" auf. Dies bedeutet im Zusammenhang mit der vorliegenden Erfindung eine Verringerung des Anteils der Summe an Seitenketten der Stärke mit einem DP (= Degree of Polymerisation) größer als 123 auf 0,1% -3,8%, bevorzugt auf 0,3%-3,0 % und besonders bevorzugt auf 0,5%-2,5% im Vergleich zum Anteil an Seitenketten mit einem DP größer als 123 von Kartoffelstärke aus entsprechenden Kartoffel-Wildtyp-Pflanzen (100%).

In einer weiteren bevorzugten Ausführungsform der Erfindung weisen die erfindungsgemäßen Kartoffelstärken ein Verhältnis von Gesamtphosphatgehalt zu Phosphatgehalt in C6-Position von 1.20-1.50, besonders bevorzugt von 1,30-1,40 auf.

In einer weiteren Ausführungsform der Erfindung weisen die erfindungsgemäßen Kartoffelstärken eine hohe Gefrier/Tau-Stabilität auf.

Der Begriff "hohe Gefrier/Tau-Stabilität" bedeutet im Zusammenhang mit der vorliegenden Erfindung eine Gefrier/Tau-Stabilität von mindestens 60%, insbesondere von mindestens 70%, bevorzugt von mindestens 80% und besonders bevorzugt von mindestens 95%. Die Bestimmung der Gefrier/Tau-Stabilität erfolgt im Zusammenhang mit der vorliegenden Erfindung nach der weiter unten beschriebenen Methode ("Allgemeine Methoden").

In einer weiteren Ausführungsform der Erfindung weisen die erfindungsgemäßen Kartoffelstärken eine hohe Hitzestabilität auf.

Der Begriff "hohe Hitzestabilität" bedeutet im Zusammenhang mit der vorliegenden Erfindung eine Hitzestabilität von mindestens 30%, insbesondere von mindestens 40% und bevorzugt von mindestens 50%. Die Bestimmung der Hitzestabilität erfolgt im Zusammenhang mit der vorliegenden Erfindung nach der weiter unten beschriebenen Methode ("Allgemeine Methoden").

In einer weiteren Ausführungsform der Erfindung weisen die erfindungsgemäßen Kartoffelstärken ein verändertes Viskositätsverhalten (z.B. Verkleisterungstemperatur, Endviskosität) auf im Vergleich zu Kartoffelstärke aus entsprechenden Kartoffel-Wildtyp-Pflanzen. Die Bestimmung der Viskositätseigenschaften erfolgt im Zusammenhang mit der vorliegenden Erfindung nach der weiter unten beschriebenen RVA-Methode ("Allgemeine Methoden").

In einer weiteren Ausführungsform der Erfindung weisen die erfindungsgemäßen Kartoffelstärken eine veränderte DSC-Peaktemperatur auf im Vergleich zu Kartoffelstärke aus entsprechenden Kartoffel-Wildtyp-Pflanzen. Die Bestimmung der DSC Peaktemperatur erfolgt im Zusammenhang mit der vorliegenden Erfindung nach der weiter unten beschriebenen Methode ("Allgemeine Methoden").

In einer weiteren Ausführungsform der Erfindung weisen die erfindungsgemäßen Kartoffelstärken eine verringerte Gelfestigkeit auf im Vergleich zu Kartoffelstärke aus entsprechenden Kartoffel-Wildtyp-Pflanzen. Die Bestimmung der Gelfestigkeit erfolgt im Zusammenhang mit der vorliegenden Erfindung nach der weiter unten beschriebenen Methode ("Allgemeine Methoden").

Bei den erfindungsgemäßen Kartoffelstärken handelt es sich vorzugsweise um native Kartoffelstärken. Der Begriff "native Stärke" bedeutet im Zusammenhang mit der vorliegenden Erfindung, dass die Stärke nach dem Fachmann bekannten Methoden aus Pflanzen oder stärkespeichemden Teilen von Pflanzen extrahiert wird, ohne dass die extrahierte Stärke nach der Extraktion chemisch modifiziert wird.

Verfahren zur Isolierung von Stärke aus Pflanzen oder von Stärke speichernden Teilen von Pflanzen sind dem Fachmann bekannt. Weiterhin sind Verfahren zur Extraktion der Stärke aus verschiedenen Stärke speichernde Pflanzen beschrieben, z. B. in Starch: Chemistry and Technology (Hrsg.: Whistler, BeMiller und Paschall (1994), 2. Ausgabe, Academic Press Inc. London Ltd; ISBN 0-12-746270-8; siehe z. B. Kapitel XII, Seite 412-468: Mais und Sorghum Stärken: Herstellung; von Watson; Kapitel XIII, Seiten 469-479: Tapioca, Arrowroot und Sago Stärken: Herstellung; von Corbishley und Miller; Kapitel XIV, Seiten 479-490: Kartoffelstärke: Herstellung und Verwendungen; von Mitch; Kapitel XV, Seiten 491 bis 506: Weizenstärke: Herstellung, Modifizierung und Verwendungen; von Knight und Oson; und Kapitel XVI, Seiten 507 bis 528: Reisstärke: Herstellung und Verwendungen; von Rohmer und Klem; Maisstärke: Eckhoff et al., Cereal Chem. 73 (1996), 54-57, die Extraktion von Maisstärke im industriellen Maßstab wird in der Regel durch das so genannte "wet milling" erreicht.

Gegenstand der vorliegenden Erfindung sind auch erfindungsgemäße Kartoffelstärken mit einer oder mehreren der vorstehend beschriebenen Eigenschaften. D.h., diese Erfindungsbeschreibung offenbart jegliche Kombination der folgenden Stärkeeigenschaften: Amylosegehalt bzw. Amylose/Amylopektin-Verhältnis, Phosphatgehalt, Seitenkettenverteilung, Gefrier/Tau-Stabilität und Hitzestabilität. Als offenbart anzusehen, sind alle Kombinationen aus zwei, drei, vier, fiinf, sechs, sieben, acht, neun und allen Eigenschaften.

Die erfindungsgemäßen Stärken weisen Eigenschaften auf, die sie besonders geeignet scheinen lassen für den Einsatz bei Prozessen, bei weichen ein starkes Quellvermögen, eine hohe Gefrier/Taustabilität und/oder eine hohe Ladungsdichte von Vorteil sind. Diese Anforderungen treffen beispielsweise auf Dickungsmittel in der Lebensmittelindustrie zu, vor allem, wenn diese zur Lagerung oder der Verarbeitung eingefroren werden und/oder eine besonders hohe Dickungsleistung wünschenswert ist.

Die erfindungsgemäßen Stärken sind aufgrund ihrer ungewöhnlich hohen Ladungsdichte, die auf die kovalent gebundenen Phosphatgruppen zurückzuführen ist, und ihrer niedrigviskosen Dickung besonders gut zum Einsatz in der Papierindustrie geeignet. Die hohe Ladungsdichte ist von Vorteil, da sie die Erzeugung von häufig eingesetzten amphoterischen Stärken in einer nur einstufigen Derivatisierungsreaktion möglich macht und auf zusätzliche Derivatisierungsreaktionen zum Einbringen negativer Ladungen in die Stärken weitgehend verzichtet werden kann.

Die erfindungsgemäßen Kartoffelstärken, vorzugsweise native Kartoffelstärken, können mit Hilfe von Standardverfahren, die dem Fachmann bekannt sind, nach der Extraktion aus den Kartoffelknollen chemisch und/oder physikalisch modifiziert werden.

Dem Fachmann ist bekannt, dass die Eigenschaften von nativer Kartoffelstärke durch z.B. physikalische (z.B. thermisch, mechanisch) und/oder chemische Derivatisierung und/oder durch enzymatische, säurehydrolytische oder thermische Zersetzung erhaltene Abbauprodukte der Stärke (z.B. Dextrine) verändert werden können. Die hierbei erhaltenen Stärken, die im Zusammenhang mit der vorliegenden Erfindung als "derivatisierte Kartoffelstärken" bezeichnet werden sollen, sind für verschiedene Anwendungen besonders geeignet. Die erfindungsgemäßen nativen Kartoffelstärken sind als Ausgangssubstanz besser geeignet zur Herstellung der derivatisierten Kartoffelstärken als herkömmliche Kartoffelstärken (aus Kartoffel-Wildtyp-Pflanzen), weil sie beispielsweise durch den höheren Gehalt an kovalent gebundenem Stärkephosphat einen höheren Anteil an reaktiven funktionalen Gruppen aufweist, stärker hydrophil ist und chemischen Agenzien besser zugänglich ist.

Gegenstand der vorliegenden Erfindung sind daher auch solche derivatisierten Kartoffelstärken enthaltend die erfindungsgemäßen, vorzugsweise nativen, Kartoffelstärken, sowie Verfahren zur Herstellung solcher derivatisierten Stärke, worin erfindungsgemäße, vorzugsweise native, Kartoffelstärke nachträglich, d.h. nach Extraktion aus der Kartoffelknolle, vorzugsweise in vitro, chemisch und/oder physikalisch modifiziert wird.

Bei der erfindungsgemäßen derivatisierten Stärke handelt es sich insbesondere um mit Hitze behandelte Stärke. Vorzugsweise betrifft die vorliegende Erfindung säuremodifizierte Stärke, die vorzugsweise im wäßrigen System bei Temperaturen von bis zu 50°C mit Säure, vorzugsweise mit Salzsäure (in einer Konzentration von bis zu IM) behandelt wurde.

In einer weiteren Ausführungsform betrifft die vorliegende Erfindung derivatisierte Stärken, die durch eine Temperaturbehandlung der erfindungsgemäßen, vorzugsweise nativen, Kartoffelstärke im trockenen System, vorzugsweise bei Temperaturen von vorzugsweise 120°C bis 140°C gewonnen wurden.

In einer weiteren Ausführungsform handelt es sich bei den derivatisierten Stärken um Stärkeether, insbeondere um Stärke-Alkylether, O-Allylether, Hydroxylalkylether, O-Carboxylmethylether, stickstoffhaltige Stärkeether, phosphathaltige Stärkeether oder schwefelhaltige Stärkeether.

In einer weiteren Ausführungsform handelt es sich bei den derivatisierten Stärken um vernetzte Stärken.

In einer weiteren Ausführungsform handelt es sich bei den derivatisierten Stärken um Stärke-Pfropf-Polymerisate.

In einer weiteren Ausführungsform handelt es sich bei den derivatisierten Stärken um oxidierte Stärken.

In einer weiteren Ausführungsform handelt es sich bei den derivatisierten Stärken um Stärkeester, insbesondere um Stärkeester, die unter Verwendung von organischen Säuren in die Stärke eingeführt wurden. Besonders bevorzugt handelt es sich um Phosphat-, Nitrat-, Sulfat-, Xanthat-, Acetat- oder Citratstärken.

Die erfindungsgemäßen derivatisierten Stärken eignen sich für verschiedene Verwendungen in der Pharmaindustrie, im Nahrungsmittel- und/oder Nicht-Nahrungsmittelbereich. Verfahren zur Herstellung von erfindungsgemäßen derivatisierten Stärken sind dem Fachmann bekannt und in der allgemeinen Literatur ausreichend beschrieben. Eine Übersicht zur Herstellung von derivatisierten Stärken findet sich z.B. bei Orthoefer (in: Corn, Chemistry and Technology, 1987, eds. Watson und Ramstad, Chapter 16, 479-499).

Derivatisierte Stärke, erhältlich nach dem erfindungsgemäßen Verfahren zur Herstellung einer derivatisierten Stärke ist ebenfalls Gegenstand der vorliegenden Erfindung.

Ferner ist die Verwendung erfindungsgemäßer, vorzugsweise nativer, Kartoffelstärken zur Herstellung von derivatisierter Kartoffelstärke Gegenstand der vorliegenden Erfindung.

Die erfindungsgemäßen Kartoffelstärken eignen sich in nativer oder derivatisierter Form für verschiedene Verwendungen im Nahrungsmittel- oder Nicht-Nahrungsmittelbereich.

Grundsätzlich lässt sich die Einsatzmöglichkeit der Stärke in zwei große Bereiche unterteilen. Der eine Bereich umfasst die Hydrolyseprodukte der Stärke, hauptsächlich Glukose und Glukanbausteine, die über enzymatische oder chemische Verfahren erhalten werden. Sie dienen als Ausgangsstoffe für weitere chemische Modifikationen und Prozesse, wie Fermentation. Für eine Reduktion der Kosten kann hierbei die Einfachheit und kostengünstige Ausführung eines Hydrolyseverfahrens von Bedeutung sein. Gegenwärtig verläuft es im Wesentlichen enzymatisch unter Verwendung von Amyloglucosidase. Vorstellbar wäre eine Kosteneinsparung durch einen geringeren Einsatz von Enzymen. Eine Strukturveränderung der Stärke, z.B. Oberflächenvergrößerung des Korns, leichtere Verdaulichkeit durch geringeren Verzweigungsgrad oder eine sterische Struktur, die die Zugänglichkeit für die eingesetzten Enzyme begrenzt, könnte dies bewirken.

Der andere Bereich, in dem die Stärke wegen ihrer polymeren Struktur als sogenannte native Stärke verwendet wird, gliedert sich in zwei weitere Einsatzgebiete:

1. Nahrungsmittelindustrie

Stärke ist ein klassischer Zusatzstoff für viele Nahrungsmittel, bei denen sie im Wesentlichen die Funktion des Bindens von wässrigen Zusatzstoffen übernimmt bzw. eine Erhöhung der Viskosität oder aber eine erhöhte Gelbildung hervorruft. Wichtige Eigenschaftsmerkmale sind das Fließ- und Sorptionsverhalten, die Quell- und Verkleisterungstemperatur, die Viskosität und Dickungsleistung, die Löslichkeit der Stärke, die Transparenz und Kleisterstruktur, die Hitze-, Scher- und Säurestabilität, die Neigung zur Retrogradation, die Fähigkeit zur Filmbildung, die Gefrier/Tau-Stabilität, die Verdaulichkeit sowie die Fähigkeit zur Komplexbildung mit z.B. anorganischen oder organischen Ionen.

2. Nicht-Nahrungsmittelindustrie

In diesem großen Bereich kann die Stärke als Hilfsstoff für unterschiedliche Herstellungsprozesse bzw. als Zusatzstoff in technischen Produkten eingesetzt werden. Bei der Verwendung der Stärke als Hilfsstoff ist hier insbesondere die Papier- und Pappeindustrie zu nennen. Die Stärke dient dabei in erster Linie zur Retardation (Zurückhaltung von Feststoffen), der Abbindung von Füllstoff- und Feinstoffteilchen, als Festigungsstoff und zur Entwässerung. Darüber hinaus werden die günstigen Eigenschaften der Stärke in bezug auf die Steifigkeit, die Härte, den Klang, den Griff, den Glanz, die Glätte, die Spaltfestigkeit sowie die Oberflächen ausgenutzt.

2.1 Papier- und Pappeindustrie

Innerhalb des Papierherstellungsprozesses sind vier Anwendungsbereiche, nämlich Oberfläche, Strich, Masse und Sprühen, zu unterscheiden.

Die Anforderungen an die Stärke in bezug auf die Oberflächenbehandlung sind im Wesentlichen ein hoher Weißegrad, eine angepasste Viskosität, eine hohe Viskositätsstabilität, eine gute Filmbildung sowie eine geringe Staubbildung. Bei der Verwendung im Strich spielt der Feststoffgehalt, eine angepasste Viskosität, ein hohes Bindevermögen sowie eine hohe Pigmentaffinität eine wichtige Rolle. Als Zusatz zur Masse ist eine rasche, gleichmäßige, verlustfreie Verteilung, eine hohe mechanische Stabilität und eine vollständige Zurückhaltung im Papiervlies von Bedeutung. Beim Einsatz der Stärke im Sprühbereich sind ebenfalls ein angepasster Feststoffgehalt, hohe Viskosität sowie ein hohes Bindevermögen von Bedeutung.

2.2 Klebstoffindustrie

Ein großer Einsatzbereich der Stärken besteht in der Klebstoffindustrie, wo man die Einsatzmöglichkeiten in vier Teilbereiche gliedert: die Verwendung als reinem Stärkeleim, die Verwendung bei mit speziellen Chemikalien aufbereiteten Stärkeleimen, die Verwendung von Stärke als Zusatz zu synthetischen Harzen und Polymerdispersionen sowie die Verwendung von Stärken als Streckmittel für synthetische Klebstoffe. 90% der Klebstoffe auf Stärkebasis werden in den Bereichen Wellpappenherstellung, Herstellung von Papiersäcken, Beuteln und Tüten, Herstellung von Verbundmaterialien für Papier und Aluminium, Herstellung von Kartonagen und Wiederbefeuchtungsleim für Briefumschläge, Briefmarken usw. eingesetzt.

2.3 Textil- und Textilpflegemittelindustrie

Ein großes Einsatzfeld für die Stärken als Hilfsmittel und Zusatzstoffe ist der Bereich Herstellung von Textilien und Textilpflegemitteln. Innerhalb der Textilindustrie sind die folgenden vier Einsatzbereiche zu unterscheiden: Der Einsatz der Stärke als Schüchtmittel, d.h. als Hilfsstoff zur Glättung und Stärkung des Klettverhaltens zum Schutz gegen die beim Weben angreifenden Zugkräfte sowie zur Erhöhung der Abriebfestigkeit beim Weben, Stärke als Mittel zur Textilaufrüstung vor allem nach qualitätsverschlechternden Vorbehandlungen, wie Bleichen, Färben usw., Stärke als Verdickungsmittel bei der Herstellung von Farbpasten zur Verhinderung von Farbstoffdiffusionen sowie Stärke als Zusatz zu Kettungsmitteln für Nähgarne.

2.4 Baustoffindustrie

Der vierte Einsatzbereich ist die Verwendung der Stärken als Zusatz bei Baustoffen. Ein Beispiel ist die Herstellung von Gipskartonplatten, bei der die im Gipsbrei vermischte Stärke mit dem Wasser verkleistert, an die Oberfläche der Gipsplatte diffundiert und dort den Karton an die Platte bindet. Weitere Einsatzbereiche sind die Beimischung zu Putz- und Mineralfasern. Bei Transportbeton werden Stärkeprodukte zur Verzögerung der Abbindung eingesetzt.

2.5 Bodenstabilisation

Ein weiterer Markt für die Stärke bietet sich bei der Herstellung von Mitteln zur Bodenstabilisation an, die bei künstlichen Erdbewegungen zum temporären Schutz der Bodenpartikel gegenüber Wasser eingesetzt werden. Kombinationsprodukte aus der Stärke und Polymeremulsionen sind nach heutiger Kenntnis in ihrer Erosions- und Verkrustungsmindemden Wirkung den bisher eingesetzten Produkten gleichzusetzen, liegen preislich aber deutlich unter diesen.

2.6 Einsatz bei Pflanzenschutz- und Düngemitteln

Ein Einsatzbereich liegt bei der Verwendung der Stärke in Pflanzenschutzmitteln zur Veränderung der spezifischen Eigenschaften der Präparate. So kann die Stärke zur Verbesserung der Benetzung von Pflanzenschutz- und Düngemitteln, zur dosierten Freigabe der Wirkstoffe, zur Umwandlung flüssiger, flüchtiger und/oder übelriechender Wirkstoffe in mikrokristalline, stabile, formbare Substanzen, zur Mischung inkompatibler Verbindungen und zur Verlängerung der Wirkdauer durch Verminderung der Zersetzung eingesetzt werden.

2.7 Pharmaka, Medizin und Kosmetikindustrie

Ein weiteres Einsatzgebiet besteht im Bereich der Pharmaka, Medizin und Kosmetikindustrie. In der pharmazeutischen Industrie kann die Stärke als Bindemittel für Tabletten oder zur Bindemittelverdünnung in Kapseln eingesetzt werden. Weiterhin kann die Stärke als Tablettensprengmittel dienen, da sie nach dem Schlucken Flüssigkeit absorbiert und nach kurzer Zeit soweit quellt, dass der Wirkstoff freigesetzt wird. Medizinische Gleit- und Wundpuder basieren aus qualitativen Gründen auf Stärke. Im Bereich der Kosmetik werden Stärken beispielsweise als Träger von Puderzusatzstoffen, wie Düften und Salicylsäure eingesetzt. Ein relativ großer Anwendungsbereich für die Stärke liegt bei Zahnpasta.

2.8 Stärkezusatz zu Kohlen und Briketts

Einen Einsatzbereich bietet die Stärke als Zusatzstoff zu Kohle und Brikett. Kohle kann mit einem Stärkezusatz quantitativ hochwertig agglomeriert bzw. brikettiert werden, wodurch ein frühzeitiges Zerfallen der Briketts verhindert wird. Der Stärkezusatz liegt bei Grillkohle zwischen 4 und 6%, bei kalorierter Kohle zwischen 0,1 und 0,5%. Des Weiteren gewinnen Stärken als Bindemittel an Bedeutung, da durch ihren Zusatz zu Kohle und Brikett der Ausstoß schädlicher Stoffe deutlich vermindert werden kann.

2.9 Erz- und Kohleschlammaufbereitung

Die Stärke kann ferner bei der Erz- und Kohleschlammaufbereitung als Flockungsmittel eingesetzt werden.

2.10 Gießereihilfsstoff

Ein weiterer Einsatzbereich besteht als Zusatz zu Gießereihilfsstoffen. Bei verschiedenen Gussverfahren werden Kerne benötigt, die aus bindemittelversetzten Sanden hergestellt werden. Als Bindemittel wird heute überwiegend Bentonit eingesetzt, das mit modifizierten Stärken, meist Quellstärken, versetzt ist.

Zweck des Stärkezusatzes ist die Erhöhung der Fließfestigkeit sowie die Verbesserung der Bindefestigkeit. Darüber hinaus können die Quellstärken weitere produktionstechnische Anforderungen, wie im kalten Wasser dispergierbar, rehydratisierbar, gut in Sand mischbar und hohes Wasserbindungsvermögen, aufweisen.

2.11 Einsatz in der Kautschukindustrie

In der Kautschukindustrie kann die Stärke zur Verbesserung der technischen und optischen Qualität eingesetzt werden. Gründe sind dabei die Verbesserung des Oberflächenglanzes, die Verbesserung des Griffs und des Aussehens (dafür wird Stärke vor der Kaltvulkanisation auf die klebrigen gummierten Flächen von Kautschukstoffen gestreut), sowie die Verbesserung der Bedruckbarkeit des Kautschuks.

2.12 Verwendung als Bohrhilfsstoff

Eine weitere Anwendungsmöglichkeit für die erfindungsgemäßen Stärken findet sich auf dem Gebiet der Förderung von Rohstoffen unter Einsatz von Bohrern. So ist es z.B. bei der Förderung von Rohöl notwendig, Hilfsstoffe und/oder Schmiermittel einzusetzen, die eine Überhitzung des Bohrers, bzw. des Bohrgestänges vermeiden.

2.13 Herstellung von Ledersatzstoffen

Eine weitere Absatzmögüchkeit der modifizierten Stärken besteht bei der Herstellung von Ledersatzstoffen.

2.14 Stärke in synthetischen Polymeren

Auf dem Kunststoffsektor zeichnen sich folgende Einsatzgebiete ab: die Einbindung von Stärkefolgeprodukten in den Verarbeitungsprozess (Stärke ist nur Füllstoff, es besteht keine direkte Bindung zwischen synthetischem Polymer und Stärke) oder alternativ die Einbindung von Stärkefolgeprodukten in die Herstellung von Polymeren (Stärke und Polymer gehen eine feste Bindung ein).

Die Verwendung der Stärke als reinem Füllstoff ist, verglichen mit den anderen Stoffen wie Talkum, nicht wettbewerbsfähig. Anders sieht es aus, wenn die spezifischen Stärkeeigenschaften zum Tragen kommen und hierdurch das Eigenschaftsprofil der Endprodukte deutlich verändert wird. Ein Beispiel hierfür ist die Anwendung von Stärkeprodukten bei der Verarbeitung von Thermoplasten, wie Polyethylen. Hierbei werden die Stärke und das synthetische Polymer durch Koexpression im Verhältnis von 1 : 1 zu einem "master batch" kombiniert, aus dem mit granuliertem Polyethylen unter Anwendung herkömmlicher Verfahrenstechniken diverse Produkte hergestellt werden. Durch die Einbindung von Stärke in Polyethylenfolien kann eine erhöhte Stoffdurchlässigkeit bei Hohlkörpern, eine verbesserte Wasserdampfdurchlässigkeit, ein verbessertes Antistatikverhalten, ein verbessertes Antiblockverhalten sowie eine verbesserte Bedruckbarkeit mit wässrigen Farben erreicht werden.

Eine andere Möglichkeit ist die Anwendung der Stärke in Polyurethanschäumen. Mit der Adaption der Stärkederivate sowie durch die verfahrenstechnische Optimierung ist es möglich, die Reaktion zwischen synthetischen Polymeren und den Hydroxygruppen der Stärken gezielt zu steuem. Das Ergebnis sind Polyurethanfolien, die durch die Anwendung von Stärke folgende Eigenschaftsprofile erhalten: eine Verringerung des Wärmeausdehnungskoeffizienten, Verringerung des Schrumpfverhaltens, Verbesserung des Druck/Spannungsverhaltens, Zunahme der Wasserdampfdurchlässigkeit ohne Veränderung der Wasseraufnahme, Verringerung der Entflammbarkeit und der Aufrissdichte, kein Abtropfen brennbarer Teile, Halogenfreiheit und verminderte Alterung. Nachteile, die gegenwärtig noch vorhanden sind, sind verringerte Druckfestigkeit sowie eine verringerte Schlagfestigkeit.
Die Produktentwicklung beschränkt sich inzwischen nicht mehr nur auf Folien. Auch feste Kunststoffprodukte, wie Töpfe, Platten und Schalen, sind mit einem Stärkegehalt von über 50 % herzustellen. Des Weiteren sind Stärke/ Polymermischungen günstig zu beurteilen, da sie eine sehr viel höhere biologische Abbaubarkeit aufweisen.

Außerordentliche Bedeutung haben weiterhin auf Grund ihres extremen Wasserbindungsvermögens Stärkepfropfpolymerisate gewonnen. Dies sind Produkte mit einem Rückgrat aus Stärke und einer nach dem Prinzip des Radikalkettenmechanismus aufgepfropften Seitengitters eines synthetischen Monomers. Die heute verfügbaren Stärkepfropfpolymerisate zeichnen sich durch ein besseres Binde- und Rückhaltevermögen von bis zu 1000 g Wasser pro g Stärke bei hoher Viskosität aus. Die Anwendungsbereiche für diese Superabsorber haben sich in den letzten Jahren stark ausgeweitet und liegen im Hygienebereich mit Produkten wie Windeln und Unterlagen sowie im landwirtschaftlichen Sektor, z.B. bei Saatgutpillierungen.

Entscheidend für den Einsatz der neuen Stärken sind zum einen die Struktur, Wassergehalt, Proteingehalt, Lipidgehalt, Fasergehalt, Asche/Phosphatgehalt, Amylose/Amylopektin-Verhälmis, Molmassenverteilung, Verzweigungsgrad, Korngröße und -form sowie Kristallinität, zum anderen auch die Eigenschaften, die in folgende Merkmale münden: Fließ- und Sorptionsverhalten, Verkleisterungstemperatur, Viskosität, Dickungsleistung, Löslichkeit, Kleisterstruktur und - transparenz, Hitze-, Scher- und Säurestabilität, Retrogradationsneigung, Gelbildung, Gefrier/Tau-Stabilität, Komplexbildung, Jodbindung, Filmbildung, Klebekraft, Enzymstabilität, Verdaulichkeit und Reaktivität.

Die erfindungsgemäße, vorzugsweise native, Kartoffelstärke kann durch Isolierung aus genetisch modifizierten Kartoffel-Pflanzen hergestellt werden, wobei die genetische Modifikation zur Verringerung der GBSSI-, SSIII- und BEI-Aktivitäten und zu einer Verringerung der Genexpression des unter SEQ ID No. 11 oder SEQ ID No. 13 angegebenen Gens führt im Vergleich zu denjenigen entsprechender Kartoffel-Wildtyp-Pflanzenzellen bzw. Kartoffel-Wildtyp-Pflanzen.

Gegenstand der vorliegenden Erfindung sind daher auch Pflanzenzellen und Pflanzen, die genetisch modifiziert sind, wobei die genetische Modifikation zur Verringerung der GBSSI-, SSIII- und BEI-Aktivitäten und zu einer Verringerung der Genexpression des unter SEQ ID No. 11 oder SEQ ID No. 13 angegebenen Gens führt im Vergleich zu denjenigen entsprechender Wüdtyp-Pflanzenzelien bzw. Wildtyp-Pflanzen.

Unter dem Begriff "GBSSI" ist im Zusammenhang mit der vorliegenden Erfindung jedes Enzym zu verstehen, das zur Klasse der Stärkekom-gebundenen Stärkesynthasen der Isoform I (EC 2.4.1.21) gehört.

Unter dem Begriff "GBSSI-Gen" ist im Zusammenhang mit der vorliegenden Erfindung ein Nukleinsäuremolekül oder Polynukleotid (cDNA, DNA) zu verstehen, das für GBSSI codiert. Polynukleotide kodierend für GBSSI sind für verschiedene Pflanzenspezies, wie z.B. für Mais (Genbank Acc. Nos. AF079260, AF079261), Weizen (Genbank Acc. Nos. AB019622, AB019623, AB019624), Reis (Genbank Acc. Nos. AF092443, AF092444, AF031162), Gerste (Genbank Acc. Nos. X07931, X07932) und Kartoffel (Genbank Acc. No. X58453) beschrieben. In einer Ausführungsform der Erfindung weist das GBSSI-Gen zu der kodierenden Region der in Seq ID No. 6 dargestellten Nukleotidsequenz eine Identität von mindestens 70% auf, insbesondere von mindestens 80%, von mindestens 90%, bevorzugt von mindestens 95%. In einer besonders bevorzugten Ausführungsform ist das GBSSI-Gen im Zusammenhang mit der vorliegenden Erfindung ein Nukleinsäuremolekül (cDNA, DNA), das für GBSSI aus Kartoffelpflanzen codiert, besonders bevorzugt ist das unter SEQ ID No. 6 angegebene GBSSI-Gen.

Unter dem Begriff "SSIII" ist im Zusammenhang mit der vorliegenden Erfindung eine bestimmte Klasse von löslichen Stärkesynthasen (ADP-Glukose 1,4-$\alpha$-D-glukan 4-$\alpha$-D-glucosyltransferase; EC 2.4.1.21) zu verstehen. Lösliche Stärkesynthasen katalysieren eine Glycosylierungsreaktion, bei der Glukosereste des Substrates ADP-Glukose unter Bildung einer $\alpha$-1,4-Verknüpfung auf $\alpha$-1,4-verknüpfte Glukanketten übertragen werden (ADP-Glukose + {(1,4)-$\alpha$-D-glucosyl}(N) <=> ADP+ {(1,4)-$\alpha$-D-glucosyl) (N+1)).

Beschrieben sind SSIII zum Beispiel bei Marshall et al. (1996, The Plant Cell 8, 1121-1135), Li et al. (2000, Plant Physiology 123, 613-624), Abel et al. (1996, The Plant Journal 10(6), 981-991) und in WO 00/66745. SSIII weisen häufig in ihrem Aufbau eine Abfolge von bestimmten Domänen auf und besitzen am N-Terminus ein Signalpeptid für den Transport in Plastiden. In Richtung C-Terminus folgen eine N-terminale Region, eine SSIII-spezifische Region und eine katalytische Domäne (Li et al., 2000, Plant Physiology 123, 613-624). Weitere Analysen, basierend auf Primärsequenzvergleichen (http://hits.isb-sib.ch/cgi-bin/PFSCAN), ergaben, dass das SSIII Protein aus Kartoffel eine so genannte Kohlenhydrat- Bindedomäne (CBM von Carbohydrate Binding Domain) aufweist. Diese Domäne (Pfam Motiv cbm 25 = SEQ ID No.3) umfasst die Aminosäuren 377 bis 437 der in SEQ ID No. 2 dargestellten Sequenz des SSIII-Proteins aus Kartoffel. Unter einem SSIII Protein soll im Zusammenhang mit der vorliegenden Erfindung ein Protein verstanden werden, das zu der in Seq ID No. 2 dargestellten Sequenz eine Identität von mindestens 70% aufweist, bevorzugt von mindestens 80%, besonders bevorzugt von mindestens 90%, ganz besonders bevorzugt von mindestens 95%.
Unter dem Begriff "SSIII-Gen" ist im Zusammenhang mit der vorliegenden Erfindung ein Nukleinsäuremolekül (DNA, cDNA) zu verstehen, welches für ein SSIII-Protein codiert. Nukleinsäuremoleküle kodierend für SSIII sind für verschiedene Pflanzenspezies, wie z.B. Kartoffel (Abel et al., 1996, The Plant Journal 10(6), 981-991) beschrieben. Unter einem "SSIII-Gen" soll im Zusammenhang mit der vorliegenden Erfindung ein Nukleinsäuremolekül verstanden werden, das zu der kodierenden Region der in SEQ ID No. 1 dargestellten Sequenz eine Identität von mindestens 70% aufweist, insbesondere von mindestens 80%, bevorzugt von mindestens 90%, ganz besonders bevorzugt von mindestens 95%.
In einer besonders bevorzugten Ausführungsform ist das SSIII-Gen im Zusammenhang mit der vorliegenden Erfindung ein Nukleinsäuremolekül (cDNA, DNA), das für SSIII aus Kartoffelpflanzen codiert, besonders bevorzugt ist das unter SEQ ID No. 1 angegebene SSIII-Gen aus Kartoffel.

Unter dem Begriff "BEI" ist im Zusammenhang mit der vorliegenden Erfindung ein Verzweigungsenzym ("Branching Enzyme" = BE) der Isoform I (α-1,4-Glukan: α-1,4-Glukan 6-Glycosyltransferase; E.C. 2.4.1.18) zu verstehen, das eine Transglycosylierungsreaktion katalysiert, in der α-1,4-Verknüpfungen eines α-1,4-Glukandonors hydrolysiert und die dabei freigesetzten α-1,4-Glukanketten auf eine α-1,4-Glukanakzeptorkette transferiert und dabei in α-1,6-Verknüpfungen überführt werden.Vorzugsweise stammt BEI aus Kartoffelpflanzen.

Die Bezeichnung der Isoformen lehnt dabei an der von Smith-White und Preiss vorgeschlagenen Nomenklatur an (Smith-White und Preiss, 1994, Plant Mol. Biol. Rep. 12, 67-71; Larsson et al., 1998, Plant Mol. Biol. 37, 505-511). Diese Nomenklatur geht davon aus, dass alle Enzyme, die zu BEI aus Mais (GenBank Acc. No. D11081; Baba et al., 1991, Biochem. Biophys. Res. Commun. 181 (1), 87-94; Kim et al., 1998, Gene 216, 233-243) eine höhere Homologie (Identität) auf Aminosäureebene aufweisen als zum BEII aus Mais (Genbank Acc. Nos. AF072725, U65948), als "Branching Enzyme" der Isoform I oder kurz als BEI bezeichnet werden.

Unter dem Begriff "BEI-Gen" ist im Zusammenhang mit der vorliegenden Erfindung ein Nukleinsäuremolekül oder Polynukleotid (cDNA, DNA) zu verstehen, das für BEI codiert. Polynukleotide kodierend für BEI sind für verschiedene Pflanzenspezies, wie z.B. für Mais (Genbank Acc. Nos. D11081, AF072724), Reis (Genbank Acc. No. D11082) und Kartoffel beschrieben. Verschiedene Formen des BEI-Gens bzw. des BEI aus Kartoffel wurden beispielsweise beschrieben von Khoshnoodi et al. (1996, Eur. J. Biochem. 242 (1), 148-155, Genbank Acc. No. Y08786) und von Kossmann et al. (1991, Mol. Gen. Genet. 230, 39-44).
In einer Ausführungsform der Erfindung weist das BEI-Gen zu der kodierenden Region der in Seq ID No. 4 dargestellten Nukleotidsequenz eine Identität von mindestens 70% auf, insbesondere von mindestens 80%, von mindestens 90%, bevorzugt von mindestens 95%. In einer besonders bevorzugten Ausführungsform ist das BEI-Gen im Zusammenhang mit der vorliegenden Erfindung ein Nukleinsäuremolekül (cDNA, DNA), das für BEI aus Kartoffelpflanzen codiert, besonders bevorzugt ist das unter SEQ ID No. 4 angegebene BEI-Gen. In Kartoffelpflanzen wird das BEI-Gen hauptsächlich in den Knollen und kaum in den Blättern exprimiert (Larsson et al., 1998, Plant Mol. Biol. 37, 505-511).

Im Rahmen der Definitionen der Begriffe "GBSSI-Gen", "SSIII-Gen" bzw. "BEI-Gen" wird durch Angabe von "Genbank Acc."-Nummern und Literaturstellen Bezug genommen auf spezifische Polynukleotidsequenzen, die für die entsprechenden Enzyme kodieren. Im Folgenden werden Ausführungsformen der vorliegenden Erfindung beschrieben, bei denen Polynukleotide mit den dort angegebenen Sequenzen verwendet werden können. Dabei ist die Erfindung selbstverständlich nicht auf die Verwendung solcher exakt beschriebenen Sequenzen oder Teilen davon beschränkt. Es können beispielsweise auch Polynukleotide verwendet werden, die zu den angegebenen Sequenzen eine Identität von mindestens 80%, bevorzugt von mindestens 90%, besonders bevorzugt von mindestens 95% und ganz besonders bevorzugt von mindestens 98% aufweisen.

Gene, die die unter SEQ ID NO 11 oder SEQ ID NO 13 angegebene Nukleotidsequenz, und deren korrespondierende Proteine, die die unter SEQ ID NO 12 oder SEQ ID NO 14 angegebene Aminosäuresequenz aufweisen, sind an der Stärkebiosynthese in Pflanzen beteiligt. Die Aminosäuresequenzen dieser Proteine weisen eine Homologie zu Proteinen aus *Arabidopsis thaliana* (GenBank Acc. No: BAB02827) auf, die dort als Verzweigungsenzym-ähnliche Proteine beschrieben werden. Im Zusammenhang mit der vorliegenden Erfindung wurde überraschenderweise gefunden, dass Kartoffel-Pflanzen, die eine verringerte Genexpression des unter SEQ ID NO 11 oder SEQ ID NO 13 angegebenen Gens aufweisen und eine Verringerung der GBSSI-, SSIII- und BEI-Aktivitäten, die erfindungsgemäßen, vorzugsweise nativen, Kartoffelstärken herstellen. Daraus kann geschlossen werden, dass das unter SEQ ID NO 11 oder SEQ ID NO 13 angegebene Gen bzw. das durch dieses Gen codierte Protein mit der unter SEQ ID NO 12 oder SEQ ID NO 14 angegebenen Aminosäuresequenz an der Synthese der Seitenketten des Amylopektins und an der Phosphorylierung von Stärke in Kartoffelpflanzen beteiligt ist.

Unter dem Begriff "Identität" ist im Zusammenhang mit der vorliegenden Erfindung die Anzahl der übereinstimmenden Aminosäuren/Nukleotide mit anderen Proteinen/Nukleinsäuren, ausgedrückt in Prozent, zu verstehen. Bevorzugt wird die Identität mit Hilfe von Computerprogrammen ermittelt. Weisen Sequenzen, die miteinander verglichen werden, unterschiedliche Längen auf, ist die Identität so zu ermitteln, dass die Anzahl an Aminosäuren, welche die kürzere Sequenz mit der längeren Sequenz gemeinsam hat, den prozentualen Anteil der Identität bestimmt. Vorzugsweise wird die Identität mittels des bekannten und der Öffentlichkeit zur Verfügung stehenden Computerprogramms ClustalW (Thompson et al., 1994, Nucleic Acids Research 22, 4673-4680) ermittelt. ClustalW wird öffentlich zur Verfügung gestellt von Julie Thompson (Thompson@EMBL-Heidelberg.de) und Toby Gibson (Gibson@EMBL-Heidelberg.de), European Molecular Biology Laboratory, Meyerhofstrasse 1, 69117 Heidelberg, Deutschland. ClustalW kann ebenfalls von verschiedenen Internetseiten, u.a. beim IGBMC (Institut de Genetique et de Biologie Moléculaire et Cellulaire, B.P. 163, 67404 Illkirch Cedex, France; ftp://ftp-igbmc.u-strasbg.fr/pub/) und beim EBI (ftp://ftp.ebi.ac.uk/pub/software/) sowie bei allen gespiegelten Internetseiten des EBI (European Bioinformatics Institute, Wellcome Trust Genome Campus, Hinxton, Cambridge CB10 1SD, UK), heruntergeladen werden.

Vorzugsweise wird das ClustalW Computerprogramm der Version 1.8 benutzt, um die Identität zwischen hierin beschriebenen Proteinen und anderen Proteinen zu bestimmen. Dabei sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP.

Vorzugsweise wird das ClustalW Computerprogramm der Version 1.8 benutzt, um die Identität zwischen den Nukleotidsequenzen der hierin beschriebenen Nukleinsäuremoleküle und der Nukleotidsequenz von anderen Nukleinsäuremolekülen zu bestimmen. Dabei sind folgende Parameter einzustellen: KTUPLE=2, TOPDIAGS=4, PAIRGAP=5, DNAMATRIX:IUB. GAPOPEN=10, GAPEXT=5, MAXDIV=40, TRANSITIONS: unweighted.

Eine Möglichkeit zum Auffinden von ähnlichen Sequenzen, ist die Durchführung von Sequenzdatenbankrecherchen. Hierbei wird eine oder werden mehrere Sequenzen als so genannte Abfrage (= query) vorgegeben. Diese Abfragesequenz wird dann mittels statistischer Computerprogramme mit Sequenzen, die in den ausgewählten Datenbanken enthalten sind, verglichen. Solche Datenbankabfragen (blast searches) sind dem Fachmann bekannt und können bei verschiedenen Anbietern durchgeführt werden. Wird eine solche Datenbankabfrage z.B. beim NCBI (National Center for Biotechnology Information, http://www.ncbi.ntm.nih.gov/) durchgeführt, so sollen die Standardeinstellungen, die für die jeweilige Vergleichsanfrage vorgegeben sind, benutzt werden. Für Proteinsequenzvergleiche (blastp) sind dies folgende Einstellungen: Limit entrez = nicht aktiviert; Filter = low compexity aktiviert; Expect value = 10; word size = 3; Matrix = BLOSUM62; Gap costs: Existence = 11, Extension = 1. Als Ergebnis einer solchen Abfrage werden neben anderen Parametern auch der Anteil an Identität zwischen der Abfragesequenz und den in den Datenbanken aufgefundenen ähnlichen Sequenzen dargestellt.

In einer Ausführungsform der vorliegenden Erfindung wird die genetische Modifikation der erfindungsgemäßen Pflanzenzellen oder der erfindungsgemäßen Pflanzen durch Mutagenese eines oder mehrerer Gene hervorgerufen. Die Art der Mutation ist dafür unerheblich, solange sie zu einer Verringerung der GBSSI- und/oder SSIII- und/oder BEI-Aktivität und zu einer Verringerung der Genexpression des unter SEQ ID NO 11 oder SEQ ID NO 13 angegebenen Gens führt.

Unter dem Begriff "Mutagenese" ist im Zusammenhang mit der vorliegenden Erfindung jegliche Art von eingeführten Mutationen zu verstehen, wie z.B. Deletionen, Punktmutationen (Nukleotidaustausche), Insertionen, Inversionen, Genkonversionen oder Chromosomentranslokationen.

Eine Mutation, die zur Verringerung der GBSSI- und/oder SSIII- und/oder BEI-Aktivität und zu einer Verringerung der Genexpression des unter SEQ ID NO 11 oder SEQ ID NO 13 angegebenen Gens führt, kann spontan in einer Pflanze auftreten, und die entsprechenden Pflanzen können mit Hilfe der unten beschriebenen Methoden selektiert und vermehrt werden.

Eine Mutation, die zur Verringerung der GBSSI- und/oder SSIII- und/oder BEI-Aktivität und zu einer Verringerung der Genexpression des unter SEQ ID NO 11 oder SEQ ID NO 13 angegebenen Gens führt, kann auch durch den Einsatz chemischer Agenzien oder energiereicher Strahlung (z.B. Röntgen-, Neutronen-, Gamma-, UV-Strahlung) erzeugt werden.

Agenzien, die zur Erzeugung chemisch induzierter Mutationen eingesetzt werden können und die durch Einwirkung der entsprechenden Mutagene dabei entstehenden Mutationen sind z.B. beschrieben von Ehrenberg und Husain (1981, Mutation Research 86, 1-113) und Müller (1972, Biologisches Zentralbtatt 91 (1), 31-48). Die Erzeugung von Reis-Mutanten unter Verwendung von Gamma-Strahlen, Ethyl-Methan-Sulfonat (EMS), N-Methyl-N-nitrosoharnstoff oder Natriumazid ($NaN_3$) ist z.B. beschrieben von Jauhar und Siddiq (1999, Indian Journal of Genetics, 59 (1), 23-28), Rao (1977, Cytologica 42, 443-450), Gupta und Sharma (1990, Oryza 27, 217-219) und Satoh und Omura (1981, Japanese Journal of Breeding 31 (3), 316-326). Die Erzeugung von Weizen-Mutanten unter Verwendung von $NaN_3$ bzw. Maleinsäurehydrazid ist von Arora et al. (1992, Annals of Biology 8 (1), 65-69) beschrieben. Eine Übersicht zur Erzeugung von Weizen-Mutanten unter Verwendung von verschiedenen Arten energiereicher Strahlung und chemischer Agenzien ist von Scarascia-Mugnozza et al. (1993, Mutation Breeding Review 10, 1-28) dargestellt. Svec et al. (1998, Cereal Research Communications 26 (4), 391-396) beschreiben die Anwendung von N-Ethyl-N-nitrosoharnstoff zur Erzeugung von Mutanten in Triticale. Die Verwendung von MMS (Methylmethansulfonsäure) und Gamma-Strahlung zur Erzeugung von Hirse-Mutanten ist von Shashidhara et al. (1990, Journal of Maharashtra Agricultural Universities 15 (1), 20-23) beschrieben.

Die Herstellung von Mutanten in Pflanzenspezies, die sich hauptsächlich vegetativ vermehren, wurde z.B. für Kartoffeln, die eine veränderte Stärke produzieren (Hovenkamp-Hermelink et al., 1987, supra) und für Minze mit erhöhtem Ölertrag bzw. veränderter Ölqualität (Dwivedi et al., 2000, Journal of Medicinal and Aromatic Plant Sciences 22, 460-463) beschrieben.

Alle diese Methoden sind grundsätzlich zur Herstellung der erfindungsgemäßen Pflanzenzellen bzw. der erfindungsgemäßen Pflanzen geeignet.

Das Auffinden von Mutationen in den entsprechenden Genen, insbesondere in Genen, die GBSSI, SSIII oder BEI kodieren, oder Genen, die die unter SEQ ID NO 11 oder SEQ ID NO 13 angegebene Nukleotidsequenz aufweisen, kann mit Hilfe von dem Fachmann bekannten Methoden geschehen. Insbesondere können hierzu Analysen, basierend auf Hybridisierungen mit Sonden ("Southern Blot"), der Amplifikation mittels Polymerasekettenreaktion (PCR), der Sequenzierung betreffender genomischer Nukleinsäure-Fragmente und die Suche nach einzelnen Nukleotidaustauschen angewandt werden. Eine Methode, um Mutationen anhand von Hybridisierungsmustern zu identifizieren, ist z.B. die Suche nach Restriktionsfragment-Längenunterschieden ("Restriction Fragment Length Polymorphism", RFLP) (Nam et al., 1989, The Plant Cell 1, 699-705; Leister and Dean, 1993, The Plant Journal 4 (4), 745-750). Eine auf PCR basierende Methode ist z.B. die Analyse von amplifizierten Fragment-Längenunterschieden ("Amplified Fragment Length Polymorphism", AFLP) (Castiglioni et al., 1998, Genetics 149, 2039-2056; Meksem et al., 2001, Molecular Genetics and Genomics 265, 207-214; Meyer et al., 1998, Molecular and General Genetics 259, 150-160). Auch die Verwendung von mit Restriktionsendonukleasen geschnittenen amplifizierten Fragmenten ("Cleaved Amplified Polymorphic Sequences", CAPS) kann zur Identifizierung von Mutationen herangezogen werden (Konieczny und Ausubel, 1993, The Plant Journal 4, 403-410; Jarvis et al., 1994, Plant Mol. Biol. 24, 685-687; Bachem et al., 1996, The Plant Journal 9 (5), 745-753). Methoden zur Ermittlung von SNPs sind u.a. von Qi et al. (2001, Nucleic Acids Research 29 (22), el 16), Drenkard et al. (2000, Plant Physiology 124, 1483-1492) und Cho et al. (1999, Nature Genetics 23, 203-207) beschrieben worden. Insbesondere sind Methoden, die es erlauben, viele Pflanzen innerhalb kurzer Zeit auf Mutationen in bestimmten Genen hin zu untersuchen, geeignet. Solch eine Methode, das sogenannte TILLING ("Targetting Induced Local Lesions In Genomes") ist von McCallum et al. (2000, Plant Physiology 123, 439-442) beschrieben worden.

Alle diese Methoden sind grundsätzlich zur Identifizierung erfindungsgemäßer Pflanzenzellen bzw. der erfindungsgemäßer Pflanzen geeignet.

Hoogkamp et al. (2000, Potato Research 43, 179-189) haben, ausgehend von einer mittels chemischer Mutagense hergestellten Kartoffel-Mutante (*amf*), stabile monoploide Mutanten hergestellt. Diese Pflanzen synthetisieren keine aktive GBSSI mehr und produzieren daher eine amylosefreie Stärke. Die erhaltenen monoploiden Kartoffelpflanzen können als Ausgangsmaterial für weitere Mutagenesen eingesetzt werden.

Eine Verringerung der GBSSI- und/oder SSIII- und/oder BEI-Aktivität(en) und eine Verringerung der Genexpression des unter SEQ ID No. SEQ ID NO 11 oder SEQ ID NO 13 angegebenen Gens kann durch eine Verringerung der Expression eines oder mehrere für GBSSI bzw. SSIII bzw. BEI kodierender Gene bzw. Genen, die die SEQ ID NO 11 oder SEQ ID NO 13 angegebene Nukleotidsequenz aufweisen und/oder durch eine Verringerung der Menge an betreffendem Enzymmaterial in den Pflanzenzellen und/oder durch eine Verringerung der enzymatischen Aktivität der betreffenden Proteine in den Pflanzenzellen erreicht werden.

Die Verringerung der Expression kann beispielsweise bestimmt werden durch Messung der Menge an Transkripten, die für die betreffenden Enzyme kodieren, z.B. durch Northem-Blot-Analyse oder RT-PCR. Eine Verringerung bedeutet dabei vorzugsweise eine Verringerung der Menge an Transkripten im Vergleich zu derjenigen in entsprechenden Wildtyp-Pflanzenzellen um mindestens 50%, insbesondere um mindestens 70%, bevorzugt um mindestens 85% und besonders bevorzugt um mindestens 95%.

Die Verringerung der Menge an GBSSI und/oder SSIII und/oder BEI, die eine Verringerung der betreffenden Enzymaktivität(en) in den Pflanzenzellen zur Folge hat, kann beispielsweise bestimmt werden durch immunologische Methoden, wie Western-Blot-Analyse, ELISA ("Enzyme Linked Immuno Sorbent Assay") oder RIA ("Radio Immune Assay"). Eine Verringerung bedeutet dabei vorzugsweise eine Verringerung der Menge an betreffendem Protein im Vergleich zu derjenigen in entsprechenden Wildtyp-Pflanzenzellen um mindestens 50%, insbesondere um mindestens 70%, bevorzugt um mindestens 85% und besonders bevorzugt um mindestens 95%.

In einer weiteren Ausführungsform der vorliegenden Erfindung besteht die genetische Modifikation der erfindungsgemäßen Pflanzenzelle in der Einführung eines oder mehrerer fremder Nukleinsäuremoleküle/Polynukleotide, deren Vorhandensein und/oder Expression zur Verringerung der GBSSI- und/oder SSIII- und/oder BEI-Aktivität(en) und zu einer Verringerung der Genexpression des SEQ ID NO 11 oder SEQ ID NO 13 angegebenen Gens führt im Vergleich zu derjenigen/denjenigen in entsprechenden Wildtyp-Pflanzenzellen. Im Speziellen versteht man unter dem Begriff der genetischen Modifikation das Einbringen von homologen und/oder heterologen und/oder mutagenisierten fremden Nukleinsäuremolekülen/Polynukleotiden in eine Pflanzenzelle, wobei besagtes Einbringen dieser Moleküle zur Verringerung der GBSSI- und/oder SSIII- und/oder BEI-Aktivität(en) und zu einer Verringerung der Genexpression des unter SEQ ID NO 11 oder SEQ ID NO 13 angegebenen Gens führt. Auf diese Weise können also die erfindungsgemäßen, transgenen Pflanzenzellen erzeugt werden.

Der Begriff "transgen" bedeutet in diesem Zusammenhang, dass die erfindungsgemäßen Pflanzenzellen aufgrund der Einführung eines fremden Nukleinsäuremoleküls/Polynukleotids oder mehrerer fremder Nukleinsäuremoleküle/Polynukleotide in die Zelle in ihrer genetischen Information von entsprechenden Wildtyp-Pflanzenzellen abweichen.

Unter dem Begriff "fremdes Nukleinsäuremolekül/Polynukleotid" bzw. "fremder Nukleinsäuremoleküle/Polynukleotide" ist im Zusammenhang mit der vorliegenden Erfindung ein solches Molekül zu verstehen, das entweder natürlicherweise in entsprechenden Wildtyp-Pflanzenzellen nicht vorkommt, oder das in der konkreten räumlichen Anordnung nicht natürlicherweise in entsprechenden Wildtyp-Pflanzenzellen vorkommt oder das an einem Ort im Genom der Pflanzenzelle lokalisiert ist, an dem es natürlicherweise nicht vorkommt. Bevorzugt ist das fremde Nukleinsäuremolekül/ Polynukleotid ein rekombinantes Molekül, das aus verschiedenen Elementen besteht, deren Kombination oder spezifische räumliche Anordnung natürlicherweise in pflanzlichen Zellen nicht auftritt.

Bei dem bzw. den zur genetischen Modifikation verwendeten fremden Nukleinsäuremolekül(en)/Polynukleotid (en) kann es sich um ein zusammengefügtes oder mehrere getrennte Nukleinsäurekonstrukte handeln, insbesondere so genannte Einfach-, Zweifach-, Dreifach- oder Vierfachkonstrukte. So kann das fremde Nukleinsäuremolekül/Polynukleotid beispielsweise ein so genanntes "Vierfachkonstrukt" sein, worunter man einen einzigen Vektor zur Pflanzentransformation versteht, der sowohl die genetische Information zur Inhibierung der Expression eines oder mehrerer endogener GBSSI-Gene als auch zur Inhibierung der Expression eines oder mehrerer SSIII-Gene als auch zur Inhibierung der Expression eines oder mehrerer BEI-Gene enthält und als auch zur Inhibierung der Genexpression des unter SEQ ID NO 11 oder SEQ ID NO 13 angegebenen Gens oder dessen Vorhandensein bzw. dessen Expression zur Verringerung der GBSSI-, SSIII- und BEI-Aktivitäten und zu einer Verringerung der Genexpression des unter SEQ ID NO 11 oder SEQ ID NO 13 angegebenen Gens führt.

In einer weiteren Ausführungsform kann das fremde Nukleinsäuremolekül/Polynukleotid ein so genanntes "Doppelkonstrukt" sein, worunter man einen Vektor zur Pflanzentransformation versteht, der die genetische Information zur Inhibierung der Expression von zwei der vier Zielgene (GBSSI-, SSIII-, BEI-Gen(e), Gen mit der unter SEQ ID NO 11 oder SEQ ID NO 13 angegebenen Nukleotidsequenz) enthält oder dessen Vorhandensein bzw. dessen Expression zur Verringerung der Aktivität von zwei der vier Enzyme (GBSSI, SSIII, BEI, Protein mit der unter SEQ ID NO 12 oder SEQ ID NO 14 angegebenen Aminosäuresequenz) führt. Die Inhibierung der Expression des dritten und vierten Zielgens und/oder die Verringerung der Aktivität des dritten und vierten Enzyms erfolgt in dieser beispielhaften Ausführungsform der Erfindung mit Hilfe eines gesonderten fremden Nukleinsäuremoleküls/Polynukleolids, das die entsprechende genetische Information zur Inhibierung dieser beiden weiteren Zielgene enthält.

In einer weiteren Ausführungsform der Erfindung wird in das Genom der Pflanzenzelle nicht ein Vierfachkonstrukt, sondern es werden mehrere unterschiedliche fremde Nukleinsäuremoleküle/Polynukleotide eingeführt, wobei eines dieser fremden Nukleinsäuremoleküle beispielsweise ein DNA-Molekül ist, das z.B. ein Cosuppressions-Konstrukt darstellt, das eine Verringerung der Expression von einem oder mehreren endogenen GBSSI-Genen bewirkt und zur Inhibierung der Genexpression des unter SEQ ID NO 11 oder SEQ ID NO 13 angegebenen Gens führt, und ein weiteres fremdes Nukleinsäuremolekül ein DNA-Molekül ist, das z.B. für eine Antisense-RNA codiert, die eine Verringerung der Expression von einem oder mehreren endogenen SSIII- und/oder BEI-Genen bewirkt. Grundsätzlich ist bei der Konstruktion der fremden Nukleinsäuremoleküle aber auch die Verwendung jeder Kombination aus Antisense-, Cosuppressions-, Ribozym- und doppelsträngigen RNA-Konstrukten oder in vivo-Mutagenese geeignet, die zu einer gleichzeitigen Verringerung der Expression endogener Gene führt, die für GBSSI, SSIII und BEI kodieren bzw. die unter SEQ ID NO 11 oder SEQ ID NO 13 angegebenen Nukleotidsequenz aufweisen, oder die zu einer gleichzeitigen Verringerung der GBSSI-, SSIII- und BEI Aktivitäten und zur Inhibierung der Genexpression des unter SEQ ID NO 11 oder SEQ ID NO 13 angegebenen Gens führt.

Die fremden Nukleinsäuremoleküle können hierbei zeitgleich ("Cotransformation") oder auch nacheinander, d.h. zeitlich aufeinanderfolgend ("Supertransformation") in das Genom der Pflanzenzelle eingeführt werden.

Die fremden Nukleinsäuremoleküle/Polynukleotide können auch in verschiedene individuelle Pflanzen einer Spezies eingeführt werden. Es können dabei Pflanzen erzeugt werden, bei welchen die Aktivität von einem Enzym (z.B. GBSSI oder SSIII oder BEI) oder zwei Enzymen (z.B. GBSSI und SSIII bzw. GBSSI und BEI bzw. SSIII und BEI) oder drei Enzymen reduziert ist. Durch anschließendes Kreuzen können dann Pflanzen erzeugt werden, bei welchen die Aktivität aller drei Enzyme (GBSSI, SSIII, BEI) reduziert ist und die Genexpression des unter SEQ ID NO 11 oder SEQ ID NO 13 angegebenen Gens verringert ist.

Weiterhin kann zur Einführung eines fremden Nukleinsäuremoleküls/Polynukleotids bzw. zur Erzeugung der erfindungsgemäßen Pflanzenzellen oder der erfindungsgemäßen Pflanzen anstelle einer Wildtyp-Pflanzenzelle bzw. -Pflanze eine Mutante, die sich dadurch auszeichnet, dass sie bereits eine verminderte Aktivität eines oder mehrerer Enzyme (GBSSI, SSIII, BEI, Protein mit der unter SEQ ID NO 12 oder SEQ ID NO 14 angegebenen Aminosäuresequenz) aufweist, herangezogen werden. Bei den Mutanten kann es sich sowohl um spontan auftretende Mutanten, als auch um solche handeln, die durch den gezielten Einsatz von Mutagenen erzeugt wurden. Möglichkeiten zur Erzeugung von solchen Mutanten sind vorstehend beschrieben worden.

Die erfindungsgemäßen Pflanzenzellen können auch durch die Verwendung der so genannten Insertionsmutagenese (Übersichtsartikel: Thorneycroft et al., 2001, Journal of Experimental Botany 52 (361), 1593-1601) hergestellt werden. Unter "Insertionsmutagenese" ist insbesondere das Inserieren von Transposons oder so genannter Transfer-DNA (T-DNA) in ein Gen kodierend für GBSSI und/oder SSIII und/oder BEI und/oder mit der unter SEQ ID NO 11 oder SEQ ID NO 13 angegebenen Nukleotidsequenz zu verstehen.

Bei den Transposons kann es sich dabei sowohl um solche handeln, die in einer Wildtyp-Pflanzenzelle natürlicherweise vorkommen (endogene Transposons), als auch um solche, die natürlicherweise nicht in besagter Zelle vorkommen, sondern mittels gentechnischer Methoden, wie z.B. Transformation der Zelle, in die Zelle eingeführt werden (heterologe Transposons). Die Veränderung der Expression von Genen mittels Transposons ist dem Fachmann bekannt. Eine Übersicht über die Nutzung von endogenen und heterologen Transposons als Werkzeuge in der Pflanzenbiotechnologie ist von Ramachandran und Sundaresan (2001, Plant Physiology and Biochemistry 39, 234-252) dargestellt. Die Möglichkeit, Mutanten zu identifizieren, bei welchen spezifische Gene durch Transposoninsertionsmutagenese inaktiviert wurden, ist in einer Übersicht von Maes et al. (1999, Trends in Plant Science 4 (3), 90-96) dargestellt. Die Erzeugung von Reis-Mutanten mit Hilfe endogener Transposons ist von Hirochika (2001, Current Opinion in Plant Biology 4, 118-122) beschrieben. Die Identifizierung von Mais-Genen, mit Hilfe endogener Retrotransposons wird z.B. von Hanley et al. (2000, The Plant Journal 22 (4), 557-566) dargestellt. Die Möglichkeit, Mutanten mit Hilfe von Retrotransposons herzustellen und Methoden, Mutanten zu identifizieren, sind von Kumar und Hirochika (2001, Trends in Plant Science 6 (3), 127-134) beschrieben. Die Aktivität von heterologen Transposons in unterschiedlichen Spezies ist sowohl für dikotelydone als auch für monokotyledone Pflanzen beschrieben worden: z.B. für Reis (Greco et al., 2001, Plant Physiology 125, 1175-1177; Liu et al., 1999, Molecular and General Genetics 262, 413-420; Hiroyuki et al., 1999, The Plant Journal 19 (5), 605-613; Jeon und Gynheung, 2001, Plant Science 161, 211-219), Gerste (Koprek et al., 2000, The Plant Journal 24 (2), 253-263), Arabidopsis thaliana (Aarts et al., 1993, Nature 363, 715-717; Schmidt und Willmitzer, 1989, Molecular and General Genetics 220, 17-24; Altmann et al., 1992, Theoretical and Applied Genetics 84, 371-383; Tissier et al., 1999, The Plant Cell 11, 1841-1852), Tomate (Belzile und Yoder, 1992, The Plant Journal 2 (2), 173-179) und Kartoffel (Frey et al., 1989, Molecular and General Genetics 217, 172-177; Knapp et al., 1988, Molecular and General Genetics 213, 285-290).

Grundsätzlich können die erfindungsgemäßen Pflanzenzellen und die erfindungsgemäßen Pflanzen sowohl mit Hilfe homologer als auch heterologer Transposons hergestellt werden, wobei unter Verwendung von homologen Transposons auch solche zu verstehen sind, die bereits natürlicherweise im Pflanzengenom vorhanden sind.

Die T-DNA-Insertionsmutagenese beruht darauf, dass bestimmte Abschnitte (T-DNA) von Ti-Plasmiden aus Agrobacterium in das Genom von pflanzlichen Zellen integrieren können. Der Ort der Integration in das pflanzliche Chromosom ist dabei nicht festgelegt, sondern kann an jeder beliebigen Stelle erfolgen. Integriert die T-DNA in einen Abschnitt des Chromosoms, der eine Genfunktion darstellt, so kann dieses zur Veränderung der Genexpression und damit auch zur Änderung der Aktivität eines durch das betreffende Gen codierten Proteins führen. Insbesondere führt die Integration einer T-DNA in den kodierenden Bereich eines Gens häufig dazu, dass das entsprechende Protein von der betreffenden Zelle gar nicht mehr oder nicht mehr in aktiver Form synthetisiert werden kann. Die Verwendung von T-DNA-Insertionen zur Erzeugung von Mutanten ist z.B. für Arabidopsis thaliana (Krysan et al., 1999, The Plant Cell 11, 2283-2290; Atipiroz-Leehan und Feldmann, 1997, Trends in Genetics 13 (4), 152-156; Parinov und Sundaresan, 2000, Current Opinion in Biotechnology 11, 157-161) und Reis (Jeon und An, 2001, Plant Science 161, 211-219; Jeon et al., 2000, The Plant Journal 22 (6), 561-570) beschrieben. Methoden zur Identifizierung von Mutanten, die mit Hilfe der T-DNA-Insertionsmutagenese erzeugt wurden, sind u.a. beschrieben von Young et al. (2001, Plamt Physiology 125, 513-518), Parinov et al. (1999, The Plant Cell 11, 2263-2270), Thorneycroft et al. (2001, Journal of Experimental Botany 52, 1593-1601 ) und McKinney et al. (1995, The Plant Journal 8 (4),613-622).

Die T-DNA-Mutagenese ist grundsätzlich zur Erzeugung der erfindungsgemäßen Pflanzenzellen und der erfindungsgemäßen Pflanzen geeignet.

In einer weiteren Ausführungsform der vorliegenden Erfindung führt das Vorhandensein und/oder die Expression eines oder mehrerer fremder Nukleinsäuremoleküle/Polynukleotide zur Inhibierung der Expression von endogenen Genen, die für GBSSI und/oder SSIII und/oder BEI kodieren und/oder die unter SEQ ID NO 11 oder SEQ ID NO 13 angegebene Nukleotidsequenz aufweisen.

Dies kann durch verschiedene, dem Fachmann bekannte Verfahren erzielt werden. Hierzu zählen beispielsweise die Expression einer entsprechenden Antisense-RNA, oder eines doppelsträngigen RNA-Konstruktes, die Bereitstellung von Molekülen oder Vektoren, die einen Cosuppressions-Effekt vermitteln, die Expression eines entsprechend konstruierten Ribozyms, das spezifisch Transkripte spaltet, die für GBSSI bzw. SSIII bzw. BEI kodieren, oder die so genannte "in vivo-Mutagenese". Ferner kann die Verringerung der GBSSI- und/oder SSIII- und/oder BEI-Aktivität (en) und/oder die Verringerung der Genexpression des unter SEQ ID NO 11 oder SEQ ID NO 13 angegebenen Gens in den Pflanzenzellen auch durch die simultane Expression von Sense- und Antisense-RNA-Molekülen des jeweiligen zu reprimierenden Zielgens, vorzugsweise des GBSSI- und/oder SSIII- und/oder BEI-Gens und/oder des Gens mit der unter SEQ ID NO 11 oder SEQ ID NO 13 angegebenen Nukleotidsequenz, hervorgerufen werden. Diese Methoden sind dem Fachmann geläufig.

Darüber hinaus ist bekannt, dass in planta die Bildung von doppelsträngigen RNA-Molekülen von Promotorsequenzen in trans zu einer Methylierung und einer transkriptionellen Inaktivierung homologer Kopien dieses Promotors führen kann (Mette et al., 2000, EMBO J. 19, 5194-5201).

Zur Inhibierung der Genexpression mittels Antisense- oder Cosuppressions-Technologie kann beispielsweise ein DNA-Molekül verwendet werden, das die gesamte für GBSSI und/oder SSIII und/oder BEI kodierende Sequenz und/oder die unter SEQ ID NO 11 oder SEQ ID NO 13 angegebene Nukleotidsequenz einschließlich eventuell vorhandener flankierender Sequenzen umfasst, als auch DNA-Moleküle, die nur Teile der kodierenden Sequenz umfassen, wobei diese Teile lang genug sein müssen, um in den Zellen einen Antisense-Effekt bzw. Cosuppressions-Effekt zu bewirken. Geeignet sind im Allgemeinen Sequenzen mit einer Mindestlänge von 15 bp, bevorzugt mit einer Mindestlänge von 20-30 bp, besonders bevorzugt mit einer Länge von 100-500 bp, für eine sehr effiziente Antisense- bzw. Cosuppressions-Inhibition insbesondere Sequenzen mit einer Länge über 500 bp.

Für Antisense- oder Cosuppressions-Ansätze geeignet, ist auch die Verwendung von Polynukleotidsequenzen, die einen hohen Grad an Identität zu den endogen in der Pflanzenzelle vorkommenden Sequenzen haben, die für GBSSI bzw. SSIII bzw. BEI kodieren bzw. die unter SEQ ID NO 11 oder SEQ ID NO 13 dargestellt sind. Die minimale Identität sollte größer als ca. 65% sein. Die Verwendung von Sequenzen mit Identitäten von mindestens 90%, insbesondere zwischen 95 und 100% ist zu bevorzugen.

Ferner ist zur Erzielung eines Antisense- oder eines Cosuppressions-Effekts auch die Verwendung von Introns denkbar, d.h. von nicht kodierenden Bereichen von Genen, die für GBSSI bzw. SSIII bzw. BEI kodieren, bzw. die die unter SEQ ID NO 11 oder SEQ ID NO 13 dargestelle Nukleotidsequenz aufweisen.

Die Verwendung von Intron-Sequenzen zur Inhibierung der Expression von Genen, die für Proteine der Stärkebiosynthese kodieren, wurde beschrieben in WO 97/04112, WO 97/04113, WO 98/37213, WO 98/37214.

Dem Fachmann ist bekannt, wie er einen Antisense- und einen Cosuppressions-Effekt erzielen kann. Das Verfahren der Cosuppressions-Inhibierung wurde beispielsweise beschrieben von Jorgensen (1990, Trends Biotechnol. 8, 340-344), Niebel et al. (1995, Top. Microbiol. Immunol. 197, 91-103), Flavell et al. (1995, Curr. Top. Microbiol. Immunol. 197, 43-46), Palauqui und Vaucheret (1995, Plant Mol. Biol. 29, 149-159), Vaucheret et al. (1995, Mol. Gen. Genet. 248, 311-317), de Borne et al. (1994, Mol.. Gen. Genet. 243, 613-621).

Auch die Expression von Ribozymen zur Verringerung der Aktivität von bestimmten Enzymen in Zellen ist dem Fachmann bekannt und ist beispielsweise beschrieben in EP-B1 0321201. Die Expression von Ribozymen in pflanzlichen Zellen wurde z.B. beschrieben von Feyter et al. (1996, Mol. Gen. Genet. 250, 329-338).

Ferner kann die Verringerung der GBSSI- und/oder SSIII- und/oder BEI-Aktivität(en) und/oder die Verringerung der Genexpression des unter SEQ ID NO 11 oder SEQ ID NO 13 dargestellten Gens in den Pflanzenzellen auch durch die so genannte "in vivo-Mutagenese" erreicht werden, bei der durch Transformation von Zellen ein hybrides RNA-DNA-Oligonukleotid ("Chimeroplast") in Zellen eingeführt wird (Kipp et al., Poster Session beim 5th International Congress of Plant Molecular Biology, 21-27. September 1997, Singapur; R. A. Dixon und C. J. Amtzen, Meeting report zu Metabolic Engineering in Transgenic Plants, Keystone Symposia, Copper Mountain, CO, USA, 1997, TIBTECH 15, 441-447; WO 95/15972; Kren et al., 1997, Hepatology 25, 1462-1468; Cole-Strauss et al., 1996, Science 273, 1386-1389; Beetham et al., 1999, PNAS 96, 8774-8778).

Ein Teil der DNA-Komponente des RNA-DNA-Oligonukleotids ist homolog zu einer Polynukleotidsequenz eines endogenen GBSSI- und/oder SSIII- und/oder BEI-Gens und/oder eines unter SEQ ID NO 11 oder SEQ ID NO 13 dargestellten Gens, weist jedoch im Vergleich zur Polynukleotidsequenz eines endogenen GBSSI- bzw. SSIII- bzw. BEI-Gens eine Mutation auf oder enthält eine heterologe Region, die von den homologen Regionen umschlossen ist. Durch Basenpaarung der homologen Regionen des RNA-DNA-Oligonukleotids und des endogenen Polynukleotids, gefolgt von homologer Rekombination, kann die in der DNA-Komponente des RNA-DNA-Oligonukleotids enthaltene Mutation oder heterologe Region in das Genom einer Pflanzenzelle übertragen werden. Dies führt zu einer Verringerung der Aktivität von GBSSI und/oder SSIII und/oder BEI und/oder zur Verringerung der Genexpression des unter SEQ ID NO 11 oder SEQ ID NO 13 angegebenen Gens.

Ferner kann die Verringerung der GBSSI- und/oder SSIII- und/oder BEI-Aktivität(en) in den Pflanzenzellen auch durch die simultane Expression von Sense- und Antisense-RNA-Molekülen des jeweiligen zu reprimierenden Zielgens, vorzugsweise des GBSSI- und/oder SSIII- und/oder BEI-Gens und/oder des unter SEQ ID NO 11 oder SEQ ID NO 13 angegebenen Gens, hervorgerufen werden.

Dies kann beispielsweise durch die Verwendung von chimären Konstrukten erreicht werden, die "inverted repeats" des jeweiligen Zielgens oder Teilen des Zielgens enthalten. Hierbei kodieren die chimären Konstrukte für Sense- und Antisense-RNA-Moleküle des jeweiligen Zielgens. Sense- und Antisense-RNA werden in planta gleichzeitig als ein RNA-Molekül synthetisiert, wobei Sense- und Antisense-RNA durch einen Spacer voneinander getrennt sein und ein doppelsträngiges RNA-Molekül bilden können (RNAi-Technologie).

Es konnte gezeigt werden, dass die Einführung von inverted-repeat-DNA-Konstrukten in das Genom von Pflanzen eine sehr effiziente Methode ist, um die zu den inverted-repeat-DNA-Konstrukten korrespondierenden Gene zu reprimieren (Waterhouse et al., 1998, Proc. Natl. Acad. Sci. USA 95, 13959-13964; Wang und Waterhouse, 2000, Plant Mol. Biol. 43, 67-82; Singh et al., 2000, Biochemical Society Transactions 28 (6), 925- 927; Liu et al., 2000, Biochemical Society Transactions 28 (6), 927-929; Smith et al., 2000, Nature 407, 319-320; WO 99/53050). Sense- und Antisense-Sequenzen des Zielgens bzw. der Zielgene können auch getrennt voneinander mittels gleicher oder unterschiedlicher Promotoren exprimiert werden (Nap et al, 6th International Congress of Plant Molecular Biology, 18.-24. Juni 2000, Quebec, Poster S7-27, Vortrag Session S7).

Die Verringerung der GBSSI- und/oder SSIII- und/oder BEI-Aktivität(en) und die Verringerung der Genexpression des unter SEQ ID NO 11 oder SEQ ID NO 13 angegebenen Gens.in den Pflanzenzellen kann somit auch durch die Erzeugung doppelsträngiger RNA-Moleküle von GBSSI- bzw. SSIII- bzw. BEI-Genen bzw. Genen mit der unter SEQ ID NO 11 oder SEQ ID NO 13 dargestellten Nukleotidsequenz erreicht werden. Vorzugsweise werden hierzu "inverted repeats" von DNA-Molekülen, die von GBSSI- bzw. SSIII bzw. BEI-Genen bzw. Genen mit der unter SEQ ID NO 11 oder SEQ ID NO 13 dargestellten Nukleotidsequenz oder -cDNAs abgeleitet sind, in das Genom von Pflanzen eingeführt, wobei die zu transkribierenden DNA-Moleküle unter Kontrolle eines Promotors stehen, der die Expression besagter DNA-Moleküle steuert.

Darüber hinaus ist bekannt, dass die Bildung von doppelsträngigen RNA-Molekülen von Promotor-DNA-Molekülen in Pflanzen in trans zu einer Methylierung und einer transkriptionellen Inaktivierung homologer Kopien dieser Promotoren führen kann, die im Folgenden als Zielpromotoren bezeichnet werden sollen (Mette et al., 2000, EMBO J. 19, 5194-5201).

Über die Inaktivierung des Zielpromotors ist es somit möglich, die Genexpression eines bestimmten Zielgens (z.B. GBSSI-, SSIII-, BEI-Gen; Gen mit der unter SEQ ID NO 11 oder SEQ ID NO 13 dargestellten Nukleotidsequenz), das natürlicherweise unter der Kontrolle dieses Zielpromotors steht, zu verringern.

D.h., die DNA-Moleküle, die die Zielpromotoren der zu reprimierenden Gene (Zielgene) umfassen, werden in diesem Fall, im Gegensatz zur ursprünglichen Funktion von Promotoren in Pflanzen, nicht als Steuerelemente zur Expression von Genen oder cDNAs, sondern selbst als transkribierbare DNA-Moleküle verwendet.

Zur Erzeugung der doppelsträngigen Zielpromotor-RNA-Moleküle in planta, die dort als RNA-Haamadel-Moleküle ("RNA hairpin") vorliegen können, werden vorzugsweise Konstrukte verwendet, die "inverted repeats" der Zielpromotor-DNA-Moleküle enthalten, wobei die Zielpromotor-DNA-Moleküle unter Kontrolle eines Promotors stehen, der die Genexpression besagter Zielpromotor-DNA-Moleküle steuert. Anschließend werden diese Konstrukte in das Genom von Pflanzen eingeführt. Die Expression der "inverted repeats" besagter Zielpromotor-DNA-Moleküle führt in planta zur Bildung doppelsträngiger Zielpromotor-RNA-Moleküle (Mette et al., 2000, EMBO J. 19, 5194-5201). Hierdurch kann der Zielpromotor inaktiviert werden.

Die Verringerung der GBSSI- und/oder SSIII- und/oder BEI-Aktivität(en) und die Inhibierung der Genexpression des unter SEQ ID NO 11 oder SEQ ID NO 13 angegebenen Gens in den Pflanzenzellen kann somit auch durch die Erzeugung doppelsträngiger RNA-Moleküle von Promotorsequenzen von GBSSI- bzw. SSIII- bzw. BEI-Genen bzw. von Genen mit der unter SEQ ID NO 11 oder SEQ ID NO 13 dargestellten Nukleotidsequenz erreicht werden. Vorzugsweise werden hierzu "inverted repeats" von Promotor-DNA-Molekülen von GBSSI- und/oder SSIII- und/oder BEI-Promotoren in das Genom von Pflanzen eingeführt, wobei die zu transkribierenden Zielpromotor-DNA-Moleküle (GBSSI-, SSIII-, BEI-Promotor) unter Kontrolle eines Promotors stehen, der die Expression besagter Zielpromotor-DNA-Moleküle steuert.

Ferner ist dem Fachmann bekannt, dass er eine Verringerung der GBSSI- und/oder SSIII- und/oder BEI-Aktivität (en) und die Inhibierung der Genexpression des unter SEQ ID No. SEQ ID NO 11 oder SEQ ID NO 13 angegebenen Gens durch die Expression von nicht funktionellen Derivaten, insbesondere trans-dominanten Mutanten, der Enzyme und/oder durch die Expression von Antagonisten/Inhibitoren der Enzyme erreichen kann.

Antagonisten/Inhibitoren der Enzyme können beispielsweise Antikörper, Antikörperfragmente oder Moleküle mit ähnlichen Bindungseigenschaften sein. Beispielsweise wurde ein cytoplasmatischer scFv-Antikörper eingesetzt, um die Aktivität des Phytochrom A-Proteins in gentechnisch veränderten Tabakpflanzen zu modulieren (Owen, 1992, Bio/Technology 10, 790-794; Review: Franken et al., 1997, Current Opinion in Biotechnology 8, 411-416; Whitelam, 1996, Trends Plant Sci. 1, 268-272).

Generell kommt für die Expression des fremden Nukleinsäuremoleküls/Polynukleotids (der fremden Nukleinsäuremoleküle/Polynukleotide) jeder in pflanzlichen Zellen aktive Promotor in Frage. Der Promotor kann dabei so gewählt sein, dass die Expression in den erfindungsgemäßen Pflanzen konstitutiv erfolgt oder nur in einem bestimmten Gewebe, zu einem bestimmten Zeitpunkt der Pflanzenentwicklung oder zu einem durch äußere Einflüsse determinierten Zeitpunkt.

In bezug auf die Pflanze kann der Promotor homolog oder heterolog sein.

Sinnvolle Promotoren für die Expression von Nukleinsäuren/Polynukleotiden, die die Aktivität eines Zielgens verringern, sind z.B. der Promotor der 35S RNA des Cauliflower Mosaic Virus und der Ubiquitin-Promotor aus Mais für eine konstitutive Expression, der Patatingen-Promotor B33 (Rocha-Sosa et al., 1989, EMBO J. 8, 23-29), der MCPI-Promotor des Metallocarboxypeptidase-Inhibitor-Gens aus Kartoffel (HU 9801674) oder der GBSSI-Promotor aus Kartoffel (WO 92/11376) für eine knollenspezifische Expression in Kartoffeln oder ein Promotor, der eine Expression lediglich in photosynthetisch aktiven Geweben sicherstellt, z.B. der ST-LS1-Promotor (Stockhaus et al., 1987, Proc. Natl. Acad. Sci. USA 84, 7943-7947; Stockhaus et al., 1989, EMBO J. 8, 2445-2451), der Ca/b-Promotor (siehe beispielsweise US 5,656,496; US 5,639,952; Bansal et al., 1992, Proc. Natl. Acad. Sci. USA 89, 3654-3658) und der Rubisco SSU-Promotor (siehe beispielsweise US 5,034,322; US 4,962,028) oder für eine Endosperm-spezifische Expression der Gluteün-Promotor (Leisy et al., 1990, Plant Mol. Biol. 14, 41-50; Zheng et al., 1993, Plant J. 4, 357-366; Yoshihara et al., 1996, FEBS Lett. 383, 213-218), der Shrunken-1 Promotor (Werr et al., 1985, EMBO J. 4, 1373-1380), der HMG-Promotor aus Weizen, der USP-Promotor, der Phaseolin-Promotor oder Promotoren von Zein-Genen aus Mais (Pedersen et al., 1982, Cell 29, 1015-1026; Quatroccio et al., 1990, Plant Mol. Biol. 15, 81-93).

Bevorzugte Promotoren für die Expression des fremden Nukleinsäuremoleküls/Polynukleotids (der fremden Nukleinsäuremoleküle/Polynukleotide) sind der Patatingen-, der MCPI- und der GBSSI-Promotor aus Kartoffel.

Die Expression des fremden Nukleinsäuremoleküls/Polynukleotids (der fremden Nukleinsäuremoleküle/Polynukleotide) ist insbesondere in solchen Organen der Pflanze von Vorteil, die Stärke speichern. Solche Organe sind z.B. die Knolle der Kartoffelpflanze oder die Körner bzw. das Endosperm von Mais-, Weizen- oder Reis-Pflanzen. Bevorzugt werden daher Promotoren verwendet, die die Expression in diesen Organen vermitteln.

Es können jedoch auch Promotoren verwendet werden, die nur zu einem durch äußere Einflüsse determinierten Zeitpunkt aktiviert werden (siehe beispielsweise WO 93/07279). Von besonderem Interesse können hierbei Promotoren von heat-shock-Proteinen sein, die eine einfache Induktion erlauben. Ferner können samenspezifische Promotoren, wie z.B. der USP-Promoter aus Vicia faba, der eine samenspezifische Expression in Vicia faba und anderen Pflanzert gewährleistet (Fiedler et al., 1993, Plant Mol. Biol. 22, 669-679; Bäumlein et al., 1991, Mol. Gen. Genet. 225, 459-467) und auch fruchtspezifische Promotoren eingesetzt werden, wie z.B. beschrieben in WO 91/01373.

Weiterhin kann eine Terminationssequenz vorhanden sein, die der korrekten Beendigung der Transkription dient sowie der Addition eines Poly-A-Schwanzes an das Transkript, dem eine Funktion bei der Stabilisierung der Transkripte beigemessen wird. Derartige Elemente sind in der Literatur beschrieben (siehe beispielsweise Gielen et al., 1989, EMBO J. 8, 23-29) und sind beliebig austauschbar.

Für die Einführung von DNA in eine pflanzliche Wirtszelle steht eine Vielzahl von Techniken zur Verfügung. Diese Techniken umfassen die Transformation pflanzlicher Zellen mit T-DNA unter Verwendung von Agrobacterium tumefaciens oder Agrobacterium rhizogenes als Transformationsmittel, die Fusion von Protoplasten, die Injektion, die Elektroporation von DNA, die Einbringung der DNA mittels des biolistischen Ansatzes sowie weitere Möglichkeiten.

Die Verwendung der Agrobakterien-vermittelten Transformation von Pflanzenzellen ist intensiv untersucht und ausreichend in EP-A 0120516 und von Hoekema (1985, The Binary Plant Vector System Offsetdrukkerij Kanters B.V., Alblasserdam, Chapter V), Fraley et al. (Crit. Rev. Plant Sci. 4, 1-46) und An et al. (1985, EMBO J. 4, 277-287) beschrieben worden. Für die Transformation von Kartoffel, siehe beispielsweise Rocha-Sosa et al., 1989, EMBO J. 8, 29-33.

Auch die Transformation monokotyledoner Pflanzen mittels auf Agrobakterium-Transformation basierender Vektoren wurde beschrieben (Chan et al., 1993, Plant Mol. Biol. 22, 491-506; Hiei et al., 1994, Plant J. 6, 271-282; Deng et al., 1990, Science in China 33, 28-34; Wilmink et al., 1992, Plant Cell Reports 11, 76-80; May et al., 1995, Bio/Technology 13, 486-492; Conner und Domisse, 1992, Int. J. Plant Sci. 153, 550-555, Ritchie et al, 1993, Transgenic Res. 2, 252-265). Ein alternatives System zur Transformation von monokotyledonen Pflanzen ist die Transformation mittels des biolistischen Ansatzes (Wan und Lemaux, 1994, Plant Physiol. 104, 37-48; Vasil et al., 1993, Bio/Technology 11, 1553-1558; Ritala et al., 1994, Plant Mol. Biol. 24, 317-325; Spencer et al., 1990, Theor. Appl. Genet. 79, 625-631), die Protoplasten-Transformation, die Elektroporation von partiell permeabilisierten Zellen, die Einbringung von DNA mittels Glasfasern. Insbesondere die Transformation von Mais wird in der Literatur mehrfach beschrieben (siehe beispielsweise WO 95/06128, EP-A 0513849, EP-A 0465875, EP-A 0292435; Fromm et al., 1990, Biotechnology 8, 833-844; Gordon-Kamm et al., 1990, Plant Cell 2, 603-618; Koziel et al., 1993, Biotechnology 11, 194-200; Moroc et al., 1990, Theor. Appl. Genet. 80, 721-726).

Auch die erfolgreiche Transformation anderer Getreidearten wurde bereits beschrieben, z.B. für Gerste (Wan und Lemaux, supra; Ritala et al., supra; Krens et al., 1982, Nature 296, 72-74) und für Weizen (Nehra et al., 1994, Plant J. 5, 285-297).

Gegenstand der vorliegenden Erfindung ist auch eine Pflanzenzelle, die genetisch modifiziert ist, wobei die genetische Modifikation zur Verringerung der GBSSI-, SSIII- und BEI-Aktivitäten und zur Inhibierung der Genexpression des unter SEQ ID NO 11 oder SEQ ID NO 13 angegebenen Gens führt im Vergleich zu denjenigen entsprechender Wildtyp-Pflanzenzellen bzw. Wildtyp-Pflanzen, und die zumindest eine fremde Nukleinsäure enthält, die ausgewählt ist aus der Gruppe bestehend aus

a) Polynukleotiden, die für mindestens eine Antisense-RNA kodieren, welche zu einer Verringerung der Expression von mindestens einem endogenen GBSSI-Gen und/oder zu einer Verringerung der Expression von mindestens einem endogenen SSIII-Gen und/oder zu einer Verringerung der Expression von mindestens einem endogenen BEI-Gen und/oder zu einer Verringerung der Expression von mindestens einem Gen mit der unter SEQ ID NO 11 oder SEQ ID NO 13 angegebenen Nukleotidsequenz führt;

b) Polynukleotiden, die über einen Cosuppressions-Effekt zu einer Verringerung der Expression von mindestens einem endogenen GBSSI-Gen und/oder zu einer Verringerung der Expression von mindestens einem endogenen SSIII-Gen und/oder zu einer Verringerung der Expression von mindestens einem endogenen BEI-Gen und/oder zu einer Verringerung der Expression von mindestens einem Gen mit der unter SEQ ID No. 11 oder SEQ ID No. 13 angegebenen Nukleotidsequenz führt;

c) Polynukleotiden, die für mindestens ein Ribozym kodieren, das spezifisch Transkripte von mindestens einem endogenen GBSSI-Gen und/oder von mindestens einem SSIII-Gen und/oder von mindestens einem BEI-Gen und/oder von mindestens einem Gen mit der unter SEQ ID No. 11 oder SEQ ID No. 13 angegebenen Nukleotidsequenz spaltet;

d) mittels in vivo-Mutagenese eingeführte Polynukleotide, die zu einer Mutation oder einer Insertion in mindestens einem endogenen GBSSI-Gen und/oder zu einer Mutation oder einer Insertion in mindestens einem endogenen SSIII-Gen und/oder zu einer Mutation oder einer Insertion in mindestens einem endogenen BEI-Gen und/oder zu einer Mutation oder einer Insertion in mindestens einem Gen mit der unter SEQ ID NO 11 oder SEQ ID NO 13 angegebenen Nukleotidsequenz führen, wobei die Mutation oder Insertion zu eine Verringerung der Expression des besagten Gens oder zur Synthese von inaktiver GBSSI und/oder von inaktiver SSIII und/oder von inaktivem BEI und/oder von einem inaktiven Protein mit der unter SEQ ID NO 12 oder SEQ ID NO 14 angegebenen Aminosäuresequenz führt;

e) Polynukleotiden, die für mindestens eine Antisense-RNA und mindestens eine Sense-RNA kodieren, wobei besagte Antisense-RNA und besagte Sense-RNA ein doppelsträngiges RNA-Molekül ausbilden können, das zu einer Verringerung der Expression von mindestens einem endogenen GBSSI-Gen und/oder zu einer Verringerung der Expression von mindestens einem endogenen SSIII-Gen und/oder zu einer Verringerung der Expression von mindestens einem endogenen BEI-Gen und/oder zu einer Verringerung der Expression von mindestens einem Gen mit der unter SEQ ID No. 11 oder SEQ ID No. 13 angegebenen Nukleotidsequenz führt;

f) Polynukleotiden, die Transposons enthalten, wobei die Integration der Transposonsequenzen zu einer Mutation oder einer Insertion in mindestens einem endogenen GBSSI-Gen und/oder zu einer Mutation oder einer Insertion in mindestens einem endogenen SSIII-Gen und/oder zu einer Mutation oder einer Insertion in mindestens einem endogenen BEI-Gen und/oder zu einer Mutation oder einer Insertion in mindestens einem Gen mit der unter SEQ ID NO 11 oder SEQ ID NO 13 angegebenen Nukleotidsequenz führt, wobei die Mutation oder Insertion zu eine Verringerung der Expression des besagten Gens oder zur Synthese von inaktiver GBSSI und/oder von inaktiver SSIII und/oder von inaktivem BEI und/oder von einem inaktiven Protein mit der unter SEQ ID NO 12 oder SEQ ID NO 14 angegebenen Aminosäuresequenz führt; und

g) T-DNA-Molekülen, die durch Insertion in mindestens einem endogenen GBSSI-Gen und/oder durch Insertion in mindestens einem endogenen SSIII-Gen und/oder durch Insertion in mindestens einem endogenen BEI-Gen und/oder durch Insertion in mindestens einem Gen mit der unter SEQ ID NO 11 oder SEQ ID NO 13 angegebenen Nukleotidsequenz zu einer Verringerung der Expression des besagten Gens oder zur Synthese von inaktiver GBSSI und/oder von inaktiver SSIII und/oder von inaktivem BEI und/oder von einem inaktiven Protein mit der unter SEQ ID NO 12 oder SEQ ID NO 14 angegebenen Aminosäuresequenz führen.

Gegenstand der vorliegenden Erfindung ist auch Vermehrungsmaterial jeglicher Art von erfindungsgemäßen Pflanzen.

Die erfindungsgemäßen Pflanzenzellen können zur Regeneration ganzer Pflanzen verwendet werden.

Die durch Regeneration der erfindungsgemäßen Pflanzenzellen erhältlichen Pflanzen sind ebenfalls Gegenstand der vorliegenden Erfindung.

Die erfindungsgemäßen Pflanzen bzw. die erfindungsgemäßen Pflanzenzellen können zu jeder beliebigen Pflanzenspezies gehören, d.h. sowohl zu monokotyledonen als auch zu dikotyledonen Pflanzen. Bevorzugt handelt es sich um landwirtschaftliche Nutzpflanzen, d.h. Pflanzen, die vom Menschen kultiviert werden für Zwecke der Ernährung oder für technische, insbesondere industrielle Zwecke und deren Zellen. Vorzugsweise betrifft die Erfindung faserbildende (z.B. Flachs, Hanf, Baumwolle), ölspeichemde (z.B. Raps, Sonnenblume, Sojabohne), zuckerspeichemde (z.B. Zuckerrübe, Zuckerrohr, Zuckerhirse) und proteinspeichemde Pflanzen (z.B. Leguminosen) und deren Zellen.

In einer weiteren bevorzugten Ausführungsform betrifft die Erfindung Futterpflanzen, insbesondere Futter- und Weidegräser (Alfalfa, Klee etc.) und Gemüsepflanzen (z.B. Tomate, Salat, Chicoree) und deren Zellen.

In einer weiteren bevorzugten Ausführungsform betrifft die Erfindung stärkespeichemden Pflanzen (z.B. Weizen, Gerste, Hafer, Roggen, Kartoffel, Mais, Reis, Erbse, Maniok), besonders bevorzugt Kartoffeln, und deren Zellen.

Der Begriff "Kartoffelpflanze" oder "Kartoffel" meint im Zusammenhang mit der vorliegenden Erfindung Pflanzenspezies der Gattung *Solanum,* besonders Knollen produzierende Spezies der Gattung *Solanum* und insbesondere *Solanum tuberosum.*

Ein weiterer Gegenstand der vorliegenden Erfindung betrifft ein Verfahren zur Herstellung einer erfindungsgemäßen Pflanze, worin

a) eine Pflanzenzelle genetisch modifiziert wird, wobei die genetische Modifikation in der Einführung eines oder mehrerer fremder Nukleinsäuremoleküle, deren Vorhandensein und/oder Expression zur Verringerung der GBSSI- und/oder SSIII- und/oder BEI-Aktivitäten und zu einer Verringerung der Genexpression des unter SEQ ID NO 11 oder SEQ ID NO 13 angegebenen Gens führt im Vergleich zu denjenigen in entsprechenden Wildtyp-Pflanzenzellen,
b) aus Pflanzenzellen von Schritt a) eine Pflanze regeneriert wird; und
c) gegebenenfalls weitere Pflanzen mit Hilfe der Pflanzen nach Schritt b) erzeugt werden.

Für die laut Schritt a) in die Pflanzenzelle eingeführte genetische Modifikation gilt, dass es sich grundsätzlich um jede Art von Modifikation handeln kann, die zur Verringerung der Aktivität eines oder mehrerer endogen in der Pflanze vorkommenden SSIII-Proteine und eines oder mehrerer endogen in der Pflanze vorkommenden BEI-Proteine und eines oder mehrerer endogen in der Pflanze vorkommenden GBSSI-Proteine und eines oder mehrerer endogen in der Pflanze vorkommenden Proteine, die mindestens eine 80%, vorzugsweise eine 90%, besonders bevorzugt eine 95% Identität mit der unter SEQ ID NO 12 oder SEQ ID NO 14 aufweisen, führt.

Die Regeneration der Pflanzen gemäß Schritt (b) kann nach dem Fachmann bekannten Methoden erfolgen (z.B. beschrieben in "Plant Cell Culture Protocols", 1999, edt. by R.D. Hall, Humana Press, ISBN 0-89603-549-2).

Die Erzeugung weiterer Pflanzen gemäß Schritt (c) des erfindungsgemäßen Verfahrens kann z.B. erfolgen durch vegetative Vermehrung (beispielsweise über Stecklinge, Knollen oder über Calluskultur und Regeneration ganzer Pflanzen) oder durch sexuelle Vermehrung. Die sexuelle Vermehrung findet dabei vorzugsweise kontrolliert statt, d.h. es werden ausgewählte Pflanzen mit bestimmten Eigenschaften miteinander gekreuzt und vermehrt. Die Auswahl erfolgt dabei bevorzugt in der Weise, dass die weiteren Pflanzen, die nach Schritt c) erhalten werden, die genetische Modifikation, die in Schritt a) eingeführt wurde, aufweisen.

Der Offenbarungsgehalt sämtlicher in der Patentanmeldung zitierter Dokumente soll in den Offenbarungsgehalt der vorliegenden Beschreibung der Erfindung eingeschlossen werden.

**Allgemeine Methoden**

In den Beispielen wurden die folgenden Methoden verwendet. Diese Methoden sollen auch im Zusammenhang mit der vorliegenden Erfindung eingesetzt werden:

<u>1. Verfahren zum Extrahieren von Stärke aus Kartoffeln</u>

Alle Knollen einer Linie (4 bis 5 kg) werden gemeinsam in einem handelsüblichen Entsafter (Multipress automatic MP80, Braun) aufgearbeitet. Das stärkehaltige Fruchtwasser wird in einem 10 L Eimer (Verhältnis Höhe des Eimers/ Durchmesser des Eimers = ca. 1,1), in dem 200 ml Leitungswasser mit einer Löffelspitze (ca. 3-4 g) Natrium-Disulfit vorgelegt wurden, aufgefangen. Im Anschluss wird der Eimer mit Leitungswasser vollständig aufgefüllt. Nach einem 2 stündigen Absetzen der Stärke wird der Überstand abdekantiert, die Stärke erneut in 101 Leitungswasser aufgeschwemmt und über ein Sieb mit 125 μm Maschenweite gegeben. Nach 2 Stunden (Stärke hat sich erneut am Boden des Eimers abgesetzt) wird erneut der wässrige Überstand dekantiert. Dieser Waschvorgang wird noch 3 mal wiederholt, so dass die Stärke insgesamt fünf Mal in frischem Leitungswasser resuspendiert wird. Im Anschluss werden die Stärken bei 37 °C auf einen Wassergehalt von 12 - 17% getrocknet und im Mörser homogenisiert. Die Stärken stehen nun für Analysen zu Verfügung.

<u>2. Stärkeanalytik</u>

a) Bestimmung des Amylose/Amylopektin-Verhältnisses

Stärke wurde, wie oben beschrieben, aus Kartoffelpflanzen isoliert, und das Verhältnis Amylose zu Amylopektin wurde nach der von Hovenkamp-Hermelink et al. (1988, Potato Research 31, 241-246) beschriebenen Methode bestimmt.

b) Bestimmung des Phosphatgehaltes in C6-Position

In der Stärke können die Positionen C2, C3 und C6 der Glukoseeinheiten phosphoryliert sein. Zur Bestimmung des C6-P-Gehaltes der Stärke wurden 50 mg Stärke in 500 µl 0,7 M HCl 4 h bei 95°C hydrolysiert. Anschließend wurden die Ansätze für 10 min bei 15500 g zentrifugiert und die Überstände abgenommen. Von den Überständen wurden 7 µl mit 193 µl Imidazol-Puffer (100 mM Imidazol, pH 6,9; 5 mM $MgCl_2$, 1 mM EDTA und 0,4 mM NAD) gemischt. Die Messung wurde im Photometer bei 340 nm durchgeführt. Nach der Etablierung einer Basisabsorption wurde die Enzymreaktion durch die Zugabe von 2U Glukose-6-Phosphat-Dehydrogenase (von Leuconostoc mesenteroides, Boehringer Mannheim) gestartet. Die Absorptionsänderung ist direkt proportional zur Konzentration des G-6-P-Gehaltes der Stärke.

c) Bestimmung der Viskositätseigenschaften mittels eines Rapid Visco Analyzers (RVA):

Die Bestimmung der Viskositätseigenschaften wurde in Anlehnung an die Methode, welche in WO 01/19975 beschrieben wird, durchgeführt.

2 g Stärke (TS) wurden in 25 ml $H_2O$ (VE Wasser, Leitfähigkeit von mindestens 15 mega Ohm) aufgenommen und für die Bestimmung der Viskositätseigenschaften in einem Rapid Visco Analyser (Newport Scientific Pty Ltd., Investmet Support Group, Warriewod NSW 2102, Australien) analysiert. Der Betrieb des Gerätes erfolgte nach den Angaben des Herstellers. Zur Bestimmung der Viskosität der wässrigen Lösung der Stärke wurde die Stärkesuspension zunächst für 10 Sekunden mit 960 UpM (Umdrehungen pro Minute) gerührt, um anschließend bei einer Rührgeschwindigkeit von 160 UpM für 1 min auf 50°C erhitzt (Schritt 1) zu werden. Danach erfolgte bei gleich bleibender Rührgeschwindigkeit eine Erhöhung der Temperatur von 50°C auf 95°C mit einer Heizgeschwindigkeit von 12°C pro min (Schritt 2). Anschließend wurde die Temperatur bei gleich bleibender Rührgeschwindigkeit für 2,5 min bei 95°C gehalten (Schritt 3). Danach wurde die Lösung bei gleich bleibender Rührgeschwindigkeit von 95°C auf 50°C abgekühlt, mit einer Heizgeschwindigkeit von 12°C pro min (Schritt 4). Der letzte Schritt (Schritt 5) hält die Temperatur von 50°C bei gleich bleibender Rührgeschwindigkeit für 2 min. Während der gesamten Dauer wurde die Viskosität bestimmt.

Nach Beendigung des Programms wurde der Rührer entfernt und der Becher abgedeckt. Die verkleisterte Stärke stand nun für die Texturanalyse nach 24 h zur Verfügung.

Im Profil der RVA-Analyse gibt es charakteristische Werte, die zum Vergleich unterschiedlicher Messungen und Substanzen dargestellt werden. Die folgenden Begriffe sind im Zusammenhang mit der vorliegenden Erfindung wie folgt zu verstehen:

Maximale Viskosität (RVA Max):

Unter der maximalen Viskosität versteht man den höchsten Viskositätswert, gemessen in cP (Centipoise), der im Schritt 2 oder 3 des Temperaturprofils erreicht wird.

Minimale Viskosität (RVA Min):

Unter der minimalen Viskosität versteht man den geringsten Viskositätswert, gemessen in cP, der nach der Maximalen Viskosität im Temperaturprofil auftritt. Normalerweise erfolgt dieses in Schritt 3 des Temperaturprofils.

Finale Viskosität (RVA Fin):

Unter der Finalen Viskosität versteht man den Viskositätswert, gemessen in cP, der am Ende der Messung auftritt.

Setback (RVA Set):

Der so genannte "Setback" wird berechnet, indem man den Wert der Minimalen Viskosität von der Finalen Viskosität subtrahiert.

Verkleisterungstemperatur (RVA PT):

Unter der Verkleisterungstemperatur versteht man die Zeit im Temperaturprofil, zu welcher die Viskosität zuerst um 55 cP über eine Periode von 20 sec ansteigt.

d) Bestimmung der Gelfestigkeit (Texture Analyser)

2 g Stärke (TS) wurden in 25 ml einer wässrigen Suspension im RVA-Gerät verkleistert (Temperaturprogramm: siehe unter c) "Bestimmung der Viskositätseigenschaften mittels eines Rapid Visco Analyzers (RVA)") und anschließend für 24 h bei Raumtemperatur in einem geschlossenen Gefäß gelagert.

Die Proben wurden unter der Sonde (zylindrischer Stempel mit planer Oberfläche) eines Texture Analysers TA-XT2 der Firma Stable Micro Systems (Surrey, UK) fixiert und die Gelfestigkeit mit folgenden Parametern bestimmt:

| | |
|---|---|
| - Test-Geschwindigkeit | 0,5 mm/sec |
| - Eindringtiefe | 7 mm |
| - Kontaktfläche | 113 $mm^2$ |
| - Druck | 2 g |

e) Analyse der Seitenkettenverteilung des Amylopektins mittels Ionenaustauschchromatographie)

Zur Trennung von Amylose und Amylopektin wurden 200 mg Stärke in 50 ml-Reaktionsgefäßen mit 12 ml 90% (v/v) DMSO in $H_2O$ gelöst. Nach Zugabe von 3 Volumen Ethanol wurde das Präzipitat durch 10-minütige Zentrifugation bei etwa 1800 g bei Raumtemperatur (RT) abgetrennt. Das Pellet wurde dann mit 30 ml Ethanol gewaschen, getrocknet und in 40 ml 1% (w/v) NaCl-Lösung bei 75°C gelöst. Nach Abkühlung der Lösung auf 30°C wurden langsam etwa 90 mg Thymol zugegeben und diese Lösung für mindestens 60 h bei 30°C inkubiert. Anschließend wurde die Lösung für 30 min bei 2000 g (RT) zentrifugiert. Der Überstand wurde mit 3 Volumen Ethanol versetzt und das ausfallende Amylopektin mittels 5-minütiger Zentrifugation bei 2000 g (RT) abgetrennt. Das Pellet (Amylopektin) wurde dann mit Ethanol gewaschen und mittels Aceton getrocknet. Anschließend wurde eine 1% Amylopektin-Lösung in 10 mM Natriumacetat, pH 3,5 hergestellt, wobei das Amylopektin bei 65-95°C für 1-2 h gelöst wurde. Von diese Lösung wurden jeweils 100 μl für den Verdau mit 180 μl 10 mM Natriumacetat, pH 3,5 und 1 μl Isoamylase (Megazyme) versetzt und bei 37°C für etwa 16 h inkubiert. Eine wässrige 1:5 Verdünnung dieses Verdaus wurde anschließend mit einem 0,2 μm Filter filtriert und 100 μl des Filtrats ionenchromatographisch analysiert (HPAEC-PAD, Dionex). Die Separation erfolgte mit einer PA-100 Säule (mit entsprechender Vorsäule), die Detektion amperometrisch.

Die Elutionsbedingungen waren wie folgt:
Lösung A - 0, 15 M NaOH
Lösung B - 1 M Natriumacetat in 0,15 M NaOH

| t (min) | Lösung A (%) | Lösung B (%) |
|---|---|---|
| 5 | 0 | 100 |
| 35 | 30 | 70 |
| 45 | 32 | 68 |
| 60 | 100 | 0 |
| 70 | 100 | 0 |
| 72 | 0 | 100 |
| 80 | 0 | 100 |
| stopp | | |

Die Bestimmung des relativen Anteils an kurzen Seitenketten am Gesamtanteil aller Seitenketten erfolgte über die Bestimmung des prozentualen Anteils einer bestimmten Seitenkette am Gesamtanteil aller Seitenketten. Der Gesamtanteil aller detektierbaren Seitenketten wurde ermittelt über die Bestimmung der Gesamtfläche unter den Peaks, die im HPLC-Chromatogramm die Polymerisationsgrade von DP6 bis 34 repräsentieren.

Der prozentuale Anteil einer bestimmten Seitenkette am Gesamtanteil aller Seitenketten wurde ermittelt über die Bestimmung des Verhältnisses der Fläche des Peaks, der diese Seitenkette im HPLC-Chromatogramm repräsentiert, zur Gesamtfläche. Zur Bestimmung der Peakflächen wurde das Programm Chromelion 6.20 Version 6.20 der Firma Dionex, USA, verwendet.

f) Analyse der Seitenkettenverteilung von Gesamtstärke mittels Gelpermeationschromatographie

Zur Bestimmung der Seitenkettenverteilung von Gesamtstärke mittels Gelpermeationschromatographie wurden 10 mg Stärke in 250 µl 90% (v/v) DMSO bei 60°C für ca. 3 h gelöst. Nach Zugabe von 375 µl $H_2O$ (dest.) wurde die Lösung ca. 1 h auf 95°C erhitzt.

Für den enzymatischen Verdau der Stärke wurden 200 µl Stärkelösung zu 300 µl 16,6 mM Natriumacetat, pH 3,5 gegeben und durch Zugabe von 2 µl Isoamylase (Megazyme) bei 37°C für etwa 16 h inkubiert. Eine wässrige 1:4 Verdünnung dieses Verdaus wurde anschließend mit einem 0,2 µm Filter filtriert und 25 µl des Filtrats mittels Gelpermeationschromatographie analysiert.

Die Separation erfolgte mit zwei Säulen, welche in Serie verbunden sind. Zuerst eine Gram 3000-Säule (Polymer Standards Service, mit entsprechender Vorsäule) gefolgt von einer Gram 100-Säule. Die Detektion erfolge mittels Refraktionsindex Detektor (RI 71, Shodex). Die Säule wurde mit 90% (v/v) DMSO, 90 mM Natriumacetat äquilibriert. Eluiert wurde mit 90% (v/v) DMSO, 90 mM Natriumacetat bei einer Flussrate von 0,7 ml/min über einen Zeitraum von 1 h.

Um das Elutionsvolumen mit der Molmasse zu korrelieren, wurden die verwendeten Säulen mit Dextranstandards kalibriert. Die verwendeten Dextrane, ihre zugehörige Molmasse und die Elutionsvolumina sind in Tabelle 1 dargestellt. Mit der daraus resultierenden Kalibrierungsgeraden wurde das Elutionsdiagramm als Molekulargewichtsverteilutig dargestellt (Figur 1):

Tab. 1: Kalibriertabelle mit Dextranstandards

| Elutionsvolumen (ml) | Molmasse [D] | Probenname |
|---|---|---|
| 19,22 | 401300 | Dextran T670P |
| 20,05 | 276500 | Dextran T410P |
| 21,03 | 196300 | Dextran T270P |
| 21,93 | 123600 | Dextran T150P |
| 22,98 | 66700 | Dextran T80 |
| 24,00 | 43500 | Dextran T50 |
| 25,43 | 21400 | Dextran T25 |
| 27,22 | 9890 | Dextran T12 |
| 28,55 | 4440 | Dextran T5 |
| 30,92 | 1080 | Dextran T1 |

Hierbei wurde die Gesamtfläche des GPC-Chromatogramms in einzelne Abschnitte unterteilt, die jeweilige Gruppen von Seitenketten unterschiedlicher Länge repräsentieren. Die gewählten Abschnitte enthalten Seitenketten mit folgendem Polymerisationsgrad (dp = Anzahl der Glukosemonomere innerhalb einer Seitenkette): dp<12, dp12-19, dp20-25, dp26-31, dp32-37. dp38-43, dp44-49, dp50-56, dp57-62, dp63-123 und >dp123. Um das Elutionsvolumen mit der Molmasse zu korrelieren, wird die verwendete GPC-Säule mit Dextranstandards (Fluka, Produkt #31430) kalibriert. Die verwendeten Dextrane, ihre zugehörige Molmasse und die Elutionsvolumina sind in Tab. 1 dargestellt. Mit der daraus resultierenden Kalibrierungsgeraden wird das Elutionsdiagramm als Molekulargewichtsverteilung dargestellt (Fig. 1). Zur Festlegung des Molekulargewichtes der einzelnen Seitenketten wurde für Glukose ein Molekulargewicht von 162 festgelegt. Die gesamte Fläche im GPC-Chromatogramm wird als 100% festgesetzt und der Anteil der Fläche der einzelnen Abschnitte bezogen und auf den Anteil der Gesamtfläche berechnet.:

g) Bestimmung der Gefrier/Tau-Stabilität

Zur Bestimmung der Gefrier/Tau-Stabilität wurden jeweils 3,5 g Stärke (Trockengewicht) mit destilliertem Wasser auf 70 ml aufgefüllt und in einem Rotationsviskosimeter (Rotovisko, Haake) bei 90°C für 15 min verkleistert (128 UpM, Schrägflügelrührer). Anschließend wurde der Stärkekleister in einem Glas mit Schraubverschluss für 15 min bei 121°C autoklaviert. Danach wurden jeweils 5 g dieses Kleisters einem 3-maligen Gefrier/Tau-Zyldus (von Raumtemperatur auf -20°C), ebenfalls in einem Glas mit Schraubverschluss, unterzogen. Diese wurden dann mit 25 ml destilliertem Wasser versetzt, in einem Ultra-Turrax mit 8000 UpM für 1 min homogenisiert und anschließend bei 37°C im Trockenschrank auf einem Magnetrührer für 1 h extrahiert. Anschließend wurde die Probe in einen 50 ml-Messkoben überführt, mit destilliertem Wasser auf 50 ml aufgefüllt, bei 2800 g für 5 min zentrifugiert und filtriert. Von diesem Filtrat wurde ein aliquoter Teil über Nacht bei 105°C eingedampft und der Rückstand gewogen. Die Gefrier/Tau-Stabilität ergab sich dann wie folgt:

$$\text{Gefrier/Tau-Stabilität (\%)} = \frac{50 \times 100 \times \text{TS im Wägeschälchen (g)}}{\text{aliquoter Teil (g)} \times \text{Stärke-TS der Probe (g)}}$$

h) Bestimmung der Hitzestabilität

Zur Bestimmung der Hitzestabilität wurden jeweils 3,5 g Stärke (Trockengewicht) mit destilliertem Wasser auf 70 ml aufgefüllt und in einem Rotationsviskosimeter (Rotovisko, Haake) bei 90°C für 15 min verkleistert (128 UpM, Schrägflügelrührer). Dann wurde der Stärkekleister in einem Glas mit Schaubverschluss für 15 min bei 121°C autoklaviert und anschließend wieder in den Becher des Rotationsviskosimeters überführt.

Nach 6 min bei 90°C und 128 UpM des Flügelrührers wurden die Skalenteile abgelesen und mit dem Messwert nach 21 min bei 128 UpM und 90°C, ohne autoklavieren, ins Verhältnis gesetzt.

$$\text{Hitzestabilität (\%)} = \frac{\text{Skalenteile (nach dem Autoklavieren)} \times 100}{\text{Skalenteile (21 min 128 UpM, 90°C)}}$$

i) DSC-Messung (Differential scanning calorimetry)

Für eine DSC-Messung wurden 10 mg Stärke in einem Edelstahlpfännchen (Volumen 50 µl) mit 30 µl detailliertem Wasser eingewogen. Als Referenz wurde ein leeres Edelstahlpfännchen verwendet. In einem Diamond DSC-Gerät (Perkin Elmer) wurde die Probe mit einer Heizgeschwindigkeit von 10 °C/min von 20 °C bis 120 °C erhitzt. Die Datenanalyse wurde mittels einem Softwareprogramm von Pyres durchgeführt. Dabei wurden T(onset), T(peak) und die freie Enthalpie bestimmt.

j) Die Bestimmung des Gesamtphosphatgehaltes erfolgte nach der Methode von Ames (Methods in Enzymology VIII, (1966), 115-118)

Es werden ca. 50 mg Stärke mit 30 µl ethanolischer Magnesiumnitrat-Lösung versetzt und drei Stunden bei 660°C im Muffelofen verascht. Der Rückstand wird mit 500 µl 0,5 M Salzsäure versetzt und 30 min bei 60°C inkubiert. Anschließend wird ein Aliquot von 10 oder 20 µl (abhängig vom erwarteten Phosphatgehalt) auf 300 µl 0,5 M Salzsäure aufgefüllt, zu einer Mischung aus 100 µl 10%iger Ascorbinsäure und 600 µl 0,42% Ammoniummolybdat in 0,5 M Schwefelsäure gegeben und 20 min bei 45°C inkubiert.
Es folgt eine photometrische Bestimmung bei 820nm unter Berücksichtigung einer Phosphat-Eichreihe als Standard.

**Beschreibung der Sequenzen**

Seq ID 1: Nukleinsäuresequnz der Stärkesynthase SSIII aus Kartoffel *(Solanum tuberosum)* mit Angabe der Sequenzen, die für das entsprechende SSIII Protein kodieren.

Seq ID 2: Aminosäuresequenz eines SSIII Proteins aus Kartoffel.

Seq ID 3: Aminosäuresequenz der Pfam cbm25 Bindedömäne des SSIII-Proteins aus Kartoffel *(Solanum tuberosum).*

Seq ID 4: Kodierende Nukleinsäuresequenz des Verzweigungsenzyms BEI aus Kartoffel *(Solanum tuberosum).*

Seq ID 5: Aminosäuresequenz des Verzweigungsenzyms BEI aus Kartoffel *(Solanum tuberosum)*

Seq ID 6: Kodierende Nukleinsäuresequenz des GBSSI-Gens aus Kartoffel *(Solanum tuberosum).*

Seq ID 7: Aminosäuresequenz der GBSSI aus Kartoffel *(Solanum tuberosum)*

Seq ID 8: Primer B1_Asp

SEQ ID NO 9: Nukleinsäuresequenz enthaltend die kodierende Region des 3'-Bereichs eines an der Stärkebiosynthese beteiligten Proteins aus *Solanum tuberosum* (cv Désirée). Diese Sequenz ist in Plasmid AN 46-196 inseriert.

SEQ ID NO 10: Nukleinsäuresequenz enthaltend die kodierende Region des 5'-Bereichs eines an der Stärkebiosynthese beteiligten Proteins aus *Solanum tuberosum* (cv Desiree). Diese Sequenz ist in Plasmid AN 47-196 inseriert.

SEQ ID NO 11: Nukleinsäuresequenz enthaltend die vollständige kodierende Region eines an der Stärkebiosynthese beteiligten Proteins aus *Solanum tuberosum* (cv Desiree). Diese Sequenz ist in Plasmid AN 49 inseriert. Dieses Plasmid wurde nach dem Budapester Vertrag hinterlegt am 15. September 2003 unter der Nummer DSM 15926 bei der Deutschen Sammlung von Mikroorganismen und Zellkulturen GmbH, Mascheroder Weg 1b, 38124 Braunschweig, Deutschland.

SEQ ID NO 12: Aminosäuresequenz kodierend eine an der Stärkebiosynthese beteiligtes Protein aus *Solanum tuberosum* (cv Desirée). Diese Sequenz ist von der in Plasmid AN 49 inserierten Nukleinsäuresequenz bzw. von der unter SEQ ID NO 11 beschriebenen Nukleinsäuresequenz ableitbar.

SEQ ID NO 13: Nukleinsäuresequenz enthaltend die vollständige kodierende Region eines an der Stärkebiosynthese beteiligten Proteins aus *Solanum tuberosum* (cv Désirée). Diese Sequenz wurde erhalten durch Zusammenfügen der unter SEQ ID NO 9 und SEQ ID NO 10 beschriebenen Nukleinsäuresequenzen. Diese Nukleinsäuresequenz stellt eine allelische Variante der unter SEQ ID NO 11 beschriebenen Nukleinsäuresequenz, kodierend ein an der Stärkebiosynthese beteiligtes Protein dar.

SEQ ID NO 14: Aminosäuresequenz kodierend ein an der Stärkebiosynthese beteiligtes Protein aus *Solanum tuberosum* (cv Désirée). Diese Sequenz ist, von der unter SEQ ID NO 13 beschriebenen Nukleinsäuresequenz ableitbar und stellt die Aminosäuresequenz einer allelischen Variante zu der unter SEQ ID NO 12 beschriebenen Aminosäuresequenz, kodierend ein an der Stärkebiosynthese beteiligtes Protein dar.

SEQ ID No. 15: Primer B2_Sal (TCA AGT CGA CCA CAA CCA GTC CAT TTC TGG)

SEQ ID No. 16 Primem KM2_Spe (5'-TCAAACTAGTCACAACCAGTCCATTTCTGG-3')

SEQ ID No. 17 Primer SoputE (5'-CACTTTAGAAGGTATCAGAGC-3')

SEQ ID No. 18 Primer So_put5' (5'-GTATTTCTGCGAAGGAACGACC-3')

SEQ ID No. 19 Primer So_putA (5'- AACAATGCTCTCTCTGTCGG-3')

SEQ ID No. 20 Primer B3_Sa1 (GCT TGT CGA CGG GAG AAT TTT GTC CAG AGG)

SEQ ID No. 21 Primer B4_Sal (GAT CGT CGA CAG CAC TTC TAC TTG GCA GAG G)

## Beschreibung der Abbildungen

Fig 1: Kalibrierkurve für GPC

### Beispiel 1

### Herstellung transgener Kartoffelpflanzen, die eine verringerte BEI-, SSIII- und GBSSI-Genexpression aufweisen

Zur Erzeugung transgener Pflanzen, die eine verringerte BEI-, SSIII- und GBSSI-Aktivität aufweisen, wurden zunächst transgene Pflanzen erzeugt, die eine verringerte BEI- und SSIII-Aktivität aufweisen. Zu diesem Zwecke wurde die T-DNA des Plasmids pB33-aBEI-aSSIII-Kan mit Hilfe von Agrobakterien, wie bei Rocha-Sosa et al. (1989, EMBO J. 8, 23-29) beschrieben, in Kartoffelpflanzen transferiert.

Zur Konstruktion des Plasmids pB33-aBEI-aSSIII-Kan wurde zunächst der Expressionsvektor pBin33-Kan konstruiert. Dazu wurde der Promotor des Patatin-Gens B33 aus Solanum tuberosum (Rocha-Sosa et al., 1989, supra) als DraI-Fragment (Nukleotide -1512 bis +14) in den mit SstI geschnittenen Vektor pUC19 (Genbank Acc. No. M77789), dessen Enden mit Hilfe der T4-DNA-Polymerase geglättet worden waren, ligiert. Daraus entstand das Plasmid pUC19-B33.

Aus diesem Plasmid wurde der B33-Promotor mit EcoRI und SmaI herausgeschnitten und in den entsprechend geschnittenen Vektor pBinAR ligiert. Hieraus entstand der pflanzliche Expressionsvektor pBin33-Kan. Das Plasmid pBinAR ist ein Derivat des Vektorplasmids pBin19 (Bevan, 1984, Nucl. Acid Research 12, 8711-8721) und wurde von Höfgen and Willmitzer (1990, Plant Sci. 66, 221-230) konstruiert. Anschließend wurde ein 1631 Bp langes HindII-Fragment, welches eine partielle cDNA kodierend für BEI aus Kartoffel enthält (Kossmann et al., 1991, Mol. Gen. Genet. 230(1-2), 39-44), geglättet und in "antisense"-Orientierung bezüglich des B33-Promotors (Promotor des Patatin-Gens B33 aus Solanum tuberosum; Rocha-Sosa et al., 1989, supra) in den mit SmaI vorgeschnittenen Vektor pBinB33 eingeführt. Das erhaltene Plasmid wurde mit BamHI aufgeschnitten. In die Schnittstelle wurde ein 1363 Bp langes BamHI-Fragment, enthaltend eine partielle cDNA kodierend für SSIII aus Kartoffel (Abel et al., 1996, supra), ebenfalls in "antisense"-Orientierung bezüglich des B33-Promotors eingeführt.

Zum Nachweis der Aktivität löslicher Stärkesynthasen durch nicht denaturierende Gelelektrophorese wurden Gewebeproben von Kartoffelknollen in 50 mM Tris-HCl pH 7,6, 2 mM DTT, 2,5 mM EDTA, 10% Glycerin und 0,4 mM PMSF aufgeschlossen. Die Elektrophorese wurde in einer MiniProtean II Kammer (BioRAD) durchgeführt. Die Monomerkonzentration der 1,5 mm dicken Gele war 7,5% (w/v), als Gel- und Laufpuffer diente 25 mM Tris-Glycin pH 8,4. Gleiche Mengen an Proteinextrakt wurden aufgetragen und für 2 h bei 10 mA je Gel aufgetrennt.

Anschließend erfolgte die Inkubation der Aktivitätsgele in 50 mM Tricine-NaOH pH 8,5, 25 mM Kaliumacetat, 2 mM EDTA, 2 mM DTT, 1 mM ADP-Glukose, 0,1% (w/v) Amylopektin und 0,5 M Natriumcitrat. Gebildete Glukane wurden mit Lugolscher Lösung angefärbt.

Der Nachweis der BEI-Aktivität erfolgte ebenfalls mit Hilfe der nicht denaturierenden Gelelektrophorese: Zur Isolierung von Proteinen aus Pflanzen wurde das Probenmaterial in flüssigem Stickstoff gemörsert, in Extraktionspuffer (50 mM Na-Citrat, pH 6,5; 1 mM EDTA, 4 mM DTT) aufgenommen und nach Zentrifugation (10 min, 14000 g, 4°C) direkt zur Messung der Proteinkonzentration nach Bradford eingesetzt. Anschließend wurde je nach Bedarf 5 bis 20 µg Gesamt-Proteinextrakt mit 4-fach Loading-Buffer (20% Glycerin, 125 mM Tris HCl, pH 6,8) versetzt und auf ein "BE-Aktivitätsgel" geladen. Der Laufpuffer (RB) setzte sich wie folgt zusammen: RB = 30,2 g Tris-Base, pH 8,0, 144 g Glycine auf 1l $H_2O$.

Nach Beendigung des Gellaufes wurden die Gele in je 25 ml "Phosphorylase - Puffer" (25 ml 1M Na-Citrat pH 7,0, 0,47 g Glukose-1-Phosphat, 12,5 mg AMP, 2,5 mg Phosphorylase a/b aus Kaninchen) über Nacht bei 37°C inkubiert. Die Färbung der Gele wurde mit Lugolscher Lösung durchgeführt.

Es konnten verschiedene Linien transgener Kartoffelpflanzen identifiziert werden, deren Knollen eine deutlich verringerte BEI- und SSIII-Aktivität aufwiesen. Die Linie (asBEI-asSSIII), deren isolierte Stärken den höchsten Phosphatgehalt aller untersuchten unabhängigen Transformanden aufwies, wurden anschließend wie beschrieben bei Rocha-Sosa et al. (1989, EMBO J. 8, 23-29) mit dem Plasmid p35SaGBSSI-Met transformiert.

Das Plasmid p35SaGBSSI-Met wurde hergestellt, indem ein 1921 Bp langes Asp718/XbaI-Fragment, enthaltend eine partielle cDNA, die für GBSSI aus Kartoffel codiert (Hergersberg, 1988, supra), in "antisense"-Orientierung bezüglich des 35S-Promotors in den ebenso geöffneten Vektor pBinAR-Met eingefügt wurde.

Der Vektor pBinAR-Met geht von dem Plasmid pGPTV-DHFR aus, welches ein Derivat des Vektors pBin19 darstellt (Becker et al., 1992, Plant Mol. Biol. 20, 1195-1197). pBinAR-Met enthält statt des nptII-Gens das dhfr-Gen, welches Resistenz gegen Methotrexat vermittelt, sowie statt des 3'-Endes des Nopalinsynthase-Gens das 3'-Ende des Gen 7 der T-DNA des Ti-Plasmids pTiACH5 (Nukleotide 2106-2316; Gielen et al., 1984, EMBO J. 3, 835-846). Ausgehend vom Plasmid pA7 (vergleiche Beschreibung des Vektors pBinAR oben) wurde das EcoRI-HindIII-Fragment umfassend den 35S-Promotor, den ocs-Terminator sowie den dazwischen liegenden Teil des Polylinkers in das entsprechend geschnittene Plasmid pGPTV-DHFR ligiert. Der entstandene Vektor wurde mit pBinAR-Met bezeichnet.

Von den durch Transformation mit dem Plasmid p35SaGBSSI-Met erhaltenen Pflanzen, die als asBEI-asSSIII-asGBSSI-Pflanzen bezeichnet wurden, wurden Gewebeproben von Knollen der unabhängigen Transformanden genommen, mit Jod-Lösung angefärbt und im Mikroskop untersucht. Die Stärken der unabhängigen Linien, deren Körner braun färbten, wurden für eine weitere Analyse der Stärkeeigenschaften herangezogen.

## Beispiel 2

### Klouierung einer Vollängensequenz eines Gens mit der unter SEQ ID NO. 11 oder 13 angegebenen Sequenz aus *Solanum tuberosum*

Die Nukleotidsequenz (SEQ ID No. 11 oder SEQ ID No. 13) kodierend für ein Protein mit der unter SEQ ID No. 12 oder SEQ ID No. 14 angegebenen Aminosäuresequenz aus *Solanum tuberosum* ist bisher noch nicht beschrieben worden.

Über Sequenzvergleiche mit verschiedenen Verzweigungsenzymen konnte eine Domäne identifiziert werden, mit deren Hilfe EST-Datenbanken durchmustert wurden. Hierbei konnte der EST TC73137 (TIGR Datenbank; http://www.tigr.org/tigr-scripts/tgi/tc_report.pl?tc=TC73137&species=potato) aus Kartoffel identifiziert werden.
Mit Hilfe der Primer B1_Asp (GAT GGG TAC CAG CAC TTC TAC TTG GCA GAG G = SEQ ID No. 8) und B2_Sal (TCA AGT CGA CCA CAA CCA GTC CAT TTC TGG = SEQ ID No. 15) konnte eine zu dieser EST-Sequenz korrespondierende Sequenz aus einer knollespezifischen cDNA-Bank von *Solanum tuberosum* (cv. Désirée) amplifiziert werden. Versuche, blattspezifische, "sink"- oder "source"-Gewebe spezifische cDNA-Banken als Template für die PCR Reaktion zu verwenden, führten zu keinem Amplifikat.
Um die gesamte kodierende Sequenz des betreffenden Proteins, die auch bisher unbekannte Sequenzen umfasst, zu amplifizieren, wurden Primer hergestellt, die zu den Enden der bisher bekannten Sequenz und Vektorsequenzen der betreffenden cDNA Banken komplememtär sind. Bei allen mittels dieses Ansatzes verwendeten Primerkombinationen zur Amplifikation einer vollänge Sequenz konnte kein weiterer Bereich amplifiziert werden. Daraufhin wurden EST-Datenbanken von Tomate erneut durchmustert.
Hierbei konnten zwei ESTs aus Tomate identifiziert werden (TIGR Datenbank; BG127920 und TC130382), .die entweder eine hohe Homologie zu dem oben beschriebenen Amplifikat des Proteins aus Kartoffel (TC130382) bzw. (BG127920). oder zu einem putativen Verzweigungsenzym aus *Arabidopsis* (Genbank: GP|9294564|dbj|BAB02827.1) aufweisen.
Es wurden nun erneut Primer hergestellt, um auch bisher unbekannte Sequenzen des Proteins mit der unter SEQ ID NO 12 oder SEQ ID NO 14 dargestellten Aminosäuresequenz zu amplifizieren. Mittels PCR wurde aus einer cDNA Bank, hergestellt aus Knollen von *Solanum tuberosum* (cv. Désirée), mit den Primem KM2_Spe (5'-TCAAACTAGTCACAACCAGTCCATTTCTGG-3' = SEQ ID No. 16) und SoputE (5'-CACTTTAGAAGGTATCAGAGC-3' = SEQ ID No.17) der 3'-Bereich des betreffenden Proteins amplifiziert. Das erhaltende ca. 1 kb große Fragment wurde ungerichtet in den pCR4-TOPO Vektor von Invitrogen (Produktnummer: 45-0030) kloniert. Das entstandene Plasmid wurde als AN 46-196 bezeichnet. Die Sequenz des inserierten Fragments im Plasmid AN 46-196 ist unter SEQ ID NO 9 dargestellt.

Der 5'-Bereich wurde ebenfalls mittels PCR-Technik und unter Verwendung der Primer So_put5' (5'-GTATTTCTGCGAAGGAACGACC-3' = SEQ ID No.18) und So_putA (5'-AACAATGCTCTCTCTGTCGG-3' = SEQ ID No.19) aus der selben cDNA-Bank amplifiziert. Das erhaltende ca. 2 kb große Fragment wurde ungerichtet in den pCR4-TOPO Vektor von Invitrogen (Produktnummer: 45-0030) kloniert. Das entstandene Plasmid wurde als AN 47-196 bezeichnet. Die Sequenz des inserierten Fragments im Plasmid AN 47-196 ist unter SEQ ID NO 10 dargestellt.

Es wurden nun erneut Primer hergestellt, um eine Vollängensequenz zu amplifizieren.
Folgende Primer wurden verwendet: SOputA (AACAATGCTCTCTCTGTCGG = SEQ ID No.19) und SO_putE (CACTTTAGAAGGTATCAGAGC = SEQ ID No.17). Ein ungefähr 3,2 kb großes PCR-Produkt wurde erhalten und in den pCR2.1-Vektor der Firma Invitrogen (Produktnummer: 45-0030) kloniert. Das erhaltene Plasmid (hinterlegt unter DSM 15926) wurde mit AN 49 bezeichnet. Die Sequenz des inserierten Fragments im Plasmid AN 49 ist unter SEQ ID NO 11 dargestellt.

Die Sequenzinformation der unter SEQ ID NO 13 dargestellten Nukleinsäuresequenz wurde erhalten durch Zusammenfügen der unter SEQ ID NO 9 und SEQ ID NO 10 beschriebenen Nukleinsäuresequenzen. Diese Nukleinsäuresequenz stellt eine allelische Variante der unter SEQ ID NO 11 beschriebenen Nukleinsäuresequenz, kodierend ein an der Stärkebiosynthese beteiligtes Protein (SEQ ID No. 14) dar.

**Beispiel 3**

**Herstellung transgener Kartoffelpflanzen, die eine verringerte BEI-, SSIII- und GBSSI-Genexpression und eine verringerte Genexpression des unter SEQ ID No. 11 oder SEQ ID No. 13 angegebenen Gens aufweisen**

In die in Beispiel 1 beschriebenen Kartoffelpflanzen mit verminderter BEI-, SSIII- und GBSSI-Genexpression, die dort als asBEI-asSSIII-asGBSSI-Pflanzen bezeichnet wurden, wurde die T-DNA des Plasmids AN 54-196 (siehe unten) mit Hilfe von Agrobakterien, wie bei Rocha-Sosa et al. (EMBO J. 8, (1989), 23-29) beschrieben. Die durch Transformation mit dem Plasmid AN 54-196 erhaltenen Pflanzen wurden als asBEI-asSSIII-asGBSSI-iBE3 bezeichnet und weisen neben einer verminderten BEI-, SSIII- und GBSSI-Genexpression, eine Verringerung der Genexpression des unter SEQ ID No. 11 oder SEQ ID No. 13 bezeichneten Gens auf. Es wurden Gewebeproben von Knollen der unabhängigen Transformanden genommen, mit Jod angefärbt und mikroskopiert. Es wurde ferner der Phosphatgehalt in C6-Position bestimmt. Die Stärken der unabhängigen Linien, deren Körner braun färbten und die einen Phosphatgehalt aufwiesen, der über dem der Ausgangslinien (siehe Beispiel 1) lag, wurden für eine weitere Analyse der Stärkeeigenschaften herangezogen.

Angaben zum Vektor AN 54-196

AN 54-196 ist ein Derivat des Plasmides pBinB33-Hyg, welchem eine Teilsequenz der unter SEQ ID NO 11 oder SEQ ID NO 13 angegebenen kodierenden Nukleinsäuresequenz als "inverted repeat" ,(RNAi Technologie) unter der Kontrolle des Promotors des Patatin Gens B33 aus *Solanum tuberosum* (Rocha-Sosa et al., 1989) eingefügt wurde.

Hierzu wurde zuerst ein PCR-Produkt mit den Primem B1_Asp (GAT GGG TAC CAG CAC TTC TAC TTG GCA GAG G) und B2_Sal (TCA AGT CGA CCA CAA CCA GTC CAT TTC TGG) aus einer knollenspezifischen cDNA Bank von *Solanum tuberosum* (cv. Désirée) amplifiziert, wodurch die Schnittstellen *Asp718* und *SalI* zugefügt wurden. Das erhaltene PCR-Produkt (625 bp) wurde über diese beiden Schnittstellen in "antisense" Orientierung zum B33-Promotor kloniert. Ein zweites PCR-Fragment, welches mit den Primern B3_Sal (GCT TGT CGA CGG GAG AAT TTT GTC CAG AGG = SEQ ID No.20) und B4_Sal (GAT CGT CGA CAG CAC TTC TAC TTG GCA GAG G= SEQ ID No.21) aus einer knollenspezifischen cDNA Bank von *Solanum tuberosum* (cv. Désirée) amplifiziert wurde und mit dem 301 bp des ersten Fragments identisch ist, wurde über die *SalI*-Schnittstelle hinter das erste Fragment kloniert, jedoch in "sense"-Orientierung zum B33-Promotor. Diese Anordnung wird als "inverted repeat" (RNAi Technologie) bezeichnet.

Angaben zum Vektor pBinB33-Hyg

Ausgehend vom Plasmid pBinB33 wurde das *Eco*RI-*Hind*III-Fragment umfassend den B33-Promotor, einen Teil des Polylinkers sowie den *ocs*-Terminator herausgeschnitten und in den entsprechend geschnittenen Vektor pBIB-Hyg ligiert (Becker, 1990).
Das Plasmid pBinB33 wurde erhalten, indem der Promotor des Patatin Gens B33 aus *Solanum tuberosum* (Rocha-Sosa et al., 1989) als *Dra*I-Fragment (Nukleotide -1512 - +14) in den mit *Sst*I geschnittenen Vektor pUC19, dessen Enden mit Hilfe der T4 DNA-Polymerase geglättet worden waren, ligiert wurde. Daraus entstand das Plasmid pUC19-B33. Aus diesem Plasmid wurde der B33-Promotor mit *EcoR*I und *Sma*I herausgeschnitten und in den entsprechend geschnittenen Vektor pBinAR ligiert. Hieraus entstand der pflanzliche Expressionsvektor pBinB33.
Das Plasmid pBinAR ist ein Derivat des Vektorplasmids pBin19 (Bevan, 1984) und wurde folgendermaßen konstruiert: Ein 529 bp langes Fragment, das die Nukleotide 6909-7437 des 35S RNA-Promotors des Blumenkohl-Mosaik-Virus (Pietrzak et al., 1986, Nucleic Acids Research 14, 5857-5868) umfasst, wurde als *Eco*RI/*Kpn*I-Fragment aus dem Plasmid pDH51 (Pietrzak et al., 1986) isoliert und zwischen die *Eco*RI- und *Kpn*I-Schnittstellen des Polylinkers von pUC18 ligiert. Dabei entstand das Plasmid pUC18-35S.
Aus dem Plasmid pAGV40 (Herrera-Estrella et al., 1983) wurde mit Hilfe der Restriktionsendonukleasen *Hind*III und *Pvu*II ein 192 bp langes Fragment isoliert, welches das Polyadenylierungssignal (3'-Ende) des *Octopin Synthase-Gens* (Gen 3) der T-DNA des Ti-Plasmids pTiACH5 (Gielen et al., 1984) umfasst (Nukleotide 11749-11939). Nach Addition von *Ssp*I-Linkern an die *Pvu*II-Schnittstelle wurde das Fragment zwischen die *Sph*I- und *Hind*III-Schnittstelle von pUC18-35S ligiert. Daraus resultierte das Plasmid pA7.
Aus pA7 wurde der gesamte Polylinker enthaltend den 35S-Promotor und den ocs-Terminator mit EcoR1 und HindIII herausgeschnitten und in den entsprechend geschnittenen pBin19 ligiert. Dabei entstand der pflanzliche Expressionsvektor pBinAR (Höfgen and Willmitzer, 1990).

## Beispiel 4

### Analyse der Stärke von transgenen Kartoffelpflanzen, die eine verringerte BEI-, SSIII- und GBSSI-Genexpression und eine verringerte Genexpression des unter SEQ ID No. 11 oder SEQ ID No. 13 angegebenen Gens aufweisen

Die Stärke verschiedener unabhängiger Linien der in Beispiel 3 beschriebene Transformation asBEI-asSSIII-asGBSSI-iBE3 wurden aus Kartoffelknollen isoliert. Anschließend wurden die physikalisch-chemischen Eigenschaften dieser Stärke analysiert. Die auf der Grundlage der von den erfindungsgemäßen Pflanzenzellen bzw. -Pflanzen hergestellten Stärken erzielten Ergebnisse sind im Weiteren entweder als absolute Werte oder prozentual bezogen auf Stärke aus entsprechenden Wildtyp-Pflanzenzellen bzw. -Pflanzen (nachfolgend als "WT-Stärke" bezeichnet) angegeben (Tabelle 2). Zusätzlich enthält die Tabelle Stärkedaten von "Einzel" bzw. "Doppel"-Kombinationen aus WO 00/08184 und WO 01/12782:

Tabelle 2

| | % bezogen auf WT-Stärke | | |
|---|---|---|---|
| | Phosphat in C6 | Amylose | Gelfestigkeit |
| asSSIII | 197 | 123 | 84 |
| cosSSIII | 210 | - | 83 |
| asBEI | 170 | 91 | 91 |
| asGBSSI | 110 | <18 | - |

(fortgesetzt)

| | % bezogen auf WT-Stärke | | |
|---|---|---|---|
| | Phosphat in C6 | Amylose | Gelfestigkeit |
| asBEI-asSSIII | 292 | 89 | 100 |
| asGBSSI-asBEI | 181 | <18 | 21 |
| asBEI-asSSIII-asGBSSI | 360 | <18 | 16 |
| asBEI-asSSIII-asGBSSI-iBE3 | 462 | <18 | - |

Ferner wurden für die Stärken aus den entsprechenden Wildtyppflanzen (Varietät Desiree), der Ausgangslinie (asBEI-asSSIII-asGBSSI) und der asBEI-asSSIII-asGBSSI-iBE3-Kartoffelpflanzen die Absolutwerte für den Amylosegehalt (bestimmt nach der Methoden von Hovenkamp-Hermelink) und für den Phosphatgehalt in C6-Position (Methodenbeschreibung siehe oben "Allgemeine Methoden") ermittelt (Tabelle 3):

Tabelle 3: Amylosegehalt, Phosphatgehalt in C6-Position

| Genotyp | C6-Phosphat [nmol/mg] | Amylose [% der Gesamtstärke] |
|---|---|---|
| Desiree (Wildtyp) | 12,1 | 24,1 |
| asBEI-asSSIII-asGBSSI | 43,5 | < 3 |
| asBEI-asSSIII-asGBSSI-iBE3 | 56,3 | <3 |

Eine Analyse des Seitenkettenprofils der Kartoffelstärke erfolgte über die Bestimmung des prozentualen Anteils einer bestimmten Gruppe von Seitenketten am Gesamtanteil aller Seitenketten im GPC-Chromatogramm (Tabelle 4) (siehe Allgemeine Methoden "Analyse der Seitenkettenverteilung der Gesamtstärke mittels Gelpermeationschromatographie"):

Tabelle 4: Verteilung des Seitenkettenprofils von Gesamtstärke der Linien asBEI-asSSIII, asBEI-asSSIII-asGBSSI, asBEI-asSSIII-asGBSSI-iBE3 und des entsprechenden Wildtyps, eingeteilt in Gruppen mit unterschiedlichem Grad der Polymerisation

| Grad der Polymerisation (dp) | % bezogen auf WT-Stärke | | |
|---|---|---|---|
| | asBEI-asSSIII | asBEI-asSSIII-asGBSSI | asBEI-asSSIII-asGBSSIiBE3 |
| dp<12 | 119,71 | 156,57 | 153,27 |
| dp12-19 | 103,83 | 119,00 | 147,90 |
| dp20-25 | 120,36 | 131,79 | 142,80 |
| dp26-31 | 127,31 | 142,19 | 130,42 |
| dp32-37 | 122,60 | 144,16 | 120,97 |
| dp38-43 | 103,14 | 130,00 | 110,77 |
| dp44-49 | 83,42 | 114,95 | 92,40 |
| dp50-56 | 72,51 | 106,85 | 93,47 |
| dp57-62 | 68,85 | 105,21 | 90,28 |
| dp63-123 | 56,44 | 91,78 | 77,70 |
| dp>123 | 104,21 | 3,97 | 0,96 |

Ferner wurde das Verhältnis des Gesamtphosphatgehalts zum Phosphatgehalt in C6-Position bestimmt:

| Genotyp | Gesamtphosphatgehalt/C6-Phosphatgehalt |
|---|---|
| Desiree (Wildtyp) | 1,88 |
| asBEI-asSSIII-asGBSSI | 1,62 |
| asBEI-asSSIII-asGBSSI-iBE3 | 1,37 |

## Patentansprüche

1. Kartoffelstärke **dadurch gekennzeichnet, dass** sie einen Amylosegehalt gemessen nach der Methode von Hovenkamp-Hermelink et al. (1988, Potato Research 31, 241-246) von weniger als 10 Gew.-%, einen Phosphatgehalt in C6-Position zwischen 35 und 100 nmol Phosphat pro Milligramm Stärke (Trockengewicht) und einen erhöhten Gehalt an Seitenketten mit einem DP von 12 bis 19 im Vergleich zu Kartoffelstärke aus entsprechenden Kartoffel-Wildtyp-Pflanzen aufweist.

2. Kartoffelstärke nach Anspruch 1, **dadurch gekennzeichnet, dass** sie einen Phosphatgehalt in C6-Position zwischen 40 und 85 nmol Phosphat pro Milligramm Stärke (Trockengewicht) aufweist.

3. Kartoffelstärke nach Anspruch 1, **dadurch gekennzeichnet, dass** sie einen Phosphatgehalt in C6-Position zwischen 45 und 70 nmol Phosphat pro Milligramm Stärke (Trockengewicht) aufweist.

4. Kartoffelstärke nach Anspruch 1, **dadurch gekennzeichnet, dass** sie einen Phosphatgehalt in C6-Position zwischen 50 und 65 nmol Phosphat pro Milligramm Stärke (Trockengewicht) aufweist.

5. Kartoffelstärke nach einem der Ansprüche 1 bis 4, **dadurch gekennzeichnet, dass** sie einen Amylosegehalt gemessen nach der Methode von Hovenkamp-Hermelink et al. (1988, Potato Research 31, 241-246) von weniger als 5 Gew.-% aufweist.

6. Kartoffelstärke nach einem der Ansprüche 1 bis 4, **dadurch gekennzeichnet, dass** sie einen Amylosegehalt gemessen nach der Methode von Hovenkamp-Hermelink et al. (1988, Potato Research 31, 241-246) von weniger als 3 Gew.-% aufweist.

7. Kartoffelstärke nach einem der Ansprüche 1 bis 6, wobei der Anteil der Seitenketten mit einem DP von 12 bis 19 auf 125%-200% erhöht ist im Vergleich zu Kartoffelstärke aus entsprechenden Kartoffel-Wildtyp-Pflanzen.

8. Kartoffelstärke nach einem der Ansprüche 1 bis 6, wobei der Anteil der Seitenketten mit einem DP von 12 bis 19 auf 130%-180% erhöht ist im Vergleich zu Kartoffelstärke aus entsprechenden Kartoffel-Wildtyp-Pflanzen.

9. Kartoffelstärke nach einem der Ansprüche 1 bis 8, wobei der Anteil der Seitenketten mit einem DP von 63 bis 123 verringert ist im Vergleich zu Kartoffelstärke aus entsprechenden Kartoffel-Wildtyp-Pflanzen..

10. Kartoffelstärke nach Anspruch 9, wobei der Anteil der Seitenketten mit einem DP von 63 bis 123 auf 50%-95% verringert ist im Vergleich zu Kartoffelstärke aus entsprechenden Kartoffel-Wildtyp-Pflanzen.

**11.** Derivatisierte Kartoffelstärke enthaltend Kartoffelstärke nach einem der Ansprüche 1 bis 10.

# Figur 1

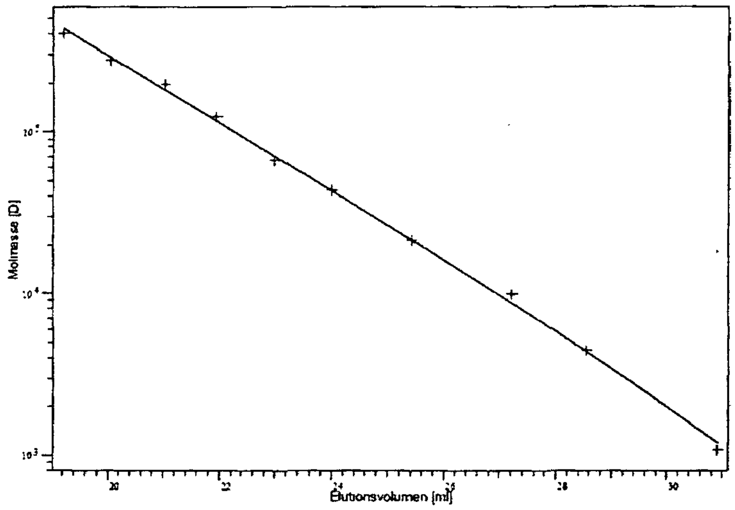


*Fit-Qualität:*
Fit-Typ: PSS Poly 3
R : 0.999274

*Mark-Houwink Koeffizienten:*
a = 0.000 K = 1.0000

| Elutionsvolumen [ml] | Molmasse [D] | Probenname | Steigung | Abweichung [%] |
|---|---|---|---|---|
| 19.22 | 401300 | Dextran T670P | -0.21 | 8.02 |
| 20.05 | 276500 | Dextran T410P | -0.21 | 4.83 |
| 21.03 | 196300 | Dextran T270P | -0.21 | -8.08 |
| 21.93 | 123600 | Dextran T150P | -0.21 | -5.29 |
| 22.98 | 66700 | Dextran T80 | -0.21 | 5.87 |
| 24.00 | 43500 | Dextran T50 | -0.21 | -0.75 |
| 25.43 | 21400 | Dextran T25 | -0.21 | 0.11 |
| 27.22 | 9890 | Dextran T12 | -0.22 | -11.73 |
| 28.55 | 4440 | Dextran T5 | -0.23 | -1.62 |
| 30.92 | 1080 | Dextran T1 | -0.25 | 11.05 |

Europäisches Patentamt

# EUROPÄISCHER RECHERCHENBERICHT

Nummer der Anmeldung
EP 05 09 0085

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| X,D | WO 01/12782 A (AVENTIS CROPSCIENCE GMBH) 22. Februar 2001 (2001-02-22)<br>* Seite 2, Absatz 4 - Seite 5, Absatz 1; Tabelle 1 *<br>* Seite 13, Absatz 4 - Seite 16, Absatz 3; Ansprüche 26-28; Beispiel 1 *<br>----- | 1-11 | C12N15/82<br>C12N9/10<br>C08B30/00 |
| X,D | WO 00/08184 A (HOECHST SCHERING AGREVO GMBH) 17. Februar 2000 (2000-02-17)<br>* Abbildung 2; Beispiel 8; Tabellen 1,2 *<br>----- | 1-11 | |
| X,D | WO 01/19975 A (NATIONAL STARCH AND CHEMICAL INVESTMENT HOLDING CORPORATION; JOBLING,) 22. März 2001 (2001-03-22)<br>* Seite 5 - Seite 7; Abbildung 8; Tabellen 1,2 *<br>* Seite 22 - Seite 23 *<br>* Seite 28 - Seite 29 *<br>----- | 1-11 | |
| | | | RECHERCHIERTE SACHGEBIETE (Int.Cl.7) |
| | | | C08B<br>C12N |

Der vorliegende Recherchenbericht wurde für alle Patentansprüche erstellt

3

| Recherchenort | Abschlußdatum der Recherche | Prüfer |
|---|---|---|
| Den Haag | 30. August 2005 | Oderwald, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 05 09 0085

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-08-2005

| Im Recherchenbericht angeführtes Patentdokument | Datum der Veröffentlichung | Mitglied(er) der Patentfamilie | Datum der Veröffentlichung |
|---|---|---|---|
| WO 0112782 A | 22-02-2001 | DE 19937643 A1 | 22-02-2001 |
| | | AU 782182 B2 | 07-07-2005 |
| | | AU 6699200 A | 13-03-2001 |
| | | CA 2381163 A1 | 22-02-2001 |
| | | WO 0112782 A2 | 22-02-2001 |
| | | EP 1203087 A2 | 08-05-2002 |
| | | JP 2003507034 T | 25-02-2003 |
| WO 0008184 A | 17-02-2000 | DE 19836098 A1 | 03-02-2000 |
| | | AU 772364 B2 | 22-04-2004 |
| | | AU 5161999 A | 28-02-2000 |
| | | BR 9912665 A | 02-05-2001 |
| | | CA 2338002 A1 | 17-02-2000 |
| | | CN 1316006 A ,C | 03-10-2001 |
| | | WO 0008184 A1 | 17-02-2000 |
| | | EP 1100937 A1 | 23-05-2001 |
| | | HU 0102998 A2 | 28-11-2001 |
| | | JP 2002525036 T | 13-08-2002 |
| | | PL 345829 A1 | 14-01-2002 |
| | | US 6596928 B1 | 22-07-2003 |
| | | US 2003167529 A1 | 04-09-2003 |
| WO 0119975 A | 22-03-2001 | AU 7031000 A | 17-04-2001 |
| | | CA 2388364 A1 | 22-03-2001 |
| | | EP 1212440 A2 | 12-06-2002 |
| | | WO 0119975 A2 | 22-03-2001 |
| | | JP 2003509047 T | 11-03-2003 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9214827 A
- WO 0119975 A
- WO 0112782 A
- WO 0008184 A
- WO 0066745 A
- WO 9704112 A
- WO 9704113 A
- WO 9837213 A
- WO 9837214 A
- EP 0321201 B1
- WO 9515972 A
- WO 9953050 A
- HU 9801674
- WO 9211376 A
- US 5656496 A
- US 5639952 A
- US 5034322 A
- US 4962028 A
- WO 9307279 A
- WO 9101373 A
- EP 0120516 A
- WO 9506128 A
- EP 0513849 A
- EP 0465875 A
- EP 0292435 A


### In der Beschreibung aufgeführte Nicht-Patentliteratur


- **HIZUKURI ; TAKAGI.** *Carbohydr. Res.,* 1984, vol. 134, 1-10
- **TAKEDA et al.** *Carbohydr. Res,* 1984, vol. 132, 83-92
- **VOET ; VOET.** Biochemistry. John Wiley & Sons, 1990
- **JANE et al.** *Cereal Foods World,* 1996, vol. 41 (11), 827-832
- **TAKEDA ; HIZUKURI.** *Starch/Stärke,* 1971, vol. 23, 267-272
- **BLENNOW et al.** *Int. J. of Biological Macromolecules,* 2000, vol. 27, 211-218
- **SHURE et al.** *Cell,* 1983, vol. 35, 225-233
- **HOVENKAMP-HERMELINK et al.** *Theoretical and Applied Genetics,* 1987, vol. 75, 217-221
- **VISSER et al.** *Mol. Gen. Genet.,* 1991, vol. 225, 289-296
- **ABEL et al.** *The Plant Journal,* 1996, vol. 10 (6), 981-991
- **LLOYD et al.** *Biochemical Journal,* 1999, vol. 338, 515-521
- **ABEL et al.** *The Plant Journal,* 1996, vol. 10 (6), 9891-9991
- **ABEL.** Dissertation. Freie Universität, 1995
- **KOSSMANN et al.** *Mol. Gen. Genet.,* 1991, vol. 230, 39-44
- **SAFFORD et al.** *Carbohydrate Polymers,* 1998, vol. 35, 155-168
- **HOVENKAMP-HERMELINK et al.** *Potato Research,* 1988, vol. 31, 241-246
- **STARCH.** Chemistry and Technology. Academic Press Inc. London Ltd, 1994
- **WATSON.** *Mais und Sorghum Stärken: Herstellung,* 412-468
- **CORBISHLEY ; MILLER.** *Tapioca, Arrowroot und Sago Stärken: Herstellung,* 469-479
- **MITCH.** *Kartoffelstärke: Herstellung und Verwendungen,* 479-490
- **KNIGHT ; OSON.** *Weizenstärke: Herstellung, Modifizierung und Verwendungen,* 491-506
- **ROHMER ; KLEM.** *Reisstärke: Herstellung und Verwendungen,* 507-528
- **ECKHOFF et al.** *Cereal Chem.,* 1996, vol. 73, 54-57
- **CORN.** *Chemistry and Technology,* 1987, vol. 16, 479-499
- **MARSHALL et al.** *The Plant Cell,* 1996, vol. 8, 1121-1135
- **LI et al.** *Plant Physiology,* 2000, vol. 123, 613-624
- **SMITH-WHITE ; PREISS.** *Plant Mol. Biol. Rep,* 1994, vol. 12, 67-71
- **LARSSON et al.** *Plant Mol. Biol.,* 1998, vol. 37, 505-511
- **BABA et al.** *Biochem. Biophys. Res. Commun.,* 1991, vol. 181 (1), 87-94
- **KIM et al.** *Gene,* 1998, vol. 216, 233-243
- **KHOSHNOODI et al.** *Eur. J. Biochem.,* 1996, vol. 242 (1), 148-155
- **THOMPSON et al.** *Nucleic Acids Research,* 1994, vol. 22, 4673-4680

- *Institut de Genetique et de Biologie Moléculaire et Cellulaire, ftp://ftp-igbmc.u-strasbg.fr/pub*
- **EHRENBERG ; HUSAIN.** *Mutation Research,* 1981, vol. 86, 1-113
- **MÜLLER.** *Biologisches Zentralbtatt,* 1972, vol. 91 (1), 31-48
- **JAUHAR ; SIDDIQ.** *Indian Journal of Genetics,* 1999, vol. 59 (1), 23-28
- *Cytologica,* 1977, vol. 42, 443-450
- **GUPTA ; SHARMA.** *Oryza,* 1990, vol. 27, 217-219
- **SATOH ; OMURA.** *Japanese Journal of Breeding,* 1981, vol. 31 (3), 316-326
- **ARORA et al.** *Annals of Biology,* 1992, vol. 8 (1), 65-69
- **SCARASCIA-MUGNOZZA et al.** *Mutation Breeding Review,* 1993, vol. 10, 1-28
- **SVEC et al.** *Cereal Research Communications,* 1998, vol. 26 (4), 391-396
- **SHASHIDHARA et al.** *Journal of Maharashtra Agricultural Universities,* 1990, vol. 15 (1), 20-23
- **DWIVEDI et al.** *Journal of Medicinal and Aromatic Plant Sciences,* 2000, vol. 22, 460-463
- **NAM et al.** *The Plant Cell,* 1989, vol. 1, 699-705
- **LEISTER ; DEAN.** *The Plant Journal,* 1993, vol. 4 (4), 745-750
- **CASTIGLIONI et al.** *Genetics,* 1998, vol. 149, 2039-2056
- **MEKSEM et al.** *Molecular Genetics and Genomics,* 2001, vol. 265, 207-214
- **MEYER et al.** *Molecular and General Genetics,* 1998, vol. 259, 150-160
- **KONIECZNY ; AUSUBEL.** *The Plant Journal,* 1993, vol. 4, 403-410
- **JARVIS et al.** *Plant Mol. Biol.,* 1994, vol. 24, 685-687
- **BACHEM et al.** *The Plant Journal,* 1996, vol. 9 (5), 745-753
- **QI et al.** *Nucleic Acids Research,* 2001, vol. 29 (22), 16
- **DRENKARD et al.** *Plant Physiology,* 2000, vol. 124, 1483-1492
- **CHO et al.** *Nature Genetics,* 1999, vol. 23, 203-207
- **MCCALLUM et al.** *Plant Physiology,* 2000, vol. 123, 439-442
- **HOOGKAMP et al.** *Potato Research,* 2000, vol. 43, 179-189
- **THORNEYCROFT et al.** *Journal of Experimental Botany,* 2001, vol. 52 (361), 1593-1601
- **RAMACHANDRAN ; SUNDARESAN.** *Plant Physiology and Biochemistry,* 2001, vol. 39, 234-252
- **MAES et al.** *Trends in Plant Science,* 1999, vol. 4 (3), 90-96
- **HIROCHIKA.** *Current Opinion in Plant Biology,* 2001, vol. 4, 118-122
- **HANLEY et al.** *The Plant Journal,* 2000, vol. 22 (4), 557-566
- **KUMAR ; HIROCHIKA.** *Trends in Plant Science,* 2001, vol. 6 (3), 127-134
- **GRECO et al.** *Plant Physiology,* 2001, vol. 125, 1175-1177
- **LIU et al.** *Molecular and General Genetics,* 1999, vol. 262, 413-420
- **HIROYUKI et al.** *The Plant Journal,* 1999, vol. 19 (5), 605-613
- **JEON ; GYNHEUNG.** *Plant Science,* 2001, vol. 161, 211-219
- **KOPREK et al.** *The Plant Journal,* 2000, vol. 24 (2), 253-263
- **AARTS et al.** *Nature,* 1993, vol. 363, 715-717
- **SCHMIDT ; WILLMITZER.** *Molecular and General Genetics,* 1989, vol. 220, 17-24
- **ALTMANN et al.** *Theoretical and Applied Genetics,* 1992, vol. 84, 371-383
- **TISSIER et al.** *The Plant Cell,* 1999, vol. 11, 1841-1852
- **BELZILE ; YODER.** *The Plant Journal,* 1992, vol. 2 (2), 173-179
- **FREY et al.** *Molecular and General Genetics,* 1989, vol. 217, 172-177
- **KNAPP et al.** *Molecular and General Genetics,* 1988, vol. 213, 285-290
- **KRYSAN et al.** *The Plant Cell,* 1999, vol. 11, 2283-2290
- **ATIPIROZ-LEEHAN ; FELDMANN.** *Trends in Genetics,* 1997, vol. 13 (4), 152-156
- **PARINOV ; SUNDARESAN.** *Current Opinion in Biotechnology,* 2000, vol. 11, 157-161
- **JEON ; AN.** *Plant Science,* 2001, vol. 161, 211-219
- **JEON et al.** *The Plant Journal,* 2000, vol. 22 (6), 561-570
- **YOUNG et al.** *Plamt Physiology,* 2001, vol. 125, 513-518
- **PARINOV et al.** *The Plant Cell,* 1999, vol. 11, 2263-2270
- **THORNEYCROFT et al.** *Journal of Experimental Botany,* 2001, vol. 52, 1593-1601
- **MCKINNEY et al.** *The Plant Journal,* 1995, vol. 8 (4), 613-622
- **METTE et al.** *EMBO J.,* 2000, vol. 19, 5194-5201
- **JORGENSEN.** *Trends Biotechnol.,* 1990, vol. 8, 340-344
- **NIEBEL et al.** *Top. Microbiol. Immunol.,* 1995, vol. 197, 91-103
- **FLAVELL et al.** *Curr. Top. Microbiol. Immunol.,* 1995, vol. 197, 43-46
- **PALAUQUI ; VAUCHERET.** *Plant Mol. Biol.,* 1995, vol. 29, 149-159
- **VAUCHERET et al.** *Mol. Gen. Genet.,* 1995, vol. 248, 311-317

- **DE BORNE et al.** *Mol.. Gen. Genet.,* 1994, vol. 243, 613-621
- **FEYTER et al.** *Mol. Gen. Genet.,* 1996, vol. 250, 329-338
- **KIPP et al.** *Poster Session beim 5th International Congress of Plant Molecular Biology,* 21. September 1997
- Meeting report zu Metabolic Engineering in Transgenic Plants. **R. A. DIXON ; C. J. AMTZEN.** Keystone Symposia. Copper Mountain, CO, 1997
- *TIBTECH,* vol. 15, 441-447
- **KREN et al.** *Hepatology,* 1997, vol. 25, 1462-1468
- **COLE-STRAUSS et al.** *Science,* 1996, vol. 273, 1386-1389
- **BEETHAM et al.** *PNAS,* 1999, vol. 96, 8774-8778
- **WATERHOUSE et al.** *Proc. Natl. Acad. Sci. USA,* 1998, vol. 95, 13959-13964
- **WANG ; WATERHOUSE.** *Plant Mol. Biol.,* 2000, vol. 43, 67-82
- **SINGH et al.** *Biochemical Society Transactions,* 2000, vol. 28 (6), 925-927
- **LIU et al.** *Biochemical Society Transactions,* 2000, vol. 28 (6), 927-929
- **SMITH et al.** *Nature,* 2000, vol. 407, 319-320
- **NAP et al.** *6th International Congress of Plant Molecular Biology,* 18. Juni 2000
- **OWEN.** *Bio/Technology,* 1992, vol. 10, 790-794
- **FRANKEN et al.** *Current Opinion in Biotechnology,* 1997, vol. 8, 411-416
- **WHITELAM.** *Trends Plant Sci.,* 1996, vol. 1, 268-272
- **ROCHA-SOSA et al.** *EMBO J.,* 1989, vol. 8, 23-29
- **STOCKHAUS et al.** *Proc. Natl. Acad. Sci. USA,* 1987, vol. 84, 7943-7947
- **STOCKHAUS et al.** *EMBO J.,* 1989, vol. 8, 2445-2451
- **BANSAL et al.** *Proc. Natl. Acad. Sci. USA,* 1992, vol. 89, 3654-3658
- **LEISY et al.** *Plant Mol. Biol.,* 1990, vol. 14, 41-50
- **ZHENG et al.** *Plant J.,* 1993, vol. 4, 357-366
- **YOSHIHARA et al.** *FEBS Lett.,* 1996, vol. 383, 213-218
- **WERR et al.** *EMBO J.,* 1985, vol. 4, 1373-1380
- **PEDERSEN et al.** *Cell,* 1982, vol. 29, 1015-1026
- **QUATROCCIO et al.** *Plant Mol. Biol.,* 1990, vol. 15, 81-93
- **FIEDLER et al.** *Plant Mol. Biol.,* 1993, vol. 22, 669-679
- **BÄUMLEIN et al.** *Mol. Gen. Genet.,* 1991, vol. 225, 459-467
- **GIELEN et al.** *EMBO J.,* 1989, vol. 8, 23-29
- **FRALEY et al.** *Crit. Rev. Plant Sci.,* vol. 4, 1-46
- **AN et al.** *EMBO J.,* 1985, vol. 4, 277-287
- **ROCHA-SOSA et al.** *EMBO J.,* 1989, vol. 8, 29-33
- **CHAN et al.** *Plant Mol. Biol.,* 1993, vol. 22, 491-506
- **HIEI et al.** *Plant J.,* 1994, vol. 6, 271-282
- **DENG et al.** *Science in China,* 1990, vol. 33, 28-34
- **WILMINK et al.** *Plant Cell Reports,* 1992, vol. 11, 76-80
- **MAY et al.** *Bio/Technology,* 1995, vol. 13, 486-492
- **CONNER ; DOMISSE.** *Int. J. Plant Sci.,* 1992, vol. 153, 550-555
- **RITCHIE et al.** *Transgenic Res.,* 1993, vol. 2, 252-265
- **WAN ; LEMAUX.** *Plant Physiol.,* 1994, vol. 104, 37-48
- **VASIL et al.** *Bio/Technology,* 1993, vol. 11, 1553-1558
- **RITALA et al.** *Plant Mol. Biol.,* 1994, vol. 24, 317-325
- **SPENCER et al.** *Theor. Appl. Genet.,* 1990, vol. 79, 625-631
- **FROMM et al.** *Biotechnology,* 1990, vol. 8, 833-844
- **GORDON-KAMM et al.** *Plant Cell,* 1990, vol. 2, 603-618
- **KOZIEL et al.** *Biotechnology,* 1993, vol. 11, 194-200
- **MOROC et al.** *Theor. Appl. Genet.,* 1990, vol. 80, 721-726
- **KRENS et al.** *Nature,* 1982, vol. 296, 72-74
- **NEHRA et al.** *Plant J.,* 1994, vol. 5, 285-297
- Plant Cell Culture Protocols. Humana Press, 1999
- **AMES.** *Methods in Enzymology,* 1966, vol. VIII, 115-118
- **BEVAN.** *Nucl. Acid Research,* 1984, vol. 12, 8711-8721
- **HÖFGEN ; WILLMITZER.** *Plant Sci.,* 1990, vol. 66, 221-230
- **KOSSMANN et al.** *Mol. Gen. Genet.,* 1991, vol. 230 (1-2), 39-44
- **BECKER et al.** *Plant Mol. Biol.,* 1992, vol. 20, 1195-1197
- **GIELEN et al.** *EMBO J.,* 1984, vol. 3, 835-846
- **PIETRZAK et al.** *Nucleic Acids Research,* 1986, vol. 14, 5857-5868

SEQUENCE LISTING

<110> Bayer CropScience GmbH

<120> Phosphorylierte waxy-Kartoffelstärke

<130> BCS 05-5003 EP

<140> EP05090085.1
<141> 2005-04-01

<160> 21

<170> PatentIn version 3.1

<210> 1
<211> 4167
<212> DNA
<213> Solanum tuberosum

<220>
<221> CDS
<222> (207)..(3899)
<223>

<300>
<301> Abel,G.J., Springer,F., Willmitzer,L. and Kossmann,J.
<302> Cloning and functional analysis of a cDNA encoding a novel 139 kDa
<303> Plant J.
<304> 10
<305> 6
<306> 981-991
<307> 1996
<308> X94400

<309> 1995-12-22

<313> (1)..(4167)

<300>

<308> EMBL / X94400

<309> 1997-04-16

<313> (1)..(4167)

<400> 1
```
tttttaata gatttttaaa accccattaa agcaaatacg tatataattg cagcacagat      60

acagagaggg agagagaaag atagtgtgtt gatgaaggag aagagagata tttcacatgg     120

gatgttctat ttgattctgt ggtgaacaag agttttacaa agaacattcc tttttctttt     180

tttcttggtt cttgtgtggg tcagcc atg gat gtt cca ttt cca ctg cat aga      233
                             Met Asp Val Pro Phe Pro Leu His Arg
                             1               5

cca ttg agt tgc aca agt gtc tcc aat gca ata acc cac ctc aag atc       281
Pro Leu Ser Cys Thr Ser Val Ser Asn Ala Ile Thr His Leu Lys Ile
10                  15                  20                  25

aaa cct ttt ctt ggg ttt gtc tct cat gga acc aca agt cta tca gta       329
Lys Pro Phe Leu Gly Phe Val Ser His Gly Thr Thr Ser Leu Ser Val
                30                  35                  40

caa tct tct tca tgg agg aag gat gga atg gtt act ggg gtt tca ttt       377
Gln Ser Ser Ser Trp Arg Lys Asp Gly Met Val Thr Gly Val Ser Phe
            45                  50                  55

cca ttt tgt gca aat ctc tcg gga aga aga cgg aga aaa gtt tca act       425
Pro Phe Cys Ala Asn Leu Ser Gly Arg Arg Arg Arg Lys Val Ser Thr
        60                  65                  70

act agg agt caa gga tct tca cct aag ggg ttt gtg cca agg aag ccc       473
Thr Arg Ser Gln Gly Ser Ser Pro Lys Gly Phe Val Pro Arg Lys Pro
    75                  80                  85

tca ggg atg agc acg caa aga aag gtt cag aag agc aat ggt gat aaa       521
Ser Gly Met Ser Thr Gln Arg Lys Val Gln Lys Ser Asn Gly Asp Lys
90                  95                  100                 105

gaa agt caa agt act tca aca tct aaa gaa tct gaa att tcc aac cag       569
Glu Ser Gln Ser Thr Ser Thr Ser Lys Glu Ser Glu Ile Ser Asn Gln
                110                 115                 120

aag acg gtt gaa gca aga gtt gaa act agt gac gat gac act aaa gta       617
Lys Thr Val Glu Ala Arg Val Glu Thr Ser Asp Asp Asp Thr Lys Val
            125                 130                 135

gtg gtg agg gac cac aag ttt ctg gag gat gag gat gaa atc aat ggt       665
Val Val Arg Asp His Lys Phe Leu Glu Asp Glu Asp Glu Ile Asn Gly
        140                 145                 150

tct act aaa tca ata agt atg tca cct gtt cgt gta tca tct caa ttt       713
Ser Thr Lys Ser Ile Ser Met Ser Pro Val Arg Val Ser Ser Gln Phe
    155                 160                 165

gtt gaa agt gaa gaa act ggt ggt gat gac aag gat gct gta aag tta       761
```

```
Val Glu Ser Glu Glu Thr Gly Gly Asp Asp Lys Asp Ala Val Lys Leu
170                 175                 180                 185

aac aaa tca aag aga tcg gaa gag agt gat ttt cta att gat tct gta       809
Asn Lys Ser Lys Arg Ser Glu Glu Ser Asp Phe Leu Ile Asp Ser Val
                190                 195                 200

ata aga gaa caa agt gga tct cag ggg gaa act aat gcc agt agc aag       857
Ile Arg Glu Gln Ser Gly Ser Gln Gly Glu Thr Asn Ala Ser Ser Lys
            205                 210                 215

gga agc cat gct gtg ggt aca aaa ctt tat gag ata ttg cag gtg gat       905
Gly Ser His Ala Val Gly Thr Lys Leu Tyr Glu Ile Leu Gln Val Asp
        220                 225                 230

gtt gag cca caa caa ttg aaa gaa aat aat gct ggg aat gtt gaa tac       953
Val Glu Pro Gln Gln Leu Lys Glu Asn Asn Ala Gly Asn Val Glu Tyr
    235                 240                 245

aaa gga cct gta gca agt aag cta ttg gaa att act aag gct agt gat      1001
Lys Gly Pro Val Ala Ser Lys Leu Leu Glu Ile Thr Lys Ala Ser Asp
250                 255                 260                 265

gtg gaa cac act gaa agc aat gag att gat gac tta gac act aat agt      1049
Val Glu His Thr Glu Ser Asn Glu Ile Asp Asp Leu Asp Thr Asn Ser
                270                 275                 280

ttc ttt aaa tca gat tta att gaa gag gat gag cca tta gct gca gga      1097
Phe Phe Lys Ser Asp Leu Ile Glu Glu Asp Glu Pro Leu Ala Ala Gly
            285                 290                 295

aca gtg gag act gga gat tct tct cta aac tta aga ttg gag atg gaa      1145
Thr Val Glu Thr Gly Asp Ser Ser Leu Asn Leu Arg Leu Glu Met Glu
        300                 305                 310

gca aat cta cgt agg cag gct ata gaa agg ctt gcc gag gaa aat tta      1193
Ala Asn Leu Arg Arg Gln Ala Ile Glu Arg Leu Ala Glu Glu Asn Leu
    315                 320                 325

ttg caa ggg atc aga tta ttt tgt ttt cca gag gtt gta aaa cct gat      1241
Leu Gln Gly Ile Arg Leu Phe Cys Phe Pro Glu Val Val Lys Pro Asp
330                 335                 340                 345

gaa gat gtc gag ata ttt ctt aac aga ggt ctt tcc act ttg aag aat      1289
Glu Asp Val Glu Ile Phe Leu Asn Arg Gly Leu Ser Thr Leu Lys Asn
                350                 355                 360

gag tct gat gtc ttg att atg gga gct ttt aat gag tgg cgc tat agg      1337
Glu Ser Asp Val Leu Ile Met Gly Ala Phe Asn Glu Trp Arg Tyr Arg
            365                 370                 375

tct ttt act aca agg cta act gag act cat ctc aat gga gat tgg tgg      1385
Ser Phe Thr Thr Arg Leu Thr Glu Thr His Leu Asn Gly Asp Trp Trp
        380                 385                 390

tct tgc aag atc cat gtt ccc aag gaa gca tac agg gct gat ttt gtg      1433
Ser Cys Lys Ile His Val Pro Lys Glu Ala Tyr Arg Ala Asp Phe Val
    395                 400                 405

ttt ttt aat gga caa gat gtc tat gac aac aat gat gga aat gac ttc      1481
Phe Phe Asn Gly Gln Asp Val Tyr Asp Asn Asn Asp Gly Asn Asp Phe
410                 415                 420                 425

agt ata act gtg aaa ggt ggt atg caa atc att gac ttt gaa aat ttc      1529
Ser Ile Thr Val Lys Gly Gly Met Gln Ile Ile Asp Phe Glu Asn Phe
                430                 435                 440

ttg ctt gag gag aaa tgg aga gaa cag gag aaa ctt gct aaa gaa caa      1577
```

```
Leu Leu Glu Glu Lys Trp Arg Glu Gln Glu Lys Leu Ala Lys Glu Gln
            445                 450                 455

gct gaa aga gaa aga cta gcg gaa gaa caa aga cga ata gaa gca gag        1625
Ala Glu Arg Glu Arg Leu Ala Glu Glu Gln Arg Arg Ile Glu Ala Glu
        460                 465                 470

aaa gct gaa att gaa gct gac aga gca caa gca aag gaa gag gct gca        1673
Lys Ala Glu Ile Glu Ala Asp Arg Ala Gln Ala Lys Glu Glu Ala Ala
    475                 480                 485

aag aaa aag aaa gta ttg cga gaa ttg atg gta aaa gcc acg aag act        1721
Lys Lys Lys Lys Val Leu Arg Glu Leu Met Val Lys Ala Thr Lys Thr
490                 495                 500                 505

cgt gat atc acg tgg tac ata gag cca agt gaa ttt aaa tgc gag gac        1769
Arg Asp Ile Thr Trp Tyr Ile Glu Pro Ser Glu Phe Lys Cys Glu Asp
                510                 515                 520

aag gtc agg tta tac tat aac aaa agt tca ggt cct ctc tcc cat gct        1817
Lys Val Arg Leu Tyr Tyr Asn Lys Ser Ser Gly Pro Leu Ser His Ala
            525                 530                 535

aag gac ttg tgg atc cac gga gga tat aat aat tgg aag gat ggt ttg        1865
Lys Asp Leu Trp Ile His Gly Gly Tyr Asn Asn Trp Lys Asp Gly Leu
        540                 545                 550

tct att gtc aaa aag ctt gtt aaa tct gag aga ata gat ggt gat tgg        1913
Ser Ile Val Lys Lys Leu Val Lys Ser Glu Arg Ile Asp Gly Asp Trp
    555                 560                 565

tgg tat aca gag gtt gtt att cct gat cag gca ctt ttc ttg gat tgg        1961
Trp Tyr Thr Glu Val Val Ile Pro Asp Gln Ala Leu Phe Leu Asp Trp
570                 575                 580                 585

gtt ttt gct gat ggt cca ccc aag cat gcc att gct tat gat aac aat        2009
Val Phe Ala Asp Gly Pro Pro Lys His Ala Ile Ala Tyr Asp Asn Asn
                590                 595                 600

cac cgc caa gac ttc cat gcc att gtc ccc aac cac att ccg gag gaa        2057
His Arg Gln Asp Phe His Ala Ile Val Pro Asn His Ile Pro Glu Glu
            605                 610                 615

tta tat tgg gtt gag gaa gaa cat cag atc ttt aag aca ctt cag gag        2105
Leu Tyr Trp Val Glu Glu Glu His Gln Ile Phe Lys Thr Leu Gln Glu
        620                 625                 630

gag aga agg ctt aga gaa gcg gct atg cgt gct aag gtt gaa aaa aca        2153
Glu Arg Arg Leu Arg Glu Ala Ala Met Arg Ala Lys Val Glu Lys Thr
    635                 640                 645

gca ctt ctg aaa act gaa aca aag gaa aga act atg aaa tca ttt tta        2201
Ala Leu Leu Lys Thr Glu Thr Lys Glu Arg Thr Met Lys Ser Phe Leu
650                 655                 660                 665

ctg tct cag aag cat gta gta tat act gag cct ctt gat atc caa gct        2249
Leu Ser Gln Lys His Val Val Tyr Thr Glu Pro Leu Asp Ile Gln Ala
                670                 675                 680

gga agc agc gtc aca gtt tac tat aat ccc gcc aat aca gta ctt aat        2297
Gly Ser Ser Val Thr Val Tyr Tyr Asn Pro Ala Asn Thr Val Leu Asn
            685                 690                 695

ggt aaa cct gaa att tgg ttc aga tgt tca ttt aat cgc tgg act cac        2345
Gly Lys Pro Glu Ile Trp Phe Arg Cys Ser Phe Asn Arg Trp Thr His
        700                 705                 710

cgc ctg ggt cca ttg cca cct cag aaa atg tcg cct gct gaa aat ggc        2393
```

```
Arg Leu Gly Pro Leu Pro Pro Gln Lys Met Ser Pro Ala Glu Asn Gly
    715                 720                 725

acc cat gtc aga gca act gtg aag gtt cca ttg gat gca tat atg atg      2441
Thr His Val Arg Ala Thr Val Lys Val Pro Leu Asp Ala Tyr Met Met
730                 735                 740                 745

gat ttt gta ttt tcc gag aga gaa gat ggt ggg att ttt gac aat aag      2489
Asp Phe Val Phe Ser Glu Arg Glu Asp Gly Gly Ile Phe Asp Asn Lys
                750                 755                 760

agc gga atg gac tat cac ata cct gtg ttt gga gga gtc gct aaa gaa      2537
Ser Gly Met Asp Tyr His Ile Pro Val Phe Gly Gly Val Ala Lys Glu
            765                 770                 775

cct cca atg cat att gtc cat att gct gtc gaa atg gca cca att gca      2585
Pro Pro Met His Ile Val His Ile Ala Val Glu Met Ala Pro Ile Ala
        780                 785                 790

aag gtg gga ggc ctt ggt gat gtt gtt act agt ctt tcc cgt gct gtt      2633
Lys Val Gly Gly Leu Gly Asp Val Val Thr Ser Leu Ser Arg Ala Val
    795                 800                 805

caa gat tta aac cat aat gtg gat att atc tta cct aag tat gac tgt      2681
Gln Asp Leu Asn His Asn Val Asp Ile Ile Leu Pro Lys Tyr Asp Cys
810                 815                 820                 825

ttg aag atg aat aat gtg aag gac ttt cgg ttt cac aaa aac tac ttt      2729
Leu Lys Met Asn Asn Val Lys Asp Phe Arg Phe His Lys Asn Tyr Phe
                830                 835                 840

tgg ggt ggg act gaa ata aaa gta tgg ttt gga aag gtg gaa ggt ctc      2777
Trp Gly Gly Thr Glu Ile Lys Val Trp Phe Gly Lys Val Glu Gly Leu
            845                 850                 855

tcg gtc tat ttt ttg gag cct caa aac ggg tta ttt tcg aaa ggg tgc      2825
Ser Val Tyr Phe Leu Glu Pro Gln Asn Gly Leu Phe Ser Lys Gly Cys
        860                 865                 870

gtc tat ggt tgt agc aat gat ggt gaa cga ttt ggt ttc ttc tgt cac      2873
Val Tyr Gly Cys Ser Asn Asp Gly Glu Arg Phe Gly Phe Phe Cys His
    875                 880                 885

gcg gct ttg gag ttt ctt ctg caa ggt gga ttt agt ccg gat atc att      2921
Ala Ala Leu Glu Phe Leu Leu Gln Gly Gly Phe Ser Pro Asp Ile Ile
890                 895                 900                 905

cat tgc cat gat tgg tct agt gct cct gtt gct tgg ctc ttt aag gaa      2969
His Cys His Asp Trp Ser Ser Ala Pro Val Ala Trp Leu Phe Lys Glu
                910                 915                 920

caa tat aca cac tat ggt cta agc aaa tct cgt ata gtc ttc acg ata      3017
Gln Tyr Thr His Tyr Gly Leu Ser Lys Ser Arg Ile Val Phe Thr Ile
            925                 930                 935

cat aat ctt gaa ttt ggg gca gat ctc att ggg aga gca atg act aac      3065
His Asn Leu Glu Phe Gly Ala Asp Leu Ile Gly Arg Ala Met Thr Asn
        940                 945                 950

gca gac aaa gct aca aca gtt tca cca act tac tca cag gag gtg tct      3113
Ala Asp Lys Ala Thr Thr Val Ser Pro Thr Tyr Ser Gln Glu Val Ser
    955                 960                 965

gga aac cct gta att gcg cct cac ctt cac aag ttc cat ggt ata gtg      3161
Gly Asn Pro Val Ile Ala Pro His Leu His Lys Phe His Gly Ile Val
970                 975                 980                 985

aat ggg att gac cca gat att tgg gat cct tta aac gat aag ttc  att     3209
```

```
Asn Gly Ile Asp Pro Asp Ile Trp Asp Pro Leu Asn Asp Lys Phe Ile
                990                 995                 1000

ccg att ccg tac acc tca gaa aac gtt gtt gaa ggc aaa aca gca      3254
Pro Ile Pro Tyr Thr Ser Glu Asn Val Val Glu Gly Lys Thr Ala
            1005                1010                1015

gcc aag gaa gct ttg cag cga aaa ctt gga ctg aaa cag gct gac      3299
Ala Lys Glu Ala Leu Gln Arg Lys Leu Gly Leu Lys Gln Ala Asp
            1020                1025                1030

ctt cct ttg gta gga att atc acc cgc tta act cac cag aaa gga      3344
Leu Pro Leu Val Gly Ile Ile Thr Arg Leu Thr His Gln Lys Gly
            1035                1040                1045

atc cac ctc att aaa cat gct att tgg cgc acc ttg gaa cgg aac      3389
Ile His Leu Ile Lys His Ala Ile Trp Arg Thr Leu Glu Arg Asn
            1050                1055                1060

gga cag gta gtc ttg ctt ggt tct gct cct gat cct agg gta caa      3434
Gly Gln Val Val Leu Leu Gly Ser Ala Pro Asp Pro Arg Val Gln
            1065                1070                1075

aac gat ttt gtt aat ttg gca aat caa ttg cac tcc aaa tat aat      3479
Asn Asp Phe Val Asn Leu Ala Asn Gln Leu His Ser Lys Tyr Asn
            1080                1085                1090

gac cgc gca cga ctc tgt cta aca tat gac gag cca ctt tct cac      3524
Asp Arg Ala Arg Leu Cys Leu Thr Tyr Asp Glu Pro Leu Ser His
            1095                1100                1105

ctg ata tat gct ggt gct gat ttt att cta gtt cct tca ata ttt      3569
Leu Ile Tyr Ala Gly Ala Asp Phe Ile Leu Val Pro Ser Ile Phe
            1110                1115                1120

gag cca tgt gga cta aca caa ctt acc gct atg aga tat ggt tca      3614
Glu Pro Cys Gly Leu Thr Gln Leu Thr Ala Met Arg Tyr Gly Ser
            1125                1130                1135

att cca gtc gtg cgt aaa act gga gga ctt tat gat act gta ttt      3659
Ile Pro Val Val Arg Lys Thr Gly Gly Leu Tyr Asp Thr Val Phe
            1140                1145                1150

gat gtt gac cat gac aaa gag aga gca caa cag tgt ggt ctt gaa      3704
Asp Val Asp His Asp Lys Glu Arg Ala Gln Gln Cys Gly Leu Glu
            1155                1160                1165

cca aat gga ttc agc ttt gat gga gca gat gct ggc gga gtt gat      3749
Pro Asn Gly Phe Ser Phe Asp Gly Ala Asp Ala Gly Gly Val Asp
            1170                1175                1180

tat gct ctg aat aga gct ctc tct gct tgg tac gat ggt cgg gat      3794
Tyr Ala Leu Asn Arg Ala Leu Ser Ala Trp Tyr Asp Gly Arg Asp
            1185                1190                1195

tgg ttc aac tct tta tgc aag cag gtc atg gaa caa gat tgg tct      3839
Trp Phe Asn Ser Leu Cys Lys Gln Val Met Glu Gln Asp Trp Ser
            1200                1205                1210

tgg aac cga cct gct ctt gat tat ttg gag ctt tac cat gct gct      3884
Trp Asn Arg Pro Ala Leu Asp Tyr Leu Glu Leu Tyr His Ala Ala
            1215                1220                1225

aga aag tta gaa tag ttagtttgtg agatgctagc agaaaaattc acgagatctg  3939
Arg Lys Leu Glu
            1230

caatctgtac aggttcagtg tttgcgtctg gacagctttt ttatttccta tatcaaagta 3999
```

taaatcaagt ctacactgag atcaatagca gacagtcctc agttcatttc attttttgtg 4059

caacatatga aagagcttag cctctaataa tgtagtcatt gatgattatt tgttttggga 4119

agaaatgaga aatcaaagga tgcaaaatac tctgaaaaaa aaaaaaaa 4167

<210> 2

<211> 1230

<212> PRT

<213> Solanum tuberosum

<400> 2

Met Asp Val Pro Phe Pro Leu His Arg Pro Leu Ser Cys Thr Ser Val
1 5 10 15

Ser Asn Ala Ile Thr His Leu Lys Ile Lys Pro Phe Leu Gly Phe Val
20 25 30

Ser His Gly Thr Thr Ser Leu Ser Val Gln Ser Ser Ser Trp Arg Lys
35 40 45

Asp Gly Met Val Thr Gly Val Ser Phe Pro Phe Cys Ala Asn Leu Ser
50 55 60

Gly Arg Arg Arg Arg Lys Val Ser Thr Thr Arg Ser Gln Gly Ser Ser
65 70 75 80

Pro Lys Gly Phe Val Pro Arg Lys Pro Ser Gly Met Ser Thr Gln Arg
85 90 95

Lys Val Gln Lys Ser Asn Gly Asp Lys Glu Ser Gln Ser Thr Ser Thr
100 105 110

Ser Lys Glu Ser Glu Ile Ser Asn Gln Lys Thr Val Glu Ala Arg Val
115 120 125

Glu Thr Ser Asp Asp Asp Thr Lys Val Val Val Arg Asp His Lys Phe
130 135 140

Leu Glu Asp Glu Asp Glu Ile Asn Gly Ser Thr Lys Ser Ile Ser Met
145 150 155 160

Ser Pro Val Arg Val Ser Ser Gln Phe Val Glu Ser Glu Glu Thr Gly
165 170 175

Gly Asp Asp Lys Asp Ala Val Lys Leu Asn Lys Ser Lys Arg Ser Glu
180 185 190

```
Glu Ser Asp Phe Leu Ile Asp Ser Val Ile Arg Glu Gln Ser Gly Ser
        195                 200                 205

Gln Gly Glu Thr Asn Ala Ser Ser Lys Gly Ser His Ala Val Gly Thr
    210                 215                 220

Lys Leu Tyr Glu Ile Leu Gln Val Asp Val Glu Pro Gln Gln Leu Lys
225                 230                 235                 240

Glu Asn Asn Ala Gly Asn Val Glu Tyr Lys Gly Pro Val Ala Ser Lys
                245                 250                 255

Leu Leu Glu Ile Thr Lys Ala Ser Asp Val Glu His Thr Glu Ser Asn
            260                 265                 270

Glu Ile Asp Asp Leu Asp Thr Asn Ser Phe Phe Lys Ser Asp Leu Ile
        275                 280                 285

Glu Glu Asp Glu Pro Leu Ala Ala Gly Thr Val Glu Thr Gly Asp Ser
    290                 295                 300

Ser Leu Asn Leu Arg Leu Glu Met Glu Ala Asn Leu Arg Arg Gln Ala
305                 310                 315                 320

Ile Glu Arg Leu Ala Glu Glu Asn Leu Leu Gln Gly Ile Arg Leu Phe
                325                 330                 335

Cys Phe Pro Glu Val Val Lys Pro Asp Glu Asp Val Glu Ile Phe Leu
            340                 345                 350

Asn Arg Gly Leu Ser Thr Leu Lys Asn Glu Ser Asp Val Leu Ile Met
        355                 360                 365

Gly Ala Phe Asn Glu Trp Arg Tyr Arg Ser Phe Thr Thr Arg Leu Thr
    370                 375                 380

Glu Thr His Leu Asn Gly Asp Trp Trp Ser Cys Lys Ile His Val Pro
385                 390                 395                 400

Lys Glu Ala Tyr Arg Ala Asp Phe Val Phe Phe Asn Gly Gln Asp Val
                405                 410                 415

Tyr Asp Asn Asn Asp Gly Asn Asp Phe Ser Ile Thr Val Lys Gly Gly
            420                 425                 430

Met Gln Ile Ile Asp Phe Glu Asn Phe Leu Leu Glu Glu Lys Trp Arg
        435                 440                 445

Glu Gln Glu Lys Leu Ala Lys Glu Gln Ala Glu Arg Glu Arg Leu Ala
    450                 455                 460
```

```
Glu Glu Gln Arg Arg Ile Glu Ala Glu Lys Ala Glu Ile Glu Ala Asp
465                 470                 475                 480

Arg Ala Gln Ala Lys Glu Glu Ala Ala Lys Lys Lys Lys Val Leu Arg
                485                 490                 495

Glu Leu Met Val Lys Ala Thr Lys Thr Arg Asp Ile Thr Trp Tyr Ile
            500                 505                 510

Glu Pro Ser Glu Phe Lys Cys Glu Asp Lys Val Arg Leu Tyr Tyr Asn
        515                 520                 525

Lys Ser Ser Gly Pro Leu Ser His Ala Lys Asp Leu Trp Ile His Gly
    530                 535                 540

Gly Tyr Asn Asn Trp Lys Asp Gly Leu Ser Ile Val Lys Lys Leu Val
545                 550                 555                 560

Lys Ser Glu Arg Ile Asp Gly Asp Trp Trp Tyr Thr Glu Val Val Ile
                565                 570                 575

Pro Asp Gln Ala Leu Phe Leu Asp Trp Val Phe Ala Asp Gly Pro Pro
            580                 585                 590

Lys His Ala Ile Ala Tyr Asp Asn Asn His Arg Gln Asp Phe His Ala
        595                 600                 605

Ile Val Pro Asn His Ile Pro Glu Glu Leu Tyr Trp Val Glu Glu Glu
    610                 615                 620

His Gln Ile Phe Lys Thr Leu Gln Glu Glu Arg Arg Leu Arg Glu Ala
625                 630                 635                 640

Ala Met Arg Ala Lys Val Glu Lys Thr Ala Leu Leu Lys Thr Glu Thr
                645                 650                 655

Lys Glu Arg Thr Met Lys Ser Phe Leu Leu Ser Gln Lys His Val Val
            660                 665                 670

Tyr Thr Glu Pro Leu Asp Ile Gln Ala Gly Ser Ser Val Thr Val Tyr
        675                 680                 685

Tyr Asn Pro Ala Asn Thr Val Leu Asn Gly Lys Pro Glu Ile Trp Phe
    690                 695                 700

Arg Cys Ser Phe Asn Arg Trp Thr His Arg Leu Gly Pro Leu Pro Pro
705                 710                 715                 720

Gln Lys Met Ser Pro Ala Glu Asn Gly Thr His Val Arg Ala Thr Val
                725                 730                 735
```

Lys Val Pro Leu Asp Ala Tyr Met Met Asp Phe Val Phe Ser Glu Arg
740 745 750

Glu Asp Gly Gly Ile Phe Asp Asn Lys Ser Gly Met Asp Tyr His Ile
755 760 765

Pro Val Phe Gly Gly Val Ala Lys Glu Pro Pro Met His Ile Val His
770 775 780

Ile Ala Val Glu Met Ala Pro Ile Ala Lys Val Gly Gly Leu Gly Asp
785 790 795 800

Val Val Thr Ser Leu Ser Arg Ala Val Gln Asp Leu Asn His Asn Val
805 810 815

Asp Ile Ile Leu Pro Lys Tyr Asp Cys Leu Lys Met Asn Asn Val Lys
820 825 830

Asp Phe Arg Phe His Lys Asn Tyr Phe Trp Gly Gly Thr Glu Ile Lys
835 840 845

Val Trp Phe Gly Lys Val Glu Gly Leu Ser Val Tyr Phe Leu Glu Pro
850 855 860

Gln Asn Gly Leu Phe Ser Lys Gly Cys Val Tyr Gly Cys Ser Asn Asp
865 870 875 880

Gly Glu Arg Phe Gly Phe Phe Cys His Ala Ala Leu Glu Phe Leu Leu
885 890 895

Gln Gly Gly Phe Ser Pro Asp Ile Ile His Cys His Asp Trp Ser Ser
900 905 910

Ala Pro Val Ala Trp Leu Phe Lys Glu Gln Tyr Thr His Tyr Gly Leu
915 920 925

Ser Lys Ser Arg Ile Val Phe Thr Ile His Asn Leu Glu Phe Gly Ala
930 935 940

Asp Leu Ile Gly Arg Ala Met Thr Asn Ala Asp Lys Ala Thr Thr Val
945 950 955 960

Ser Pro Thr Tyr Ser Gln Glu Val Ser Gly Asn Pro Val Ile Ala Pro
965 970 975

His Leu His Lys Phe His Gly Ile Val Asn Gly Ile Asp Pro Asp Ile
980 985 990

Trp Asp Pro Leu Asn Asp Lys Phe Ile Pro Ile Pro Tyr Thr Ser Glu
995 1000 1005

Asn Val Val Glu Gly Lys Thr Ala Ala Lys Glu Ala Leu Gln Arg
    1010                1015                1020

Lys Leu Gly Leu Lys Gln Ala Asp Leu Pro Leu Val Gly Ile Ile
    1025                1030                1035

Thr Arg Leu Thr His Gln Lys Gly Ile His Leu Ile Lys His Ala
    1040                1045                1050

Ile Trp Arg Thr Leu Glu Arg Asn Gly Gln Val Val Leu Leu Gly
    1055                1060                1065

Ser Ala Pro Asp Pro Arg Val Gln Asn Asp Phe Val Asn Leu Ala
    1070                1075                1080

Asn Gln Leu His Ser Lys Tyr Asn Asp Arg Ala Arg Leu Cys Leu
    1085                1090                1095

Thr Tyr Asp Glu Pro Leu Ser His Leu Ile Tyr Ala Gly Ala Asp
    1100                1105                1110

Phe Ile Leu Val Pro Ser Ile Phe Glu Pro Cys Gly Leu Thr Gln
    1115                1120                1125

Leu Thr Ala Met Arg Tyr Gly Ser Ile Pro Val Val Arg Lys Thr
    1130                1135                1140

Gly Gly Leu Tyr Asp Thr Val Phe Asp Val Asp His Asp Lys Glu
    1145                1150                1155

Arg Ala Gln Gln Cys Gly Leu Glu Pro Asn Gly Phe Ser Phe Asp
    1160                1165                1170

Gly Ala Asp Ala Gly Gly Val Asp Tyr Ala Leu Asn Arg Ala Leu
    1175                1180                1185

Ser Ala Trp Tyr Asp Gly Arg Asp Trp Phe Asn Ser Leu Cys Lys
    1190                1195                1200

Gln Val Met Glu Gln Asp Trp Ser Trp Asn Arg Pro Ala Leu Asp
    1205                1210                1215

Tyr Leu Glu Leu Tyr His Ala Ala Arg Lys Leu Glu
    1220                1225                1230

<210> 3

<211> 61

<212> PRT

<213> Solanum tuberosum

<400> 3

Arg Ser Phe Thr Thr Arg Leu Thr Glu Thr His Leu Asn Gly Asp Trp
1 5 10 15

Trp Ser Cys Lys Ile His Val Pro Lys Glu Ala Tyr Arg Ala Asp Phe
20 25 30

Val Phe Phe Asn Gly Gln Asp Val Tyr Asp Asn Asn Asp Gly Asn Asp
35 40 45

Phe Ser Ile Thr Val Lys Gly Gly Met Gln Ile Ile Asp
50 55 60

<210> 4

<211> 1641

<212> DNA

<213> Solanum tuberosum

<400> 4
```
atgaagcaca gttcagctat ttccgctgtt ttgaccgatg acaattcgac aatggcaccc      60
ctagaggaag atgtcaacac tgaaaatatt ggcctcctaa atttggatcc aactttggaa     120
ccttatctag atcacttcag acacagaatg aagagatatg tggatcagaa aatgctcatt     180
gaaaaatatg agggacccct tgaggaattt gctcaaggtt atttaaaatt tggattcaac     240
agggaagatg gttgcatagt ctatcgtgaa tgggctcctg ctgctcagga agcagaagtt     300
attggcgatt tcaatggtag gaacggttct aaccacatga tggagaagga ccagtttggt     360
gtttggagta ttagaattcc tgatgttgac agtaagccag tcattccaca caactccaga     420
gttaagtttc gtttcaaaca tggtaatgga gtgtgggtag atcgtatccc tgcttggata     480
aagtatgcca ctgcagacgc cacaaagttt gcagcaccat atgatggtgt ctactgggac     540
ccaccacctt cagaaaggta ccacttcaaa taccctcgcc ctcccaaacc ccgagcccca     600
cgaatctacg aagcacatgt cggcatgagc agctctgagc cacgtgtaaa ttcgtatcgt     660
gagtttgcag atgatgtttt acctcggatt aaggcaaata actataatac tgtccagttg     720
atggccataa tggaacattc ttactatgga tcatttggat atcatgttac aaactttttt     780
gctgtgagca atagatatgg aaacccggag gacctaaagt atctgataga taaagcacat     840
agcttgggtt tacaggttct ggtggatgta gttcacagtc atgcaagcaa taatgtcact     900
gatggcctca atggctttga tattggccaa ggttctcaag aatcctactt tcatgctgga     960
gagcgagggt accataagtt gtgggatagc aggctgttca actatgccaa ttgggaggtt    1020
cttcgtttcc ttctttccaa cttgaggtgg tggctagaag agtataactt tgacggattt    1080
cgatttgatg gaataacttc tatgctgtat gttcatcatg gaatcaatat gggatttaca    1140
```

ggaaactata atgagtattt cagcgaggct acagatgttg atgctgtggt ctatttaatg 1200
ttggccaata atctgattca caagattttc ccagacgcaa ctgttattgc cgaagatgtt 1260
tctggtatgc cgggccttag ccggcctgtt tctgagggag gaattggttt tgattaccgc 1320
ctggcaatgg caatcccaga taagtggata gattatttaa agaataagaa tgatgaagat 1380
tggtccatga aggaagtaac atcgagtttg acaaatagga gatatacaga gaagtgtata 1440
gcatatgcgg agagccatga tcagtctatt gtcggtgaca agaccattgc atttctccta 1500
atgaacaaag agatgtattc tggcatgtct tgcttgacag atgcttctcc tgttgttgat 1560
gcaggaattg cgcttgacaa gatgatccat ttttttcaca atggccttgg gaggagaggg 1620
gtacctcaat ttcatgggta a 1641

<210> 5
<211> 546
<212> PRT
<213> Solanum tuberosum

<300>
<308> Swiss Prot / P30924
<309> 1993-07-26

<400> 5

Met Lys His Ser Ser Ala Ile Ser Ala Val Leu Thr Asp Asp Asn Ser
1 5 10 15

Thr Met Ala Pro Leu Glu Glu Asp Val Asn Thr Glu Asn Ile Gly Leu
20 25 30

Leu Asn Leu Asp Pro Thr Leu Glu Pro Tyr Leu Asp His Phe Arg His
35 40 45

Arg Met Lys Arg Tyr Val Asp Gln Lys Met Leu Ile Glu Lys Tyr Glu
50 55 60

Gly Pro Leu Glu Glu Phe Ala Gln Gly Tyr Leu Lys Phe Gly Phe Asn
65 70 75 80

Arg Glu Asp Gly Cys Ile Val Tyr Arg Glu Trp Ala Pro Ala Ala Gln
85 90 95

Glu Ala Glu Val Ile Gly Asp Phe Asn Gly Arg Asn Gly Ser Asn His
100 105 110

```
Met Met Glu Lys Asp Gln Phe Gly Val Trp Ser Ile Arg Ile Pro Asp
        115                 120                 125

Val Asp Ser Lys Pro Val Ile Pro His Asn Ser Arg Val Lys Phe Arg
    130                 135                 140

Phe Lys His Gly Asn Gly Val Trp Val Asp Arg Ile Pro Ala Trp Ile
145                 150                 155                 160

Lys Tyr Ala Thr Ala Asp Ala Thr Lys Phe Ala Ala Pro Tyr Asp Gly
                165                 170                 175

Val Tyr Trp Asp Pro Pro Pro Ser Glu Arg Tyr His Phe Lys Tyr Pro
            180                 185                 190

Arg Pro Pro Lys Pro Arg Ala Pro Arg Ile Tyr Glu Ala His Val Gly
        195                 200                 205

Met Ser Ser Ser Glu Pro Arg Val Asn Ser Tyr Arg Glu Phe Ala Asp
    210                 215                 220

Asp Val Leu Pro Arg Ile Lys Ala Asn Asn Tyr Asn Thr Val Gln Leu
225                 230                 235                 240

Met Ala Ile Met Glu His Ser Tyr Tyr Gly Ser Phe Gly Tyr His Val
                245                 250                 255

Thr Asn Phe Phe Ala Val Ser Asn Arg Tyr Gly Asn Pro Glu Asp Leu
            260                 265                 270

Lys Tyr Leu Ile Asp Lys Ala His Ser Leu Gly Leu Gln Val Leu Val
        275                 280                 285

Asp Val Val His Ser His Ala Ser Asn Asn Val Thr Asp Gly Leu Asn
    290                 295                 300

Gly Phe Asp Ile Gly Gln Gly Ser Gln Glu Ser Tyr Phe His Ala Gly
305                 310                 315                 320

Glu Arg Gly Tyr His Lys Leu Trp Asp Ser Arg Leu Phe Asn Tyr Ala
                325                 330                 335

Asn Trp Glu Val Leu Arg Phe Leu Leu Ser Asn Leu Arg Trp Trp Leu
            340                 345                 350

Glu Glu Tyr Asn Phe Asp Gly Phe Arg Phe Asp Gly Ile Thr Ser Met
        355                 360                 365

Leu Tyr Val His His Gly Ile Asn Met Gly Phe Thr Gly Asn Tyr Asn
    370                 375                 380
```

Glu Tyr Phe Ser Glu Ala Thr Asp Val Asp Ala Val Val Tyr Leu Met
385                 390                 395                 400

Leu Ala Asn Asn Leu Ile His Lys Ile Phe Pro Asp Ala Thr Val Ile
                405                 410                 415

Ala Glu Asp Val Ser Gly Met Pro Gly Leu Ser Arg Pro Val Ser Glu
            420                 425                 430

Gly Gly Ile Gly Phe Asp Tyr Arg Leu Ala Met Ala Ile Pro Asp Lys
        435                 440                 445

Trp Ile Asp Tyr Leu Lys Asn Lys Asn Asp Glu Asp Trp Ser Met Lys
    450                 455                 460

Glu Val Thr Ser Ser Leu Thr Asn Arg Arg Tyr Thr Glu Lys Cys Ile
465                 470                 475                 480

Ala Tyr Ala Glu Ser His Asp Gln Ser Ile Val Gly Asp Lys Thr Ile
                485                 490                 495

Ala Phe Leu Leu Met Asn Lys Glu Met Tyr Ser Gly Met Ser Cys Leu
            500                 505                 510

Thr Asp Ala Ser Pro Val Val Asp Ala Gly Ile Ala Leu Asp Lys Met
        515                 520                 525

Ile His Phe Phe His Asn Gly Leu Gly Arg Arg Gly Val Pro Gln Phe
    530                 535                 540

His Gly
545

<210> 6

<211> 1824

<212> DNA

<213> Solanum tuberosum

<400> 6
atggcaagca tcacagcttc acaccacttt gtgtcaagaa gccaaacttc actagacacc 60

aaatcaacct tgtcacagat aggactcagg aaccatactc tgactcacaa tggtttaagg 120

gctgttaaca agcttgatgg gctccaatca agaactaata ctaaggtaac acccaagatg 180

gcatccagaa ctgagaccaa gagacctgga tgctcagcta ccattgtttg tggaaaggga 240

atgaacttga tctttgtggg tactgaggtt ggtccttgga gcaaaactgg tggactaggt 300

gatgttcttg gtggactacc accagccctt gcagcccgcg gacatcgggt aatgacaata 360

tccccccgtt atgaccaata caaagatgct tgggatacta gcgttgcggt tgaggtcaaa 420

gttggagaca gcattgaaat tgttcgtttc tttcactgct ataaacgtgg ggttgatcgt 480
gtttttgttg accacccaat gttcttggag aaagtttggg gcaaaactgg ttcaaaaatc 540
tatggcccca aagctggact agattatctg gacaatgaac ttaggttcag cttgttgtgt 600
caagcagccc tagaggcacc taaagttttg aatttgaaca gtagcaacta cttctcagga 660
ccatatggag aggatgttct cttcattgcc aatgattggc acacagctct cattccttgc 720
tacttgaagt caatgtacca gtccagagga atctatttga atgccaaggt cgctttctgc 780
atccataaca ttgcctacca aggccgattt tctttctctg acttccctct tctcaatctt 840
cctgatgaat tcaggggttc ttttgatttc attgatggat atgagaagcc tgttaagggt 900
aggaaaatca actggatgaa ggctgggata ttagaatcac atagggtggt tacagtgagc 960
ccatactatg cccaagaact tgtctctgct gttgacaagg gtgttgaatt ggacagtgtc 1020
cttcgtaaga cttgcataac tgggattgtg aatggcatgg atacacaaga gtggaaccca 1080
gcgactgaca aatacacaga tgtcaaatac gatataacca ctgtcatgga cgcaaaacct 1140
ttactaaagg aggctcttca agcagcagtt ggcttgcctg ttgacaagaa gatccctttg 1200
attggcttca tcggcagact tgaggagcag aaaggttcag atattcttgt tgctgcaatt 1260
cacaagttca tcggattgga tgttcaaatt gtagtccttg gaactggcaa aaaggagttt 1320
gagcaggaga ttgaacagct cgaagtgttg taccctaaca aagctaaagg agtggcaaaa 1380
ttcaatgtcc ctttggctca catgatcact gctggtgctg attttatgtt ggttccaagc 1440
agatttgaac cttgtggtct cattcagtta catgctatgc gatatggaac agtgccaatc 1500
tgtgcatcga ctggtggact tgttgacact gtgaaagaag gctatactgg attccatatg 1560
ggagccttca atgttgaatg cgatgttgtt gacccagctg atgtgcttaa gatagtaaca 1620
acagttgcta gagctcttgc agtctatggc accctcgcat ttgctgagat gataaaaaat 1680
tgcatgtcag aggaactctc ctggaaggaa cctgccaaga aatgggagac attgctattg 1740
ggcttaggag cttctggcag tgaacccggt gttgaagggg aagaaatcgc tccacttgcc 1800
aaggaaaatg tagccactcc ctaa 1824

<210> 7

<211> 607

<212> PRT

<213> Solanum tuberosum

<400> 7

```
Met Ala Ser Ile Thr Ala Ser His His Phe Val Ser Arg Ser Gln Thr
1               5                   10                  15

Ser Leu Asp Thr Lys Ser Thr Leu Ser Gln Ile Gly Leu Arg Asn His
            20                  25                  30
```

```
Thr Leu Thr His Asn Gly Leu Arg Ala Val Asn Lys Leu Asp Gly Leu
        35                  40                  45

Gln Ser Arg Thr Asn Thr Lys Val Thr Pro Lys Met Ala Ser Arg Thr
    50                  55                  60

Glu Thr Lys Arg Pro Gly Cys Ser Ala Thr Ile Val Cys Gly Lys Gly
65                  70                  75                  80

Met Asn Leu Ile Phe Val Gly Thr Glu Val Gly Pro Trp Ser Lys Thr
                85                  90                  95

Gly Gly Leu Gly Asp Val Leu Gly Gly Leu Pro Pro Ala Leu Ala Ala
            100                 105                 110

Arg Gly His Arg Val Met Thr Ile Ser Pro Arg Tyr Asp Gln Tyr Lys
        115                 120                 125

Asp Ala Trp Asp Thr Ser Val Ala Val Glu Val Lys Val Gly Asp Ser
    130                 135                 140

Ile Glu Ile Val Arg Phe Phe His Cys Tyr Lys Arg Gly Val Asp Arg
145                 150                 155                 160

Val Phe Val Asp His Pro Met Phe Leu Glu Lys Val Trp Gly Lys Thr
                165                 170                 175

Gly Ser Lys Ile Tyr Gly Pro Lys Ala Gly Leu Asp Tyr Leu Asp Asn
            180                 185                 190

Glu Leu Arg Phe Ser Leu Leu Cys Gln Ala Ala Leu Glu Ala Pro Lys
        195                 200                 205

Val Leu Asn Leu Asn Ser Ser Asn Tyr Phe Ser Gly Pro Tyr Gly Glu
    210                 215                 220

Asp Val Leu Phe Ile Ala Asn Asp Trp His Thr Ala Leu Ile Pro Cys
225                 230                 235                 240

Tyr Leu Lys Ser Met Tyr Gln Ser Arg Gly Ile Tyr Leu Asn Ala Lys
                245                 250                 255

Val Ala Phe Cys Ile His Asn Ile Ala Tyr Gln Gly Arg Phe Ser Phe
            260                 265                 270

Ser Asp Phe Pro Leu Leu Asn Leu Pro Asp Glu Phe Arg Gly Ser Phe
        275                 280                 285

Asp Phe Ile Asp Gly Tyr Glu Lys Pro Val Lys Gly Arg Lys Ile Asn
    290                 295                 300
```

Trp Met Lys Ala Gly Ile Leu Glu Ser His Arg Val Val Thr Val Ser
305                 310                 315                 320

Pro Tyr Tyr Ala Gln Glu Leu Val Ser Ala Val Asp Lys Gly Val Glu
                325                 330                 335

Leu Asp Ser Val Leu Arg Lys Thr Cys Ile Thr Gly Ile Val Asn Gly
            340                 345                 350

Met Asp Thr Gln Glu Trp Asn Pro Ala Thr Asp Lys Tyr Thr Asp Val
        355                 360                 365

Lys Tyr Asp Ile Thr Thr Val Met Asp Ala Lys Pro Leu Leu Lys Glu
    370                 375                 380

Ala Leu Gln Ala Ala Val Gly Leu Pro Val Asp Lys Lys Ile Pro Leu
385                 390                 395                 400

Ile Gly Phe Ile Gly Arg Leu Glu Glu Gln Lys Gly Ser Asp Ile Leu
                405                 410                 415

Val Ala Ala Ile His Lys Phe Ile Gly Leu Asp Val Gln Ile Val Val
            420                 425                 430

Leu Gly Thr Gly Lys Lys Glu Phe Glu Gln Glu Ile Glu Gln Leu Glu
        435                 440                 445

Val Leu Tyr Pro Asn Lys Ala Lys Gly Val Ala Lys Phe Asn Val Pro
    450                 455                 460

Leu Ala His Met Ile Thr Ala Gly Ala Asp Phe Met Leu Val Pro Ser
465                 470                 475                 480

Arg Phe Glu Pro Cys Gly Leu Ile Gln Leu His Ala Met Arg Tyr Gly
                485                 490                 495

Thr Val Pro Ile Cys Ala Ser Thr Gly Gly Leu Val Asp Thr Val Lys
            500                 505                 510

Glu Gly Tyr Thr Gly Phe His Met Gly Ala Phe Asn Val Glu Cys Asp
        515                 520                 525

Val Val Asp Pro Ala Asp Val Leu Lys Ile Val Thr Thr Val Ala Arg
    530                 535                 540

Ala Leu Ala Val Tyr Gly Thr Leu Ala Phe Ala Glu Met Ile Lys Asn
545                 550                 555                 560

Cys Met Ser Glu Glu Leu Ser Trp Lys Glu Pro Ala Lys Lys Trp Glu
                565                 570                 575

Thr Leu Leu Leu Gly Leu Gly Ala Ser Gly Ser Glu Pro Gly Val Glu
580 585 590

Gly Glu Glu Ile Ala Pro Leu Ala Lys Glu Asn Val Ala Thr Pro
595 600 605

<210> 8

<211> 31

<212> DNA

<213> Artificial sequence

<220>

<223> Oligonucleotide

<400> 8
gatgggtacc agcacttcta cttggcagag g 31

<210> 9

<211> 1004

<212> DNA

<213> Solanum tuberosum

<400> 9
tcaaactagt cacaaccagt ccatttctgg aggtcgttcc ttcgcagaaa tactgattgg 60
taactccttg gggaaatcct ccatatcaca agagtcatta cttagaggct gctcgttaca 120
caagatgatc agattaatta catctacaat tggtggtcat gcatacctca acttcatggg 180
caatgaattt ggtcacccaa agagagtaga gtttccaatg tcaagcaaca atttctcctt 240
ttcactggct aaccgtcgct gggatctatt ggaagatgtt gtacattatc aattgttctc 300
atttgataag ggtatgatgg acttggataa aaatgggaga attttgtcca gaggtcttgc 360
caacattcac catgtcaatg atactaccat ggtgatttct tacttgagag gtcccaatct 420
ctttgtgttc aactttcatc ctgtcaattc atatgaaaga tacattatag gtgtggaaga 480
agctggagag tatcaagtca cattaaatac agatgaaaac aagtatggtg gtagaggact 540
acttggccat gatcagaata ttcaaagaac cattagtaga agagctgatg gaatgagatt 600
ttgcttggaa gtgcctctgc caagtagaag tgctcaggtc tacaagttga cccgaattct 660
aagagcatga tcactctagt aatcaaagtg cctcatatga tgacacaaaa ggaaaggttc 720
tacattgccc ttacactgat caatattgac acctttccga ggtgagtttc tgtgattctt 780
gagcagactg ttggctagtc aattatcatg aacttttgcc ttcagcatcc ggatagtcgc 840
ttctcctgtg caatgagggc atggacgaat tttttttgg cttgtcatgg gggtcataag 900

catccgccag attaagattt cacaggcctc gagtaaaacc atcacttact ttaaggatac 960

acaaacacac caacggggtg caggctctga taccttctaa agtg 1004

<210> 10

<211> 2096

<212> DNA

<213> Solanum tuberosum

<400> 10

aacaatgctc tctctgtcgg attcaattcg aatttcttca ccattgagcg attctcgtct 60

tagttttcta tctcaaaccg gaagcagaac cagtcgccag cttaaatttg ttcgcagccg 120

ccgggctcga gtttcgaggt gtagatgctc agcaacggag caaccgccac cgcaacgacg 180

gaagcaacga ccggagaagt acaaacagtc ggaggaaggg aaaggaatcg atcctgttgg 240

atttctcagc aaatacggca ttactcataa agcgtttgct caatttcttc gtgaaagata 300

taaatcattg aaggacttga aggatgaaat attgactcgt catttcagtc tcaaggagat 360

gtctactggg tatgaattaa tgggtatgca tcgcaacata caacatcgag tggatttctt 420

ggaatgggct ccaggtgctc gctactgtgc tctgattggt gacttcaatg ggtggtcaac 480

aactggtaac tgtgccagag agggtcattt tggtcatgac gattatgggt attggtttat 540

tattcttgaa gataaattac gtgaaggaga agaacctgat aaattgtatt ttcaacagta 600

caattatgcg gaggactatg gtaaaggtga cacgggtatt accgtcgagg aaatctttaa 660

aaaagcaaat gatgagtatt gggaacctgg agaagatcgc ttcattaaat cacgttatga 720

ggtggcagca aagttatatg aggaaatgtt cggaccaaat ggacctcaaa cagaagagga 780

actagaagca atgcctgatg cagctacacg atacaaaact tggaaagagc aacaaaaaga 840

ggatccggca agcaatttgc catcgtatga tgtggtagat agtggaaaag aatatgatat 900

ttacaatatt ataggtgatc ctgaatcgtt taagaaattt cgtatgaaac agcctcctat 960

tgcttactgg ttagaaacta aaaagggaag gaaaggctgg ttacagaaat atatgcctgc 1020

tttacctcat ggaagcaaat acagggtgta ttttaacaca ccaaatgggc ctcttgaacg 1080

agttcctgcg tgggccaatt ttgtcattcc agatgcaggc gggatggcat tagcagtcca 1140

ttgggaacca cctcctgaat atgcttataa atggaaacac aagctaccag tcaagcctaa 1200

gtccttgcgc atatatgaat gtcatgttgg catctctggc caggaaccaa aagtttcatc 1260

tttcaatgat tttattagca aggtccttcc gcatgtaaaa gaagctggat acaatgcaat 1320

acaaattatt ggagttgttg agcacaagga ttatttcact gttggatata gagtgaccaa 1380

tttttatgct gttagtagcc gttatggcac accggatgac ttcaagcgct tggttgatga 1440

agcacatggg cttggactgc ttgtcttttt ggagattgtg cactcttatg cagcagcaga 1500

tgaaatggtt gggttatctc tttttgatgg agcaaatgat tgctatttcc acactggtaa 1560

```
acgtggacac cacaaattct ggggcacacg gatgttcaaa tatggagatc ttgatgttct   1620

gcactttctt ctttcaaatc tgaactggtg ggtggaggag tatcatgtcg atggcttcca   1680

ttttcattcg ctctcgtcca tgttgtatac gcataatgga tttgcttcat ttactggtga   1740

catggatgaa tactgtaacc aatatgttga caaggaggcc ttattgtacc tcatattagc   1800

aaatgaagta ttacatgctc ttcatcctaa tgtgatcacg attgctgagg atgcaactct   1860

gtatcctgga ctctgcgatc caacatctca aggtggactg ggctttgatt attttgccaa   1920

tctttctgcc tcagagatgt ggcttgcatt acttgaaaat actcctgatc atgaatggtg   1980

catgagtaag attgttagca cattagtggg cgatagacaa aatactgata aaatgctttt   2040

gtatgcagaa aatcacaacc agtccatttc tggaggtcgt tccttcgcag aaatac       2096
```

<210> 11

<211> 3204

<212> DNA

<213> Solanum tuberosum

<220>

<221> CDS

<222> (99)..(2804)

<223>

<400> 11

```
gaattgtaat acgactcact atagggcgaa ttgggccctc tagatgcatg ctcgagcggc     60

cgccagtgtg atggatatct gcagaattcg gcttaaca atg ctc tct ctg tcg gat    116
                                          Met Leu Ser Leu Ser Asp
                                          1               5

tca att cga att tct tca cca ttg agc gat tct cgt ctt agt ttt cta      164
Ser Ile Arg Ile Ser Ser Pro Leu Ser Asp Ser Arg Leu Ser Phe Leu
            10                  15                  20

tct caa acc gga agc aga acc agt cgc cag ctt aaa ttt gtt cgc agc      212
Ser Gln Thr Gly Ser Arg Thr Ser Arg Gln Leu Lys Phe Val Arg Ser
        25                  30                  35

cgc cgg gct cga gtt tcg agg tgt aga tgc tca gca acg gag caa ccg      260
Arg Arg Ala Arg Val Ser Arg Cys Arg Cys Ser Ala Thr Glu Gln Pro
    40                  45                  50

cca ccg caa cga cgg aag caa cga ccg gag aag tac aaa cag tcg gag      308
Pro Pro Gln Arg Arg Lys Gln Arg Pro Glu Lys Tyr Lys Gln Ser Glu
55                  60                  65                  70

gaa gag aaa gga atc gat cct gtt gga ttt ctc agc aaa tac ggc att      356
Glu Glu Lys Gly Ile Asp Pro Val Gly Phe Leu Ser Lys Tyr Gly Ile
                75                  80                  85

act cat aaa gcg ttt gct caa ttt ctt cgt gaa aga tat aaa tca ttg      404
```

```
Thr His Lys Ala Phe Ala Gln Phe Leu Arg Glu Arg Tyr Lys Ser Leu
                90                  95                  100

aag gac ttg aag gat gaa ata ttg act cgt cat ttc agt ctc aag gag      452
Lys Asp Leu Lys Asp Glu Ile Leu Thr Arg His Phe Ser Leu Lys Glu
        105                 110                 115

atg tct act ggg tat gaa tta atg ggt atg cat cgc aac ata caa cat      500
Met Ser Thr Gly Tyr Glu Leu Met Gly Met His Arg Asn Ile Gln His
    120                 125                 130

cga gtg gat ttc ttg gaa tgg gct cca ggt gct cgc tac tgt gct ctg      548
Arg Val Asp Phe Leu Glu Trp Ala Pro Gly Ala Arg Tyr Cys Ala Leu
135                 140                 145                 150

att ggt gac ttc aat ggg tgg tca aca act ggt aac tgt gcc aga gag      596
Ile Gly Asp Phe Asn Gly Trp Ser Thr Thr Gly Asn Cys Ala Arg Glu
                155                 160                 165

ggt cat ttt ggt cat gac gat tat ggg tat tgg ttt att att ctt gaa      644
Gly His Phe Gly His Asp Asp Tyr Gly Tyr Trp Phe Ile Ile Leu Glu
            170                 175                 180

gat aaa tta cgt gaa gga gaa gaa cct gat aaa ttg tat ttt caa cag      692
Asp Lys Leu Arg Glu Gly Glu Glu Pro Asp Lys Leu Tyr Phe Gln Gln
        185                 190                 195

tac aat tat gcg gag gac tat gat aaa ggt gac acg ggt att acc gtc      740
Tyr Asn Tyr Ala Glu Asp Tyr Asp Lys Gly Asp Thr Gly Ile Thr Val
    200                 205                 210

gag gaa atc ttt aaa aaa gca aat gat gag tat tgg gaa cct gga gaa      788
Glu Glu Ile Phe Lys Lys Ala Asn Asp Glu Tyr Trp Glu Pro Gly Glu
215                 220                 225                 230

gat cgc ttc att aaa tca cgt tat gag gtg gca gca aag tta tat gag      836
Asp Arg Phe Ile Lys Ser Arg Tyr Glu Val Ala Ala Lys Leu Tyr Glu
                235                 240                 245

gaa atg ttc gga cca aat gga cct caa aca gaa gag gaa cta gaa gca      884
Glu Met Phe Gly Pro Asn Gly Pro Gln Thr Glu Glu Glu Leu Glu Ala
            250                 255                 260

atg cct gat gca gct aca cga tac aaa act tgg aaa gag caa caa aaa      932
Met Pro Asp Ala Ala Thr Arg Tyr Lys Thr Trp Lys Glu Gln Gln Lys
        265                 270                 275

aag gat ccg gca agc aat ttg cca tcg tat gat gtg gta gat agt gga      980
Lys Asp Pro Ala Ser Asn Leu Pro Ser Tyr Asp Val Val Asp Ser Gly
    280                 285                 290

aaa gaa tat gat att tac aat att ata ggt gat cct gaa tcg ttt aag     1028
Lys Glu Tyr Asp Ile Tyr Asn Ile Ile Gly Asp Pro Glu Ser Phe Lys
295                 300                 305                 310

aaa ttt cgt atg aaa cag cct cct att gct tac tgg tta gaa act aaa     1076
Lys Phe Arg Met Lys Gln Pro Pro Ile Ala Tyr Trp Leu Glu Thr Lys
                315                 320                 325

aag gga agg aaa ggc tgg tta cag aaa tat atg cct gct tta cct cat     1124
Lys Gly Arg Lys Gly Trp Leu Gln Lys Tyr Met Pro Ala Leu Pro His
            330                 335                 340

gga agc aaa cac agg gtg tat ttt aac aca cca aat ggg cct ctt gaa     1172
Gly Ser Lys His Arg Val Tyr Phe Asn Thr Pro Asn Gly Pro Leu Glu
        345                 350                 355

cga gtt cct gcg tgg gcc aat ttt gtc att cca gat gca gac ggg atg     1220
```

```
Arg Val Pro Ala Trp Ala Asn Phe Val Ile Pro Asp Ala Asp Gly Met
    360                 365                 370

gca tta gca gtc cat tgg gaa cca cct cct gaa tat gct tat aaa tgg      1268
Ala Leu Ala Val His Trp Glu Pro Pro Pro Glu Tyr Ala Tyr Lys Trp
375                 380                 385                 390

aaa cac aag cta cca gtc aag cct aag tcc ttg cgc ata tat gaa tgt      1316
Lys His Lys Leu Pro Val Lys Pro Lys Ser Leu Arg Ile Tyr Glu Cys
                395                 400                 405

cat gtt ggc atc tct ggc cag gaa cca aaa gtt tca tct ttc aat gat      1364
His Val Gly Ile Ser Gly Gln Glu Pro Lys Val Ser Ser Phe Asn Asp
            410                 415                 420

ttt att agc aag gtc ctt ccg cat gta aaa gaa gct gga tac aat gca      1412
Phe Ile Ser Lys Val Leu Pro His Val Lys Glu Ala Gly Tyr Asn Ala
        425                 430                 435

acg caa att att gga gtt gtt gag cac aag gat tat ttc act gtt gga      1460
Thr Gln Ile Ile Gly Val Val Glu His Lys Asp Tyr Phe Thr Val Gly
    440                 445                 450

tat aga gtg acc aat ttt tat gct gtt agt agc cgt tat ggc aca ccg      1508
Tyr Arg Val Thr Asn Phe Tyr Ala Val Ser Ser Arg Tyr Gly Thr Pro
455                 460                 465                 470

gat gac ttc aag cgc ttg gtt gat gaa gca cat ggg ctt gga ctg ctt      1556
Asp Asp Phe Lys Arg Leu Val Asp Glu Ala His Gly Leu Gly Leu Leu
                475                 480                 485

gtc ttt ttg gag att gtg cac tcc tat gca gca gca gat gaa atg gtt      1604
Val Phe Leu Glu Ile Val His Ser Tyr Ala Ala Ala Asp Glu Met Val
            490                 495                 500

ggg tta tct ctt ttt gat gga gca aat gat tgc tat ttc cac act ggt      1652
Gly Leu Ser Leu Phe Asp Gly Ala Asn Asp Cys Tyr Phe His Thr Gly
        505                 510                 515

aaa cgt gga cac cac aaa ttc tgg ggc aca cgg atg ttc aaa tat gga      1700
Lys Arg Gly His His Lys Phe Trp Gly Thr Arg Met Phe Lys Tyr Gly
    520                 525                 530

gat cct gat gtt ctg cac ttt ctt ctt tca aat ctg aac tgg tgg gtg      1748
Asp Pro Asp Val Leu His Phe Leu Leu Ser Asn Leu Asn Trp Trp Val
535                 540                 545                 550

gag gag tat cat gtc gat ggc ttc cat ttt cat tcg ctc tcg tcc atg      1796
Glu Glu Tyr His Val Asp Gly Phe His Phe His Ser Leu Ser Ser Met
                555                 560                 565

ttg tat acg cat aat gga ttt gct tca ttt act ggt gac atg gat gaa      1844
Leu Tyr Thr His Asn Gly Phe Ala Ser Phe Thr Gly Asp Met Asp Glu
            570                 575                 580

tac tgt aac caa tat gtt gac aag gag gcc tta ttg tac ctc ata tta      1892
Tyr Cys Asn Gln Tyr Val Asp Lys Glu Ala Leu Leu Tyr Leu Ile Leu
        585                 590                 595

gca aat gaa gta tta cat gct ctt cat cct aat gtg atc acg att gct      1940
Ala Asn Glu Val Leu His Ala Leu His Pro Asn Val Ile Thr Ile Ala
    600                 605                 610

gtg gat gca act ctg tat cct gga ctc tgc gat cca aca tct caa ggt      1988
Val Asp Ala Thr Leu Tyr Pro Gly Leu Cys Asp Pro Thr Ser Gln Gly
615                 620                 625                 630

gga ctg ggc ttt gat tat ttt gcc aat ctt tct gcc tca gag atg tgg      2036
```

```
Gly Leu Gly Phe Asp Tyr Phe Ala Asn Leu Ser Ala Ser Glu Met Trp
                635                 640                 645

ctt gca tta ctt gaa aat act cct gat cat gaa tgg tgc atg agt aag      2084
Leu Ala Leu Leu Glu Asn Thr Pro Asp His Glu Trp Cys Met Ser Lys
            650                 655                 660

att gtt agc aca tta gtg ggc gat aga caa aat act gat aaa atg ctt      2132
Ile Val Ser Thr Leu Val Gly Asp Arg Gln Asn Thr Asp Lys Met Leu
        665                 670                 675

ttg tat gca gaa aat cac aac cag tcc att tct gga ggt cgt tcc ttc      2180
Leu Tyr Ala Glu Asn His Asn Gln Ser Ile Ser Gly Gly Arg Ser Phe
    680                 685                 690

gca gaa ata ctg att ggt aac tcc ttg ggg aaa tct tcc ata tca caa      2228
Ala Glu Ile Leu Ile Gly Asn Ser Leu Gly Lys Ser Ser Ile Ser Gln
695                 700                 705                 710

gag tca tta ctt aga ggc tgc tcg tta cac aag atg atc aga tta att      2276
Glu Ser Leu Leu Arg Gly Cys Ser Leu His Lys Met Ile Arg Leu Ile
                715                 720                 725

aca tct aca att ggt ggt cat gca tac ctc aac ttc atg ggc aat gaa      2324
Thr Ser Thr Ile Gly Gly His Ala Tyr Leu Asn Phe Met Gly Asn Glu
            730                 735                 740

ttt ggt cac cca aag aga gta gag ttt cca atg tca agc aac aat ttc      2372
Phe Gly His Pro Lys Arg Val Glu Phe Pro Met Ser Ser Asn Asn Phe
        745                 750                 755

tcc ttt tca ctg gct aac cgt cgc tgg gat cta ttg gaa gat gtt gta      2420
Ser Phe Ser Leu Ala Asn Arg Arg Trp Asp Leu Leu Glu Asp Val Val
    760                 765                 770

cat tat caa tta ttc tca ttt gat aag gat atg atg gac ttg gat aaa      2468
His Tyr Gln Leu Phe Ser Phe Asp Lys Asp Met Met Asp Leu Asp Lys
775                 780                 785                 790

aat ggg aga att ttg tcc aga ggt ctt gcc aac att cac cat gtc aat      2516
Asn Gly Arg Ile Leu Ser Arg Gly Leu Ala Asn Ile His His Val Asn
                795                 800                 805

gat act acc atg gtg att tct tac ttg aga ggt ccc aat ctc ttt gtg      2564
Asp Thr Thr Met Val Ile Ser Tyr Leu Arg Gly Pro Asn Leu Phe Val
            810                 815                 820

ttc aac ttt cat cct gtc aat tca tat gaa aga tac att ata ggt gtg      2612
Phe Asn Phe His Pro Val Asn Ser Tyr Glu Arg Tyr Ile Ile Gly Val
        825                 830                 835

gaa gaa gct gga gag tat caa gtc aca tta aat aca gat gaa aac aag      2660
Glu Glu Ala Gly Glu Tyr Gln Val Thr Leu Asn Thr Asp Glu Asn Lys
    840                 845                 850

tat ggt ggt aga gga cta ctt ggc cat gat cag aat act caa aga acc      2708
Tyr Gly Gly Arg Gly Leu Leu Gly His Asp Gln Asn Thr Gln Arg Thr
855                 860                 865                 870

att agt aga aga gct gat gga atg aga ttt tgc ttg gaa gta cct ctg      2756
Ile Ser Arg Arg Ala Asp Gly Met Arg Phe Cys Leu Glu Val Pro Leu
                875                 880                 885

cca agt aga agt gct cag gtc tac aag ttg acc cga att cta aga gca      2804
Pro Ser Arg Ser Ala Gln Val Tyr Lys Leu Thr Arg Ile Leu Arg Ala
            890                 895                 900

tgatcactct agcaatcaaa gtgcctcata tgatcacaca aaagggaagg ttctacattg    2864
```

ccccttatact gaccaatatt gtggcctttc cgaggtgagt ttctgtgatt cttgagcaca 2924
ggctgttggc tagtcagtta tcatgaactt ttgccttcag catctggata agcgcttctc 2984
ctgtgcaatg agggcatgga cgaaattttt ttggttcgtc atgggagtca aaagcatctg 3044
ccagattaag atttcacagg cctcgagtaa aaccatcact tacttaggat acacaaacac 3104
atcaacgggg tgcaggctct gataccttct aaagtgaagc cgaattccag cacactggcg 3164
gccgttacta gtggatccga gctcggtacc aagcttggcg 3204

<210> 12

<211> 902

<212> PRT

<213> Solanum tuberosum

<400> 12

Met Leu Ser Leu Ser Asp Ser Ile Arg Ile Ser Ser Pro Leu Ser Asp
1 5 10 15

Ser Arg Leu Ser Phe Leu Ser Gln Thr Gly Ser Arg Thr Ser Arg Gln
20 25 30

Leu Lys Phe Val Arg Ser Arg Arg Ala Arg Val Ser Arg Cys Arg Cys
35 40 45

Ser Ala Thr Glu Gln Pro Pro Pro Gln Arg Arg Lys Gln Arg Pro Glu
50 55 60

Lys Tyr Lys Gln Ser Glu Glu Glu Lys Gly Ile Asp Pro Val Gly Phe
65 70 75 80

Leu Ser Lys Tyr Gly Ile Thr His Lys Ala Phe Ala Gln Phe Leu Arg
85 90 95

Glu Arg Tyr Lys Ser Leu Lys Asp Leu Lys Asp Glu Ile Leu Thr Arg
100 105 110

His Phe Ser Leu Lys Glu Met Ser Thr Gly Tyr Glu Leu Met Gly Met
115 120 125

His Arg Asn Ile Gln His Arg Val Asp Phe Leu Glu Trp Ala Pro Gly
130 135 140

Ala Arg Tyr Cys Ala Leu Ile Gly Asp Phe Asn Gly Trp Ser Thr Thr
145 150 155 160

Gly Asn Cys Ala Arg Glu Gly His Phe Gly His Asp Asp Tyr Gly Tyr
165 170 175

Trp Phe Ile Ile Leu Glu Asp Lys Leu Arg Glu Gly Glu Glu Pro Asp
180 185 190

Lys Leu Tyr Phe Gln Gln Tyr Asn Tyr Ala Glu Asp Tyr Asp Lys Gly
195 200 205

Asp Thr Gly Ile Thr Val Glu Glu Ile Phe Lys Lys Ala Asn Asp Glu
210 215 220

Tyr Trp Glu Pro Gly Glu Asp Arg Phe Ile Lys Ser Arg Tyr Glu Val
225 230 235 240

Ala Ala Lys Leu Tyr Glu Glu Met Phe Gly Pro Asn Gly Pro Gln Thr
245 250 255

Glu Glu Glu Leu Glu Ala Met Pro Asp Ala Ala Thr Arg Tyr Lys Thr
260 265 270

Trp Lys Glu Gln Gln Lys Lys Asp Pro Ala Ser Asn Leu Pro Ser Tyr
275 280 285

Asp Val Val Asp Ser Gly Lys Glu Tyr Asp Ile Tyr Asn Ile Ile Gly
290 295 300

Asp Pro Glu Ser Phe Lys Lys Phe Arg Met Lys Gln Pro Pro Ile Ala
305 310 315 320

Tyr Trp Leu Glu Thr Lys Lys Gly Arg Lys Gly Trp Leu Gln Lys Tyr
325 330 335

Met Pro Ala Leu Pro His Gly Ser Lys His Arg Val Tyr Phe Asn Thr
340 345 350

Pro Asn Gly Pro Leu Glu Arg Val Pro Ala Trp Ala Asn Phe Val Ile
355 360 365

Pro Asp Ala Asp Gly Met Ala Leu Ala Val His Trp Glu Pro Pro Pro
370 375 380

Glu Tyr Ala Tyr Lys Trp Lys His Lys Leu Pro Val Lys Pro Lys Ser
385 390 395 400

Leu Arg Ile Tyr Glu Cys His Val Gly Ile Ser Gly Gln Glu Pro Lys
405 410 415

Val Ser Ser Phe Asn Asp Phe Ile Ser Lys Val Leu Pro His Val Lys
420 425 430

Glu Ala Gly Tyr Asn Ala Thr Gln Ile Ile Gly Val Val Glu His Lys
435 440 445

Asp Tyr Phe Thr Val Gly Tyr Arg Val Thr Asn Phe Tyr Ala Val Ser
450 455 460

Ser Arg Tyr Gly Thr Pro Asp Asp Phe Lys Arg Leu Val Asp Glu Ala
465 470 475 480

His Gly Leu Gly Leu Leu Val Phe Leu Glu Ile Val His Ser Tyr Ala
485 490 495

Ala Ala Asp Glu Met Val Gly Leu Ser Leu Phe Asp Gly Ala Asn Asp
500 505 510

Cys Tyr Phe His Thr Gly Lys Arg Gly His His Lys Phe Trp Gly Thr
515 520 525

Arg Met Phe Lys Tyr Gly Asp Pro Asp Val Leu His Phe Leu Leu Ser
530 535 540

Asn Leu Asn Trp Trp Val Glu Glu Tyr His Val Asp Gly Phe His Phe
545 550 555 560

His Ser Leu Ser Ser Met Leu Tyr Thr His Asn Gly Phe Ala Ser Phe
565 570 575

Thr Gly Asp Met Asp Glu Tyr Cys Asn Gln Tyr Val Asp Lys Glu Ala
580 585 590

Leu Leu Tyr Leu Ile Leu Ala Asn Glu Val Leu His Ala Leu His Pro
595 600 605

Asn Val Ile Thr Ile Ala Val Asp Ala Thr Leu Tyr Pro Gly Leu Cys
610 615 620

Asp Pro Thr Ser Gln Gly Gly Leu Gly Phe Asp Tyr Phe Ala Asn Leu
625 630 635 640

Ser Ala Ser Glu Met Trp Leu Ala Leu Leu Glu Asn Thr Pro Asp His
645 650 655

Glu Trp Cys Met Ser Lys Ile Val Ser Thr Leu Val Gly Asp Arg Gln
660 665 670

Asn Thr Asp Lys Met Leu Leu Tyr Ala Glu Asn His Asn Gln Ser Ile
675 680 685

Ser Gly Gly Arg Ser Phe Ala Glu Ile Leu Ile Gly Asn Ser Leu Gly
690 695 700

Lys Ser Ser Ile Ser Gln Glu Ser Leu Leu Arg Gly Cys Ser Leu His
705 710 715 720

Lys Met Ile Arg Leu Ile Thr Ser Thr Ile Gly Gly His Ala Tyr Leu
725 730 735

Asn Phe Met Gly Asn Glu Phe Gly His Pro Lys Arg Val Glu Phe Pro
740 745 750

Met Ser Ser Asn Asn Phe Ser Phe Ser Leu Ala Asn Arg Arg Trp Asp
755 760 765

Leu Leu Glu Asp Val Val His Tyr Gln Leu Phe Ser Phe Asp Lys Asp
770 775 780

Met Met Asp Leu Asp Lys Asn Gly Arg Ile Leu Ser Arg Gly Leu Ala
785 790 795 800

Asn Ile His His Val Asn Asp Thr Thr Met Val Ile Ser Tyr Leu Arg
805 810 815

Gly Pro Asn Leu Phe Val Phe Asn Phe His Pro Val Asn Ser Tyr Glu
820 825 830

Arg Tyr Ile Ile Gly Val Glu Glu Ala Gly Glu Tyr Gln Val Thr Leu
835 840 845

Asn Thr Asp Glu Asn Lys Tyr Gly Gly Arg Gly Leu Leu Gly His Asp
850 855 860

Gln Asn Thr Gln Arg Thr Ile Ser Arg Arg Ala Asp Gly Met Arg Phe
865 870 875 880

Cys Leu Glu Val Pro Leu Pro Ser Arg Ser Ala Gln Val Tyr Lys Leu
885 890 895

Thr Arg Ile Leu Arg Ala
900

<210> 13

<211> 3047

<212> DNA

<213> Solanum tuberosum

<220>

<221> CDS

<222> (5)..(2710)

<223>

```
<400>  13
aaca atg ctc tct ctg tcg gat tca att cga att tct tca cca ttg agc         49
     Met Leu Ser Leu Ser Asp Ser Ile Arg Ile Ser Ser Pro Leu Ser
     1               5                   10                  15

gat tct cgt ctt agt ttt cta tct caa acc gga agc aga acc agt cgc          97
Asp Ser Arg Leu Ser Phe Leu Ser Gln Thr Gly Ser Arg Thr Ser Arg
                20                  25                  30

cag ctt aaa ttt gtt cgc agc cgc cgg gct cga gtt tcg agg tgt aga         145
Gln Leu Lys Phe Val Arg Ser Arg Arg Ala Arg Val Ser Arg Cys Arg
            35                  40                  45

tgc tca gca acg gag caa ccg cca ccg caa cga cgg aag caa cga ccg         193
Cys Ser Ala Thr Glu Gln Pro Pro Pro Gln Arg Arg Lys Gln Arg Pro
        50                  55                  60

gag aag tac aaa cag tcg gag gaa ggg aaa gga atc gat cct gtt gga         241
Glu Lys Tyr Lys Gln Ser Glu Glu Gly Lys Gly Ile Asp Pro Val Gly
    65                  70                  75

ttt ctc agc aaa tac ggc att act cat aaa gcg ttt gct caa ttt ctt         289
Phe Leu Ser Lys Tyr Gly Ile Thr His Lys Ala Phe Ala Gln Phe Leu
80                  85                  90                  95

cgt gaa aga tat aaa tca ttg aag gac ttg aag gat gaa ata ttg act         337
Arg Glu Arg Tyr Lys Ser Leu Lys Asp Leu Lys Asp Glu Ile Leu Thr
                100                 105                 110

cgt cat ttc agt ctc aag gag atg tct act ggg tat gaa tta atg ggt         385
Arg His Phe Ser Leu Lys Glu Met Ser Thr Gly Tyr Glu Leu Met Gly
            115                 120                 125

atg cat cgc aac ata caa cat cga gtg gat ttc ttg gaa tgg gct cca         433
Met His Arg Asn Ile Gln His Arg Val Asp Phe Leu Glu Trp Ala Pro
        130                 135                 140

ggt gct cgc tac tgt gct ctg att ggt gac ttc aat ggg tgg tca aca         481
Gly Ala Arg Tyr Cys Ala Leu Ile Gly Asp Phe Asn Gly Trp Ser Thr
    145                 150                 155

act ggt aac tgt gcc aga gag ggt cat ttt ggt cat gac gat tat ggg         529
Thr Gly Asn Cys Ala Arg Glu Gly His Phe Gly His Asp Asp Tyr Gly
160                 165                 170                 175

tat tgg ttt att att ctt gaa gat aaa tta cgt gaa gga gaa gaa cct         577
Tyr Trp Phe Ile Ile Leu Glu Asp Lys Leu Arg Glu Gly Glu Glu Pro
                180                 185                 190

gat aaa ttg tat ttt caa cag tac aat tat gcg gag gac tat ggt aaa         625
Asp Lys Leu Tyr Phe Gln Gln Tyr Asn Tyr Ala Glu Asp Tyr Gly Lys
            195                 200                 205

ggt gac acg ggt att acc gtc gag gaa atc ttt aaa aaa gca aat gat         673
Gly Asp Thr Gly Ile Thr Val Glu Glu Ile Phe Lys Lys Ala Asn Asp
        210                 215                 220

gag tat tgg gaa cct gga gaa gat cgc ttc att aaa tca cgt tat gag         721
Glu Tyr Trp Glu Pro Gly Glu Asp Arg Phe Ile Lys Ser Arg Tyr Glu
    225                 230                 235

gtg gca gca aag tta tat gag gaa atg ttc gga cca aat gga cct caa         769
Val Ala Ala Lys Leu Tyr Glu Glu Met Phe Gly Pro Asn Gly Pro Gln
240                 245                 250                 255

aca gaa gag gaa cta gaa gca atg cct gat gca gct aca cga tac aaa         817
```

```
Thr Glu Glu Glu Leu Glu Ala Met Pro Asp Ala Ala Thr Arg Tyr Lys
                260                 265                 270

act tgg aaa gag caa caa aaa gag gat ccg gca agc aat ttg cca tcg        865
Thr Trp Lys Glu Gln Gln Lys Glu Asp Pro Ala Ser Asn Leu Pro Ser
            275                 280                 285

tat gat gtg gta gat agt gga aaa gaa tat gat att tac aat att ata        913
Tyr Asp Val Val Asp Ser Gly Lys Glu Tyr Asp Ile Tyr Asn Ile Ile
        290                 295                 300

ggt gat cct gaa tcg ttt aag aaa ttt cgt atg aaa cag cct cct att        961
Gly Asp Pro Glu Ser Phe Lys Lys Phe Arg Met Lys Gln Pro Pro Ile
    305                 310                 315

gct tac tgg tta gaa act aaa aag gga agg aaa ggc tgg tta cag aaa       1009
Ala Tyr Trp Leu Glu Thr Lys Lys Gly Arg Lys Gly Trp Leu Gln Lys
320                 325                 330                 335

tat atg cct gct tta cct cat gga agc aaa tac agg gtg tat ttt aac       1057
Tyr Met Pro Ala Leu Pro His Gly Ser Lys Tyr Arg Val Tyr Phe Asn
                340                 345                 350

aca cca aat ggg cct ctt gaa cga gtt cct gcg tgg gcc aat ttt gtc       1105
Thr Pro Asn Gly Pro Leu Glu Arg Val Pro Ala Trp Ala Asn Phe Val
            355                 360                 365

att cca gat gca ggc ggg atg gca tta gca gtc cat tgg gaa cca cct       1153
Ile Pro Asp Ala Gly Gly Met Ala Leu Ala Val His Trp Glu Pro Pro
        370                 375                 380

cct gaa tat gct tat aaa tgg aaa cac aag cta cca gtc aag cct aag       1201
Pro Glu Tyr Ala Tyr Lys Trp Lys His Lys Leu Pro Val Lys Pro Lys
    385                 390                 395

tcc ttg cgc ata tat gaa tgt cat gtt ggc atc tct ggc cag gaa cca       1249
Ser Leu Arg Ile Tyr Glu Cys His Val Gly Ile Ser Gly Gln Glu Pro
400                 405                 410                 415

aaa gtt tca tct ttc aat gat ttt att agc aag gtc ctt ccg cat gta       1297
Lys Val Ser Ser Phe Asn Asp Phe Ile Ser Lys Val Leu Pro His Val
                420                 425                 430

aaa gaa gct gga tac aat gca ata caa att att gga gtt gtt gag cac       1345
Lys Glu Ala Gly Tyr Asn Ala Ile Gln Ile Ile Gly Val Val Glu His
            435                 440                 445

aag gat tat ttc act gtt gga tat aga gtg acc aat ttt tat gct gtt       1393
Lys Asp Tyr Phe Thr Val Gly Tyr Arg Val Thr Asn Phe Tyr Ala Val
        450                 455                 460

agt agc cgt tat ggc aca ccg gat gac ttc aag cgc ttg gtt gat gaa       1441
Ser Ser Arg Tyr Gly Thr Pro Asp Asp Phe Lys Arg Leu Val Asp Glu
    465                 470                 475

gca cat ggg ctt gga ctg ctt gtc ttt ttg gag att gtg cac tct tat       1489
Ala His Gly Leu Gly Leu Leu Val Phe Leu Glu Ile Val His Ser Tyr
480                 485                 490                 495

gca gca gca gat gaa atg gtt ggg tta tct ctt ttt gat gga gca aat       1537
Ala Ala Ala Asp Glu Met Val Gly Leu Ser Leu Phe Asp Gly Ala Asn
                500                 505                 510

gat tgc tat ttc cac act ggt aaa cgt gga cac cac aaa ttc tgg ggc       1585
Asp Cys Tyr Phe His Thr Gly Lys Arg Gly His His Lys Phe Trp Gly
            515                 520                 525

aca cgg atg ttc aaa tat gga gat ctt gat gtt ctg cac ttt ctt ctt       1633
```

```
Thr Arg Met Phe Lys Tyr Gly Asp Leu Asp Val Leu His Phe Leu Leu
        530                 535                 540

tca aat ctg aac tgg tgg gtg gag gag tat cat gtc gat ggc ttc cat      1681
Ser Asn Leu Asn Trp Trp Val Glu Glu Tyr His Val Asp Gly Phe His
    545                 550                 555

ttt cat tcg ctc tcg tcc atg ttg tat acg cat aat gga ttt gct tca      1729
Phe His Ser Leu Ser Ser Met Leu Tyr Thr His Asn Gly Phe Ala Ser
560                 565                 570                 575

ttt act ggt gac atg gat gaa tac tgt aac caa tat gtt gac aag gag      1777
Phe Thr Gly Asp Met Asp Glu Tyr Cys Asn Gln Tyr Val Asp Lys Glu
                580                 585                 590

gcc tta ttg tac ctc ata tta gca aat gaa gta tta cat gct ctt cat      1825
Ala Leu Leu Tyr Leu Ile Leu Ala Asn Glu Val Leu His Ala Leu His
            595                 600                 605

cct aat gtg atc acg att gct gag gat gca act ctg tat cct gga ctc      1873
Pro Asn Val Ile Thr Ile Ala Glu Asp Ala Thr Leu Tyr Pro Gly Leu
        610                 615                 620

tgc gat cca aca tct caa ggt gga ctg ggc ttt gat tat ttt gcc aat      1921
Cys Asp Pro Thr Ser Gln Gly Gly Leu Gly Phe Asp Tyr Phe Ala Asn
    625                 630                 635

ctt tct gcc tca gag atg tgg ctt gca tta ctt gaa aat act cct gat      1969
Leu Ser Ala Ser Glu Met Trp Leu Ala Leu Leu Glu Asn Thr Pro Asp
640                 645                 650                 655

cat gaa tgg tgc atg agt aag att gtt agc aca tta gtg ggc gat aga      2017
His Glu Trp Cys Met Ser Lys Ile Val Ser Thr Leu Val Gly Asp Arg
                660                 665                 670

caa aat act gat aaa atg ctt ttg tat gca gaa aat cac aac cag tcc      2065
Gln Asn Thr Asp Lys Met Leu Leu Tyr Ala Glu Asn His Asn Gln Ser
            675                 680                 685

att tct gga ggt cgt tcc ttc gca gaa ata ctg att ggt aac tcc ttg      2113
Ile Ser Gly Gly Arg Ser Phe Ala Glu Ile Leu Ile Gly Asn Ser Leu
        690                 695                 700

ggg aaa tcc tcc ata tca caa gag tca tta ctt aga ggc tgc tcg tta      2161
Gly Lys Ser Ser Ile Ser Gln Glu Ser Leu Leu Arg Gly Cys Ser Leu
    705                 710                 715

cac aag atg atc aga tta att aca tct aca att ggt ggt cat gca tac      2209
His Lys Met Ile Arg Leu Ile Thr Ser Thr Ile Gly Gly His Ala Tyr
720                 725                 730                 735

ctc aac ttc atg ggc aat gaa ttt ggt cac cca aag aga gta gag ttt      2257
Leu Asn Phe Met Gly Asn Glu Phe Gly His Pro Lys Arg Val Glu Phe
                740                 745                 750

cca atg tca agc aac aat ttc tcc ttt tca ctg gct aac cgt cgc tgg      2305
Pro Met Ser Ser Asn Asn Phe Ser Phe Ser Leu Ala Asn Arg Arg Trp
            755                 760                 765

gat cta ttg gaa gat gtt gta cat tat caa ttg ttc tca ttt gat aag      2353
Asp Leu Leu Glu Asp Val Val His Tyr Gln Leu Phe Ser Phe Asp Lys
        770                 775                 780

ggt atg atg gac ttg gat aaa aat ggg aga att ttg tcc aga ggt ctt      2401
Gly Met Met Asp Leu Asp Lys Asn Gly Arg Ile Leu Ser Arg Gly Leu
    785                 790                 795

gcc aac att cac cat gtc aat gat act acc atg gtg att tct tac ttg      2449
```

```
Ala Asn Ile His His Val Asn Asp Thr Thr Met Val Ile Ser Tyr Leu
800                 805                 810                 815

aga ggt ccc aat ctc ttt gtg ttc aac ttt cat cct gtc aat tca tat      2497
Arg Gly Pro Asn Leu Phe Val Phe Asn Phe His Pro Val Asn Ser Tyr
                820                 825                 830

gaa aga tac att ata ggt gtg gaa gaa gct gga gag tat caa gtc aca      2545
Glu Arg Tyr Ile Ile Gly Val Glu Glu Ala Gly Glu Tyr Gln Val Thr
            835                 840                 845

tta aat aca gat gaa aac aag tat ggt ggt aga gga cta ctt ggc cat      2593
Leu Asn Thr Asp Glu Asn Lys Tyr Gly Gly Arg Gly Leu Leu Gly His
        850                 855                 860

gat cag aat att caa aga acc att agt aga aga gct gat gga atg aga      2641
Asp Gln Asn Ile Gln Arg Thr Ile Ser Arg Arg Ala Asp Gly Met Arg
    865                 870                 875

ttt tgc ttg gaa gtg cct ctg cca agt aga agt gct cag gtc tac aag      2689
Phe Cys Leu Glu Val Pro Leu Pro Ser Arg Ser Ala Gln Val Tyr Lys
880                 885                 890                 895

ttg acc cga att cta aga gca tgatcactct agtaatcaaa gtgcctcata         2740
Leu Thr Arg Ile Leu Arg Ala
                900

tgatgacaca aaaggaaagg ttctacattg cccttacact gatcaatatt gacacctttc    2800

cgaggtgagt ttctgtgatt cttgagcaga ctgttggcta gtcaattatc atgaactttt    2860

gccttcagca tccggatagt cgcttctcct gtgcaatgag ggcatggacg aatttttttt    2920

tggcttgtca tgggggtcat aagcatccgc cagattaaga tttcacaggc ctcgagtaaa    2980

accatcactt actttaagga tacacaaaca caccaacggg gtgcaggctc tgataccttc    3040

taaagtg                                                              3047
```

<210> 14

<211> 902

<212> PRT

<213> Solanum tuberosum

<400> 14

```
Met Leu Ser Leu Ser Asp Ser Ile Arg Ile Ser Ser Pro Leu Ser Asp
1               5                   10                  15

Ser Arg Leu Ser Phe Leu Ser Gln Thr Gly Ser Arg Thr Ser Arg Gln
            20                  25                  30

Leu Lys Phe Val Arg Ser Arg Arg Ala Arg Val Ser Arg Cys Arg Cys
        35                  40                  45

Ser Ala Thr Glu Gln Pro Pro Pro Gln Arg Arg Lys Gln Arg Pro Glu
    50                  55                  60
```

```
Lys Tyr Lys Gln Ser Glu Glu Gly Lys Gly Ile Asp Pro Val Gly Phe
65                  70                  75                  80

Leu Ser Lys Tyr Gly Ile Thr His Lys Ala Phe Ala Gln Phe Leu Arg
                85                  90                  95

Glu Arg Tyr Lys Ser Leu Lys Asp Leu Lys Asp Glu Ile Leu Thr Arg
            100                 105                 110

His Phe Ser Leu Lys Glu Met Ser Thr Gly Tyr Glu Leu Met Gly Met
        115                 120                 125

His Arg Asn Ile Gln His Arg Val Asp Phe Leu Glu Trp Ala Pro Gly
    130                 135                 140

Ala Arg Tyr Cys Ala Leu Ile Gly Asp Phe Asn Gly Trp Ser Thr Thr
145                 150                 155                 160

Gly Asn Cys Ala Arg Glu Gly His Phe Gly His Asp Asp Tyr Gly Tyr
                165                 170                 175

Trp Phe Ile Ile Leu Glu Asp Lys Leu Arg Glu Gly Glu Glu Pro Asp
            180                 185                 190

Lys Leu Tyr Phe Gln Gln Tyr Asn Tyr Ala Glu Asp Tyr Gly Lys Gly
        195                 200                 205

Asp Thr Gly Ile Thr Val Glu Glu Ile Phe Lys Lys Ala Asn Asp Glu
    210                 215                 220

Tyr Trp Glu Pro Gly Glu Asp Arg Phe Ile Lys Ser Arg Tyr Glu Val
225                 230                 235                 240

Ala Ala Lys Leu Tyr Glu Glu Met Phe Gly Pro Asn Gly Pro Gln Thr
                245                 250                 255

Glu Glu Glu Leu Glu Ala Met Pro Asp Ala Ala Thr Arg Tyr Lys Thr
            260                 265                 270

Trp Lys Glu Gln Gln Lys Glu Asp Pro Ala Ser Asn Leu Pro Ser Tyr
        275                 280                 285

Asp Val Val Asp Ser Gly Lys Glu Tyr Asp Ile Tyr Asn Ile Ile Gly
    290                 295                 300

Asp Pro Glu Ser Phe Lys Lys Phe Arg Met Lys Gln Pro Pro Ile Ala
305                 310                 315                 320

Tyr Trp Leu Glu Thr Lys Lys Gly Arg Lys Gly Trp Leu Gln Lys Tyr
                325                 330                 335
```

Met Pro Ala Leu Pro His Gly Ser Lys Tyr Arg Val Tyr Phe Asn Thr
340 345 350

Pro Asn Gly Pro Leu Glu Arg Val Pro Ala Trp Ala Asn Phe Val Ile
355 360 365

Pro Asp Ala Gly Gly Met Ala Leu Ala Val His Trp Glu Pro Pro Pro
370 375 380

Glu Tyr Ala Tyr Lys Trp Lys His Lys Leu Pro Val Lys Pro Lys Ser
385 390 395 400

Leu Arg Ile Tyr Glu Cys His Val Gly Ile Ser Gly Gln Glu Pro Lys
405 410 415

Val Ser Ser Phe Asn Asp Phe Ile Ser Lys Val Leu Pro His Val Lys
420 425 430

Glu Ala Gly Tyr Asn Ala Ile Gln Ile Ile Gly Val Val Glu His Lys
435 440 445

Asp Tyr Phe Thr Val Gly Tyr Arg Val Thr Asn Phe Tyr Ala Val Ser
450 455 460

Ser Arg Tyr Gly Thr Pro Asp Asp Phe Lys Arg Leu Val Asp Glu Ala
465 470 475 480

His Gly Leu Gly Leu Leu Val Phe Leu Glu Ile Val His Ser Tyr Ala
485 490 495

Ala Ala Asp Glu Met Val Gly Leu Ser Leu Phe Asp Gly Ala Asn Asp
500 505 510

Cys Tyr Phe His Thr Gly Lys Arg Gly His His Lys Phe Trp Gly Thr
515 520 525

Arg Met Phe Lys Tyr Gly Asp Leu Asp Val Leu His Phe Leu Leu Ser
530 535 540

Asn Leu Asn Trp Trp Val Glu Glu Tyr His Val Asp Gly Phe His Phe
545 550 555 560

His Ser Leu Ser Ser Met Leu Tyr Thr His Asn Gly Phe Ala Ser Phe
565 570 575

Thr Gly Asp Met Asp Glu Tyr Cys Asn Gln Tyr Val Asp Lys Glu Ala
580 585 590

Leu Leu Tyr Leu Ile Leu Ala Asn Glu Val Leu His Ala Leu His Pro
595 600 605

Asn Val Ile Thr Ile Ala Glu Asp Ala Thr Leu Tyr Pro Gly Leu Cys
610 615 620

Asp Pro Thr Ser Gln Gly Gly Leu Gly Phe Asp Tyr Phe Ala Asn Leu
625 630 635 640

Ser Ala Ser Glu Met Trp Leu Ala Leu Leu Glu Asn Thr Pro Asp His
645 650 655

Glu Trp Cys Met Ser Lys Ile Val Ser Thr Leu Val Gly Asp Arg Gln
660 665 670

Asn Thr Asp Lys Met Leu Leu Tyr Ala Glu Asn His Asn Gln Ser Ile
675 680 685

Ser Gly Gly Arg Ser Phe Ala Glu Ile Leu Ile Gly Asn Ser Leu Gly
690 695 700

Lys Ser Ser Ile Ser Gln Glu Ser Leu Leu Arg Gly Cys Ser Leu His
705 710 715 720

Lys Met Ile Arg Leu Ile Thr Ser Thr Ile Gly Gly His Ala Tyr Leu
725 730 735

Asn Phe Met Gly Asn Glu Phe Gly His Pro Lys Arg Val Glu Phe Pro
740 745 750

Met Ser Ser Asn Asn Phe Ser Phe Ser Leu Ala Asn Arg Arg Trp Asp
755 760 765

Leu Leu Glu Asp Val Val His Tyr Gln Leu Phe Ser Phe Asp Lys Gly
770 775 780

Met Met Asp Leu Asp Lys Asn Gly Arg Ile Leu Ser Arg Gly Leu Ala
785 790 795 800

Asn Ile His His Val Asn Asp Thr Thr Met Val Ile Ser Tyr Leu Arg
805 810 815

Gly Pro Asn Leu Phe Val Phe Asn Phe His Pro Val Asn Ser Tyr Glu
820 825 830

Arg Tyr Ile Ile Gly Val Glu Glu Ala Gly Glu Tyr Gln Val Thr Leu
835 840 845

Asn Thr Asp Glu Asn Lys Tyr Gly Gly Arg Gly Leu Leu Gly His Asp
850 855 860

Gln Asn Ile Gln Arg Thr Ile Ser Arg Arg Ala Asp Gly Met Arg Phe
865 870 875 880

<213> Artificial sequence

<220>

<223> Oligonucleotide

<400> 18
gtatttctgc gaaggaacga cc 22

<210> 19

<211> 20

<212> DNA

<213> Artificial sequence

<220>

<223> Oligonucleotide

<400> 19
aacaatgctc tctctgtcgg 20

<210> 20

<211> 30

<212> DNA

<213> Artificial sequence

<220>

<223> Oligonucleotide

<400> 20
gcttgtcgac gggagaattt tgtccagagg 30

<210> 21

<211> 31

<212> DNA

<213> Artificial sequence

<220>

<223> Oligonucleotide

<400> 21
gatcgtcgac agcacttcta cttggcagag g 31

Cys Leu Glu Val Pro Leu Pro Ser Arg Ser Ala Gln Val Tyr Lys Leu
885 890 895

Thr Arg Ile Leu Arg Ala
900

<210> 15

<211> 30

<212> DNA

<213> Artificial sequence

<220>

<223> Oligonucleotide

<400> 15
tcaagtcgac cacaaccagt ccatttctgg 30

<210> 16

<211> 30

<212> DNA

<213> Artificial sequence

<220>

<223> Oligonucleotide

<400> 16
tcaaactagt cacaaccagt ccatttctgg 30

<210> 17

<211> 21

<212> DNA

<213> Artificial sequence

<220>

<223> Oligonucleotide

<400> 17
cactttagaa ggtatcagag c 21

<210> 18

<211> 22

<212> DNA

SEQUENCE LISTING

<110> Bayer CropScience GmbH

<120> Phosphorylierte waxy-Kartoffelstärke

<130> BCS 05-5003 EP

<160> 21

<170> PatentIn version 3.1

<210> 1
<211> 4167
<212> DNA
<213> Solanum tuberosum

<220>
<221> CDS
<222> (207)..(3899)
<223>

<300>
<301> Abel,G.J., Springer,F., Willmitzer,L. and Kossmann,J.
<302> Cloning and functional analysis of a cDNA encoding a novel 139 kDa
<303> Plant J.
<304> 10
<305> 6
<306> 981-991
<307> 1996
<308> X94400
<309> 1995-12-22
<313> (1)..(4167)

<300>

<308> EMBL / X94400

<309> 1997-04-16

<313> (1)..(4167)

```
<400>  1
ttttttaata gatttttaaa accccattaa agcaaatacg tatataattg cagcacagat      60

acagagaggg agagagaaag atagtgtgtt gatgaaggag aagagagata tttcacatgg     120

gatgttctat ttgattctgt ggtgaacaag agttttacaa agaacattcc tttttctttt     180

tttcttggtt cttgtgtggg tcagcc atg gat gtt cca ttt cca ctg cat aga      233
                             Met Asp Val Pro Phe Pro Leu His Arg
                             1               5

cca ttg agt tgc aca agt gtc tcc aat gca ata acc cac ctc aag atc       281
Pro Leu Ser Cys Thr Ser Val Ser Asn Ala Ile Thr His Leu Lys Ile
10                  15                  20                  25

aaa cct ttt ctt ggg ttt gtc tct cat gga acc aca agt cta tca gta       329
Lys Pro Phe Leu Gly Phe Val Ser His Gly Thr Thr Ser Leu Ser Val
                30                  35                  40

caa tct tct tca tgg agg aag gat gga atg gtt act ggg gtt tca ttt       377
Gln Ser Ser Ser Trp Arg Lys Asp Gly Met Val Thr Gly Val Ser Phe
            45                  50                  55

cca ttt tgt gca aat ctc tcg gga aga aga cgg aga aaa gtt tca act       425
Pro Phe Cys Ala Asn Leu Ser Gly Arg Arg Arg Arg Lys Val Ser Thr
        60                  65                  70

act agg agt caa gga tct tca cct aag ggg ttt gtg cca agg aag ccc       473
Thr Arg Ser Gln Gly Ser Ser Pro Lys Gly Phe Val Pro Arg Lys Pro
    75                  80                  85

tca ggg atg agc acg caa aga aag gtt cag aag agc aat ggt gat aaa       521
Ser Gly Met Ser Thr Gln Arg Lys Val Gln Lys Ser Asn Gly Asp Lys
90                  95                  100                 105

gaa agt caa agt act tca aca tct aaa gaa tct gaa att tcc aac cag       569
Glu Ser Gln Ser Thr Ser Thr Ser Lys Glu Ser Glu Ile Ser Asn Gln
                110                 115                 120

aag acg gtt gaa gca aga gtt gaa act agt gac gat gac act aaa gta       617
Lys Thr Val Glu Ala Arg Val Glu Thr Ser Asp Asp Asp Thr Lys Val
            125                 130                 135

gtg gtg agg gac cac aag ttt ctg gag gat gag gat gaa atc aat ggt       665
Val Val Arg Asp His Lys Phe Leu Glu Asp Glu Asp Glu Ile Asn Gly
        140                 145                 150

tct act aaa tca ata agt atg tca cct gtt cgt gta tca tct caa ttt       713
Ser Thr Lys Ser Ile Ser Met Ser Pro Val Arg Val Ser Ser Gln Phe
    155                 160                 165

gtt gaa agt gaa gaa act ggt ggt gat gac aag gat gct gta aag tta       761
Val Glu Ser Glu Glu Thr Gly Gly Asp Asp Lys Asp Ala Val Lys Leu
170                 175                 180                 185

aac aaa tca aag aga tcg gaa gag agt gat ttt cta att gat tct gta       809
Asn Lys Ser Lys Arg Ser Glu Glu Ser Asp Phe Leu Ile Asp Ser Val
                190                 195                 200
```

```
ata aga gaa caa agt gga tct cag ggg gaa act aat gcc agt agc aag      857
Ile Arg Glu Gln Ser Gly Ser Gln Gly Glu Thr Asn Ala Ser Ser Lys
            205                 210                 215

gga agc cat gct gtg ggt aca aaa ctt tat gag ata ttg cag gtg gat      905
Gly Ser His Ala Val Gly Thr Lys Leu Tyr Glu Ile Leu Gln Val Asp
        220                 225                 230

gtt gag cca caa caa ttg aaa gaa aat aat gct ggg aat gtt gaa tac      953
Val Glu Pro Gln Gln Leu Lys Glu Asn Asn Ala Gly Asn Val Glu Tyr
    235                 240                 245

aaa gga cct gta gca agt aag cta ttg gaa att act aag gct agt gat     1001
Lys Gly Pro Val Ala Ser Lys Leu Leu Glu Ile Thr Lys Ala Ser Asp
250                 255                 260                 265

gtg gaa cac act gaa agc aat gag att gat gac tta gac act aat agt     1049
Val Glu His Thr Glu Ser Asn Glu Ile Asp Asp Leu Asp Thr Asn Ser
                270                 275                 280

ttc ttt aaa tca gat tta att gaa gag gat gag cca tta gct gca gga     1097
Phe Phe Lys Ser Asp Leu Ile Glu Glu Asp Glu Pro Leu Ala Ala Gly
            285                 290                 295

aca gtg gag act gga gat tct tct cta aac tta aga ttg gag atg gaa     1145
Thr Val Glu Thr Gly Asp Ser Ser Leu Asn Leu Arg Leu Glu Met Glu
        300                 305                 310

gca aat cta cgt agg cag gct ata gaa agg ctt gcc gag gaa aat tta     1193
Ala Asn Leu Arg Arg Gln Ala Ile Glu Arg Leu Ala Glu Glu Asn Leu
    315                 320                 325

ttg caa ggg atc aga tta ttt tgt ttt cca gag gtt gta aaa cct gat     1241
Leu Gln Gly Ile Arg Leu Phe Cys Phe Pro Glu Val Val Lys Pro Asp
330                 335                 340                 345

gaa gat gtc gag ata ttt ctt aac aga ggt ctt tcc act ttg aag aat     1289
Glu Asp Val Glu Ile Phe Leu Asn Arg Gly Leu Ser Thr Leu Lys Asn
                350                 355                 360

gag tct gat gtc ttg att atg gga gct ttt aat gag tgg cgc tat agg     1337
Glu Ser Asp Val Leu Ile Met Gly Ala Phe Asn Glu Trp Arg Tyr Arg
            365                 370                 375

tct ttt act aca agg cta act gag act cat ctc aat gga gat tgg tgg     1385
Ser Phe Thr Thr Arg Leu Thr Glu Thr His Leu Asn Gly Asp Trp Trp
        380                 385                 390

tct tgc aag atc cat gtt ccc aag gaa gca tac agg gct gat ttt gtg     1433
Ser Cys Lys Ile His Val Pro Lys Glu Ala Tyr Arg Ala Asp Phe Val
    395                 400                 405

ttt ttt aat gga caa gat gtc tat gac aac aat gat gga aat gac ttc     1481
Phe Phe Asn Gly Gln Asp Val Tyr Asp Asn Asn Asp Gly Asn Asp Phe
410                 415                 420                 425

agt ata act gtg aaa ggt ggt atg caa atc att gac ttt gaa aat ttc     1529
Ser Ile Thr Val Lys Gly Gly Met Gln Ile Ile Asp Phe Glu Asn Phe
                430                 435                 440

ttg ctt gag gag aaa tgg aga gaa cag gag aaa ctt gct aaa gaa caa     1577
Leu Leu Glu Glu Lys Trp Arg Glu Gln Glu Lys Leu Ala Lys Glu Gln
            445                 450                 455

gct gaa aga gaa aga cta gcg gaa gaa caa aga cga ata gaa gca gag     1625
Ala Glu Arg Glu Arg Leu Ala Glu Glu Gln Arg Arg Ile Glu Ala Glu
        460                 465                 470
```

```
aaa gct gaa att gaa gct gac aga gca caa gca aag gaa gag gct gca      1673
Lys Ala Glu Ile Glu Ala Asp Arg Ala Gln Ala Lys Glu Glu Ala Ala
    475                 480                 485

aag aaa aag aaa gta ttg cga gaa ttg atg gta aaa gcc acg aag act      1721
Lys Lys Lys Lys Val Leu Arg Glu Leu Met Val Lys Ala Thr Lys Thr
490                 495                 500                 505

cgt gat atc acg tgg tac ata gag cca agt gaa ttt aaa tgc gag gac      1769
Arg Asp Ile Thr Trp Tyr Ile Glu Pro Ser Glu Phe Lys Cys Glu Asp
                510                 515                 520

aag gtc agg tta tac tat aac aaa agt tca ggt cct ctc tcc cat gct      1817
Lys Val Arg Leu Tyr Tyr Asn Lys Ser Ser Gly Pro Leu Ser His Ala
            525                 530                 535

aag gac ttg tgg atc cac gga gga tat aat aat tgg aag gat ggt ttg      1865
Lys Asp Leu Trp Ile His Gly Gly Tyr Asn Asn Trp Lys Asp Gly Leu
        540                 545                 550

tct att gtc aaa aag ctt gtt aaa tct gag aga ata gat ggt gat tgg      1913
Ser Ile Val Lys Lys Leu Val Lys Ser Glu Arg Ile Asp Gly Asp Trp
    555                 560                 565

tgg tat aca gag gtt gtt att cct gat cag gca ctt ttc ttg gat tgg      1961
Trp Tyr Thr Glu Val Val Ile Pro Asp Gln Ala Leu Phe Leu Asp Trp
570                 575                 580                 585

gtt ttt gct gat ggt cca ccc aag cat gcc att gct tat gat aac aat      2009
Val Phe Ala Asp Gly Pro Pro Lys His Ala Ile Ala Tyr Asp Asn Asn
                590                 595                 600

cac cgc caa gac ttc cat gcc att gtc ccc aac cac att ccg gag gaa      2057
His Arg Gln Asp Phe His Ala Ile Val Pro Asn His Ile Pro Glu Glu
            605                 610                 615

tta tat tgg gtt gag gaa gaa cat cag atc ttt aag aca ctt cag gag      2105
Leu Tyr Trp Val Glu Glu Glu His Gln Ile Phe Lys Thr Leu Gln Glu
        620                 625                 630

gag aga agg ctt aga gaa gcg gct atg cgt gct aag gtt gaa aaa aca      2153
Glu Arg Arg Leu Arg Glu Ala Ala Met Arg Ala Lys Val Glu Lys Thr
    635                 640                 645

gca ctt ctg aaa act gaa aca aag gaa aga act atg aaa tca ttt tta      2201
Ala Leu Leu Lys Thr Glu Thr Lys Glu Arg Thr Met Lys Ser Phe Leu
650                 655                 660                 665

ctg tct cag aag cat gta gta tat act gag cct ctt gat atc caa gct      2249
Leu Ser Gln Lys His Val Val Tyr Thr Glu Pro Leu Asp Ile Gln Ala
                670                 675                 680

gga agc agc gtc aca gtt tac tat aat ccc gcc aat aca gta ctt aat      2297
Gly Ser Ser Val Thr Val Tyr Tyr Asn Pro Ala Asn Thr Val Leu Asn
            685                 690                 695

ggt aaa cct gaa att tgg ttc aga tgt tca ttt aat cgc tgg act cac      2345
Gly Lys Pro Glu Ile Trp Phe Arg Cys Ser Phe Asn Arg Trp Thr His
        700                 705                 710

cgc ctg ggt cca ttg cca cct cag aaa atg tcg cct gct gaa aat ggc      2393
Arg Leu Gly Pro Leu Pro Pro Gln Lys Met Ser Pro Ala Glu Asn Gly
    715                 720                 725

acc cat gtc aga gca act gtg aag gtt cca ttg gat gca tat atg atg      2441
Thr His Val Arg Ala Thr Val Lys Val Pro Leu Asp Ala Tyr Met Met
730                 735                 740                 745
```

```
gat ttt gta ttt tcc gag aga gaa gat ggt ggg att ttt gac aat aag      2489
Asp Phe Val Phe Ser Glu Arg Glu Asp Gly Gly Ile Phe Asp Asn Lys
                750                 755                 760

agc gga atg gac tat cac ata cct gtg ttt gga gga gtc gct aaa gaa      2537
Ser Gly Met Asp Tyr His Ile Pro Val Phe Gly Gly Val Ala Lys Glu
            765                 770                 775

cct cca atg cat att gtc cat att gct gtc gaa atg gca cca att gca      2585
Pro Pro Met His Ile Val His Ile Ala Val Glu Met Ala Pro Ile Ala
        780                 785                 790

aag gtg gga ggc ctt ggt gat gtt gtt act agt ctt tcc cgt gct gtt      2633
Lys Val Gly Gly Leu Gly Asp Val Val Thr Ser Leu Ser Arg Ala Val
    795                 800                 805

caa gat tta aac cat aat gtg gat att atc tta cct aag tat gac tgt      2681
Gln Asp Leu Asn His Asn Val Asp Ile Ile Leu Pro Lys Tyr Asp Cys
810                 815                 820                 825

ttg aag atg aat aat gtg aag gac ttt cgg ttt cac aaa aac tac ttt      2729
Leu Lys Met Asn Asn Val Lys Asp Phe Arg Phe His Lys Asn Tyr Phe
                830                 835                 840

tgg ggt ggg act gaa ata aaa gta tgg ttt gga aag gtg gaa ggt ctc      2777
Trp Gly Gly Thr Glu Ile Lys Val Trp Phe Gly Lys Val Glu Gly Leu
            845                 850                 855

tcg gtc tat ttt ttg gag cct caa aac ggg tta ttt tcg aaa ggg tgc      2825
Ser Val Tyr Phe Leu Glu Pro Gln Asn Gly Leu Phe Ser Lys Gly Cys
        860                 865                 870

gtc tat ggt tgt agc aat gat ggt gaa cga ttt ggt ttc ttc tgt cac      2873
Val Tyr Gly Cys Ser Asn Asp Gly Glu Arg Phe Gly Phe Phe Cys His
    875                 880                 885

gcg gct ttg gag ttt ctt ctg caa ggt gga ttt agt ccg gat atc att      2921
Ala Ala Leu Glu Phe Leu Leu Gln Gly Gly Phe Ser Pro Asp Ile Ile
890                 895                 900                 905

cat tgc cat gat tgg tct agt gct cct gtt gct tgg ctc ttt aag gaa      2969
His Cys His Asp Trp Ser Ser Ala Pro Val Ala Trp Leu Phe Lys Glu
                910                 915                 920

caa tat aca cac tat ggt cta agc aaa tct cgt ata gtc ttc acg ata      3017
Gln Tyr Thr His Tyr Gly Leu Ser Lys Ser Arg Ile Val Phe Thr Ile
            925                 930                 935

cat aat ctt gaa ttt ggg gca gat ctc att ggg aga gca atg act aac      3065
His Asn Leu Glu Phe Gly Ala Asp Leu Ile Gly Arg Ala Met Thr Asn
        940                 945                 950

gca gac aaa gct aca aca gtt tca cca act tac tca cag gag gtg tct      3113
Ala Asp Lys Ala Thr Thr Val Ser Pro Thr Tyr Ser Gln Glu Val Ser
    955                 960                 965

gga aac cct gta att gcg cct cac ctt cac aag ttc cat ggt ata gtg      3161
Gly Asn Pro Val Ile Ala Pro His Leu His Lys Phe His Gly Ile Val
970                 975                 980                 985

aat ggg att gac cca gat att tgg gat cct tta aac gat aag ttc  att     3209
Asn Gly Ile Asp Pro Asp Ile Trp Asp Pro Leu Asn Asp Lys Phe  Ile
                990                 995                 1000

ccg att ccg tac  acc tca gaa aac gtt  gtt gaa ggc aaa aca  gca       3254
Pro Ile Pro Tyr  Thr Ser Glu Asn Val  Val Glu Gly Lys Thr  Ala
            1005                 1010                 1015
```

```
gcc aag gaa gct  ttg cag cga aaa ctt  gga ctg aaa cag gct  gac      3299
Ala Lys Glu Ala  Leu Gln Arg Lys Leu  Gly Leu Lys Gln Ala  Asp
            1020                 1025                 1030

ctt cct ttg gta  gga att atc acc cgc  tta act cac cag aaa  gga      3344
Leu Pro Leu Val  Gly Ile Ile Thr Arg  Leu Thr His Gln Lys  Gly
            1035                 1040                 1045

atc cac ctc att  aaa cat gct att tgg  cgc acc ttg gaa cgg  aac      3389
Ile His Leu Ile  Lys His Ala Ile Trp  Arg Thr Leu Glu Arg  Asn
            1050                 1055                 1060

gga cag gta gtc  ttg ctt ggt tct gct  cct gat cct agg gta  caa      3434
Gly Gln Val Val  Leu Leu Gly Ser Ala  Pro Asp Pro Arg Val  Gln
            1065                 1070                 1075

aac gat ttt gtt  aat ttg gca aat caa  ttg cac tcc aaa tat  aat      3479
Asn Asp Phe Val  Asn Leu Ala Asn Gln  Leu His Ser Lys Tyr  Asn
            1080                 1085                 1090

gac cgc gca cga  ctc tgt cta aca tat  gac gag cca ctt tct  cac      3524
Asp Arg Ala Arg  Leu Cys Leu Thr Tyr  Asp Glu Pro Leu Ser  His
            1095                 1100                 1105

ctg ata tat gct  ggt gct gat ttt att  cta gtt cct tca ata  ttt      3569
Leu Ile Tyr Ala  Gly Ala Asp Phe Ile  Leu Val Pro Ser Ile  Phe
            1110                 1115                 1120

gag cca tgt gga  cta aca caa ctt acc  gct atg aga tat ggt  tca      3614
Glu Pro Cys Gly  Leu Thr Gln Leu Thr  Ala Met Arg Tyr Gly  Ser
            1125                 1130                 1135

att cca gtc gtg  cgt aaa act gga gga  ctt tat gat act gta  ttt      3659
Ile Pro Val Val  Arg Lys Thr Gly Gly  Leu Tyr Asp Thr Val  Phe
            1140                 1145                 1150

gat gtt gac cat  gac aaa gag aga gca  caa cag tgt ggt ctt  gaa      3704
Asp Val Asp His  Asp Lys Glu Arg Ala  Gln Gln Cys Gly Leu  Glu
            1155                 1160                 1165

cca aat gga ttc  agc ttt gat gga gca  gat gct ggc gga gtt  gat      3749
Pro Asn Gly Phe  Ser Phe Asp Gly Ala  Asp Ala Gly Gly Val  Asp
            1170                 1175                 1180

tat gct ctg aat  aga gct ctc tct gct  tgg tac gat ggt cgg  gat      3794
Tyr Ala Leu Asn  Arg Ala Leu Ser Ala  Trp Tyr Asp Gly Arg  Asp
            1185                 1190                 1195

tgg ttc aac tct  tta tgc aag cag gtc  atg gaa caa gat tgg  tct      3839
Trp Phe Asn Ser  Leu Cys Lys Gln Val  Met Glu Gln Asp Trp  Ser
            1200                 1205                 1210

tgg aac cga cct  gct ctt gat tat ttg  gag ctt tac cat gct  gct      3884
Trp Asn Arg Pro  Ala Leu Asp Tyr Leu  Glu Leu Tyr His Ala  Ala
            1215                 1220                 1225

aga aag tta gaa  tag ttagtttgtg agatgctagc agaaaaattc acgagatctg   3939
Arg Lys Leu Glu
            1230

caatctgtac aggttcagtg tttgcgtctg gacagctttt ttatttccta tatcaaagta   3999

taaatcaagt ctacactgag atcaatagca gacagtcctc agttcatttc atttttgtg    4059

caacatatga aagagcttag cctctaataa tgtagtcatt gatgattatt tgttttggga   4119

agaaatgaga aatcaaagga tgcaaaatac tctgaaaaa aaaaaaa                  4167
```

<210> 2

<211> 1230

<212> PRT

<213> Solanum tuberosum

<400> 2

Met Asp Val Pro Phe Pro Leu His Arg Pro Leu Ser Cys Thr Ser Val
1               5                   10                  15

Ser Asn Ala Ile Thr His Leu Lys Ile Lys Pro Phe Leu Gly Phe Val
            20                  25                  30

Ser His Gly Thr Thr Ser Leu Ser Val Gln Ser Ser Ser Trp Arg Lys
        35                  40                  45

Asp Gly Met Val Thr Gly Val Ser Phe Pro Phe Cys Ala Asn Leu Ser
    50                  55                  60

Gly Arg Arg Arg Arg Lys Val Ser Thr Thr Arg Ser Gln Gly Ser Ser
65                  70                  75                  80

Pro Lys Gly Phe Val Pro Arg Lys Pro Ser Gly Met Ser Thr Gln Arg
                85                  90                  95

Lys Val Gln Lys Ser Asn Gly Asp Lys Glu Ser Gln Ser Thr Ser Thr
            100                 105                 110

Ser Lys Glu Ser Glu Ile Ser Asn Gln Lys Thr Val Glu Ala Arg Val
        115                 120                 125

Glu Thr Ser Asp Asp Asp Thr Lys Val Val Val Arg Asp His Lys Phe
    130                 135                 140

Leu Glu Asp Glu Asp Glu Ile Asn Gly Ser Thr Lys Ser Ile Ser Met
145                 150                 155                 160

Ser Pro Val Arg Val Ser Ser Gln Phe Val Glu Ser Glu Glu Thr Gly
                165                 170                 175

Gly Asp Asp Lys Asp Ala Val Lys Leu Asn Lys Ser Lys Arg Ser Glu
            180                 185                 190

Glu Ser Asp Phe Leu Ile Asp Ser Val Ile Arg Glu Gln Ser Gly Ser
        195                 200                 205

Gln Gly Glu Thr Asn Ala Ser Ser Lys Gly Ser His Ala Val Gly Thr
    210                 215                 220

Lys Leu Tyr Glu Ile Leu Gln Val Asp Val Glu Pro Gln Gln Leu Lys
225 230 235 240

Glu Asn Asn Ala Gly Asn Val Glu Tyr Lys Gly Pro Val Ala Ser Lys
245 250 255

Leu Leu Glu Ile Thr Lys Ala Ser Asp Val Glu His Thr Glu Ser Asn
260 265 270

Glu Ile Asp Asp Leu Asp Thr Asn Ser Phe Phe Lys Ser Asp Leu Ile
275 280 285

Glu Glu Asp Glu Pro Leu Ala Ala Gly Thr Val Glu Thr Gly Asp Ser
290 295 300

Ser Leu Asn Leu Arg Leu Glu Met Glu Ala Asn Leu Arg Arg Gln Ala
305 310 315 320

Ile Glu Arg Leu Ala Glu Glu Asn Leu Leu Gln Gly Ile Arg Leu Phe
325 330 335

Cys Phe Pro Glu Val Val Lys Pro Asp Glu Asp Val Glu Ile Phe Leu
340 345 350

Asn Arg Gly Leu Ser Thr Leu Lys Asn Glu Ser Asp Val Leu Ile Met
355 360 365

Gly Ala Phe Asn Glu Trp Arg Tyr Arg Ser Phe Thr Thr Arg Leu Thr
370 375 380

Glu Thr His Leu Asn Gly Asp Trp Trp Ser Cys Lys Ile His Val Pro
385 390 395 400

Lys Glu Ala Tyr Arg Ala Asp Phe Val Phe Phe Asn Gly Gln Asp Val
405 410 415

Tyr Asp Asn Asn Asp Gly Asn Asp Phe Ser Ile Thr Val Lys Gly Gly
420 425 430

Met Gln Ile Ile Asp Phe Glu Asn Phe Leu Leu Glu Glu Lys Trp Arg
435 440 445

Glu Gln Glu Lys Leu Ala Lys Glu Gln Ala Glu Arg Glu Arg Leu Ala
450 455 460

Glu Glu Gln Arg Arg Ile Glu Ala Glu Lys Ala Glu Ile Glu Ala Asp
465 470 475 480

Arg Ala Gln Ala Lys Glu Glu Ala Ala Lys Lys Lys Lys Val Leu Arg
485 490 495

Glu Leu Met Val Lys Ala Thr Lys Thr Arg Asp Ile Thr Trp Tyr Ile
500 505 510

Glu Pro Ser Glu Phe Lys Cys Glu Asp Lys Val Arg Leu Tyr Tyr Asn
515 520 525

Lys Ser Ser Gly Pro Leu Ser His Ala Lys Asp Leu Trp Ile His Gly
530 535 540

Gly Tyr Asn Asn Trp Lys Asp Gly Leu Ser Ile Val Lys Lys Leu Val
545 550 555 560

Lys Ser Glu Arg Ile Asp Gly Asp Trp Trp Tyr Thr Glu Val Val Ile
565 570 575

Pro Asp Gln Ala Leu Phe Leu Asp Trp Val Phe Ala Asp Gly Pro Pro
580 585 590

Lys His Ala Ile Ala Tyr Asp Asn Asn His Arg Gln Asp Phe His Ala
595 600 605

Ile Val Pro Asn His Ile Pro Glu Glu Leu Tyr Trp Val Glu Glu Glu
610 615 620

His Gln Ile Phe Lys Thr Leu Gln Glu Glu Arg Arg Leu Arg Glu Ala
625 630 635 640

Ala Met Arg Ala Lys Val Glu Lys Thr Ala Leu Leu Lys Thr Glu Thr
645 650 655

Lys Glu Arg Thr Met Lys Ser Phe Leu Leu Ser Gln Lys His Val Val
660 665 670

Tyr Thr Glu Pro Leu Asp Ile Gln Ala Gly Ser Ser Val Thr Val Tyr
675 680 685

Tyr Asn Pro Ala Asn Thr Val Leu Asn Gly Lys Pro Glu Ile Trp Phe
690 695 700

Arg Cys Ser Phe Asn Arg Trp Thr His Arg Leu Gly Pro Leu Pro Pro
705 710 715 720

Gln Lys Met Ser Pro Ala Glu Asn Gly Thr His Val Arg Ala Thr Val
725 730 735

Lys Val Pro Leu Asp Ala Tyr Met Met Asp Phe Val Phe Ser Glu Arg
740 745 750

Glu Asp Gly Gly Ile Phe Asp Asn Lys Ser Gly Met Asp Tyr His Ile
755 760 765

```
Pro Val Phe Gly Gly Val Ala Lys Glu Pro Pro Met His Ile Val His
    770                 775                 780

Ile Ala Val Glu Met Ala Pro Ile Ala Lys Val Gly Gly Leu Gly Asp
785                 790                 795                 800

Val Val Thr Ser Leu Ser Arg Ala Val Gln Asp Leu Asn His Asn Val
                805                 810                 815

Asp Ile Ile Leu Pro Lys Tyr Asp Cys Leu Lys Met Asn Asn Val Lys
            820                 825                 830

Asp Phe Arg Phe His Lys Asn Tyr Phe Trp Gly Gly Thr Glu Ile Lys
        835                 840                 845

Val Trp Phe Gly Lys Val Glu Gly Leu Ser Val Tyr Phe Leu Glu Pro
    850                 855                 860

Gln Asn Gly Leu Phe Ser Lys Gly Cys Val Tyr Gly Cys Ser Asn Asp
865                 870                 875                 880

Gly Glu Arg Phe Gly Phe Phe Cys His Ala Ala Leu Glu Phe Leu Leu
                885                 890                 895

Gln Gly Gly Phe Ser Pro Asp Ile Ile His Cys His Asp Trp Ser Ser
            900                 905                 910

Ala Pro Val Ala Trp Leu Phe Lys Glu Gln Tyr Thr His Tyr Gly Leu
        915                 920                 925

Ser Lys Ser Arg Ile Val Phe Thr Ile His Asn Leu Glu Phe Gly Ala
    930                 935                 940

Asp Leu Ile Gly Arg Ala Met Thr Asn Ala Asp Lys Ala Thr Thr Val
945                 950                 955                 960

Ser Pro Thr Tyr Ser Gln Glu Val Ser Gly Asn Pro Val Ile Ala Pro
                965                 970                 975

His Leu His Lys Phe His Gly Ile Val Asn Gly Ile Asp Pro Asp Ile
            980                 985                 990

Trp Asp Pro Leu Asn Asp Lys Phe  Ile Pro Ile Pro Tyr  Thr Ser Glu
        995                 1000                1005

Asn Val  Val Glu Gly Lys Thr  Ala Ala Lys Glu Ala  Leu Gln Arg
    1010                 1015                 1020

Lys Leu  Gly Leu Lys Gln Ala  Asp Leu Pro Leu Val  Gly Ile Ile
    1025                 1030                 1035
```

Thr Arg Leu Thr His Gln Lys Gly Ile His Leu Ile Lys His Ala
1040 1045 1050

Ile Trp Arg Thr Leu Glu Arg Asn Gly Gln Val Val Leu Leu Gly
1055 1060 1065

Ser Ala Pro Asp Pro Arg Val Gln Asn Asp Phe Val Asn Leu Ala
1070 1075 1080

Asn Gln Leu His Ser Lys Tyr Asn Asp Arg Ala Arg Leu Cys Leu
1085 1090 1095

Thr Tyr Asp Glu Pro Leu Ser His Leu Ile Tyr Ala Gly Ala Asp
1100 1105 1110

Phe Ile Leu Val Pro Ser Ile Phe Glu Pro Cys Gly Leu Thr Gln
1115 1120 1125

Leu Thr Ala Met Arg Tyr Gly Ser Ile Pro Val Val Arg Lys Thr
1130 1135 1140

Gly Gly Leu Tyr Asp Thr Val Phe Asp Val Asp His Asp Lys Glu
1145 1150 1155

Arg Ala Gln Gln Cys Gly Leu Glu Pro Asn Gly Phe Ser Phe Asp
1160 1165 1170

Gly Ala Asp Ala Gly Gly Val Asp Tyr Ala Leu Asn Arg Ala Leu
1175 1180 1185

Ser Ala Trp Tyr Asp Gly Arg Asp Trp Phe Asn Ser Leu Cys Lys
1190 1195 1200

Gln Val Met Glu Gln Asp Trp Ser Trp Asn Arg Pro Ala Leu Asp
1205 1210 1215

Tyr Leu Glu Leu Tyr His Ala Ala Arg Lys Leu Glu
1220 1225 1230

<210> 3

<211> 61

<212> PRT

<213> Solanum tuberosum

<400> 3

Arg Ser Phe Thr Thr Arg Leu Thr Glu Thr His Leu Asn Gly Asp Trp
1 5 10 15

```
Trp Ser Cys Lys Ile His Val Pro Lys Glu Ala Tyr Arg Ala Asp Phe
            20                  25                  30

Val Phe Phe Asn Gly Gln Asp Val Tyr Asp Asn Asn Asp Gly Asn Asp
        35                  40                  45

Phe Ser Ile Thr Val Lys Gly Gly Met Gln Ile Ile Asp
    50                  55                  60
```

<210> 4

<211> 1641

<212> DNA

<213> Solanum tuberosum

<400> 4

```
atgaagcaca gttcagctat ttccgctgtt ttgaccgatg acaattcgac aatggcaccc      60
ctagaggaag atgtcaacac tgaaaatatt ggcctcctaa atttggatcc aactttggaa     120
ccttatctag atcacttcag acacagaatg aagagatatg tggatcagaa aatgctcatt     180
gaaaaatatg agggacccct tgaggaattt gctcaaggtt atttaaaatt tggattcaac     240
agggaagatg gttgcatagt ctatcgtgaa tgggctcctg ctgctcagga agcagaagtt     300
attggcgatt tcaatggtag gaacggttct aaccacatga tggagaagga ccagtttggt     360
gtttggagta ttagaattcc tgatgttgac agtaagccag tcattccaca caactccaga     420
gttaagtttc gtttcaaaca tggtaatgga gtgtgggtag atcgtatccc tgcttggata     480
aagtatgcca ctgcagacgc cacaaagttt gcagcaccat atgatggtgt ctactgggac     540
ccaccacctt cagaaaggta ccacttcaaa taccctcgcc ctcccaaacc ccgagcccca     600
cgaatctacg aagcacatgt cggcatgagc agctctgagc cacgtgtaaa ttcgtatcgt     660
gagtttgcag atgatgtttt acctcggatt aaggcaaata actataatac tgtccagttg     720
atggccataa tggaacattc ttactatgga tcatttggat atcatgttac aaactttttt     780
gctgtgagca atagatatgg aaacccggag gacctaaagt atctgataga taaagcacat     840
agcttgggtt tacaggttct ggtggatgta gttcacagtc atgcaagcaa taatgtcact     900
gatggcctca atggctttga tattggccaa ggttctcaag aatcctactt tcatgctgga     960
gagcgagggt accataagtt gtgggatagc aggctgttca actatgccaa ttgggaggtt    1020
cttcgtttcc ttctttccaa cttgaggtgg tggctagaag agtataactt tgacggattt    1080
cgatttgatg gaataacttc tatgctgtat gttcatcatg gaatcaatat gggatttaca    1140
ggaaactata atgagtattt cagcgaggct acagatgttg atgctgtggt ctatttaatg    1200
ttggccaata atctgattca caagattttc ccagacgcaa ctgttattgc cgaagatgtt    1260
tctggtatgc cgggccttag ccggcctgtt tctgagggag gaattggttt tgattaccgc    1320
```

ctggcaatgg caatcccaga taagtggata gattatttaa agaataagaa tgatgaagat 1380

tggtccatga aggaagtaac atcgagtttg acaaatagga gatatacaga gaagtgtata 1440

gcatatgcgg agagccatga tcagtctatt gtcggtgaca agaccattgc atttctccta 1500

atgaacaaag agatgtattc tggcatgtct tgcttgacag atgcttctcc tgttgttgat 1560

gcaggaattg cgcttgacaa gatgatccat ttttttcaca atggccttgg gaggagaggg 1620

gtacctcaat ttcatgggta a 1641

<210> 5

<211> 546

<212> PRT

<213> Solanum tuberosum

<300>

<308> Swiss Prot / P30924

<309> 1993-07-26

<400> 5

```
Met Lys His Ser Ser Ala Ile Ser Ala Val Leu Thr Asp Asp Asn Ser
1               5                   10                  15

Thr Met Ala Pro Leu Glu Glu Asp Val Asn Thr Glu Asn Ile Gly Leu
            20                  25                  30

Leu Asn Leu Asp Pro Thr Leu Glu Pro Tyr Leu Asp His Phe Arg His
        35                  40                  45

Arg Met Lys Arg Tyr Val Asp Gln Lys Met Leu Ile Glu Lys Tyr Glu
    50                  55                  60

Gly Pro Leu Glu Glu Phe Ala Gln Gly Tyr Leu Lys Phe Gly Phe Asn
65                  70                  75                  80

Arg Glu Asp Gly Cys Ile Val Tyr Arg Glu Trp Ala Pro Ala Ala Gln
                85                  90                  95

Glu Ala Glu Val Ile Gly Asp Phe Asn Gly Arg Asn Gly Ser Asn His
            100                 105                 110

Met Met Glu Lys Asp Gln Phe Gly Val Trp Ser Ile Arg Ile Pro Asp
        115                 120                 125

Val Asp Ser Lys Pro Val Ile Pro His Asn Ser Arg Val Lys Phe Arg
    130                 135                 140
```

```
Phe Lys His Gly Asn Gly Val Trp Val Asp Arg Ile Pro Ala Trp Ile
145                 150                 155                 160

Lys Tyr Ala Thr Ala Asp Ala Thr Lys Phe Ala Ala Pro Tyr Asp Gly
                165                 170                 175

Val Tyr Trp Asp Pro Pro Pro Ser Glu Arg Tyr His Phe Lys Tyr Pro
            180                 185                 190

Arg Pro Pro Lys Pro Arg Ala Pro Arg Ile Tyr Glu Ala His Val Gly
        195                 200                 205

Met Ser Ser Ser Glu Pro Arg Val Asn Ser Tyr Arg Glu Phe Ala Asp
    210                 215                 220

Asp Val Leu Pro Arg Ile Lys Ala Asn Asn Tyr Asn Thr Val Gln Leu
225                 230                 235                 240

Met Ala Ile Met Glu His Ser Tyr Tyr Gly Ser Phe Gly Tyr His Val
                245                 250                 255

Thr Asn Phe Phe Ala Val Ser Asn Arg Tyr Gly Asn Pro Glu Asp Leu
            260                 265                 270

Lys Tyr Leu Ile Asp Lys Ala His Ser Leu Gly Leu Gln Val Leu Val
        275                 280                 285

Asp Val Val His Ser His Ala Ser Asn Asn Val Thr Asp Gly Leu Asn
    290                 295                 300

Gly Phe Asp Ile Gly Gln Gly Ser Gln Glu Ser Tyr Phe His Ala Gly
305                 310                 315                 320

Glu Arg Gly Tyr His Lys Leu Trp Asp Ser Arg Leu Phe Asn Tyr Ala
                325                 330                 335

Asn Trp Glu Val Leu Arg Phe Leu Leu Ser Asn Leu Arg Trp Trp Leu
            340                 345                 350

Glu Glu Tyr Asn Phe Asp Gly Phe Arg Phe Asp Gly Ile Thr Ser Met
        355                 360                 365

Leu Tyr Val His His Gly Ile Asn Met Gly Phe Thr Gly Asn Tyr Asn
    370                 375                 380

Glu Tyr Phe Ser Glu Ala Thr Asp Val Asp Ala Val Val Tyr Leu Met
385                 390                 395                 400

Leu Ala Asn Asn Leu Ile His Lys Ile Phe Pro Asp Ala Thr Val Ile
                405                 410                 415
```

Ala Glu Asp Val Ser Gly Met Pro Gly Leu Ser Arg Pro Val Ser Glu
420 425 430

Gly Gly Ile Gly Phe Asp Tyr Arg Leu Ala Met Ala Ile Pro Asp Lys
435 440 445

Trp Ile Asp Tyr Leu Lys Asn Lys Asn Asp Glu Asp Trp Ser Met Lys
450 455 460

Glu Val Thr Ser Ser Leu Thr Asn Arg Arg Tyr Thr Glu Lys Cys Ile
465 470 475 480

Ala Tyr Ala Glu Ser His Asp Gln Ser Ile Val Gly Asp Lys Thr Ile
485 490 495

Ala Phe Leu Leu Met Asn Lys Glu Met Tyr Ser Gly Met Ser Cys Leu
500 505 510

Thr Asp Ala Ser Pro Val Val Asp Ala Gly Ile Ala Leu Asp Lys Met
515 520 525

Ile His Phe Phe His Asn Gly Leu Gly Arg Arg Gly Val Pro Gln Phe
530 535 540

His Gly
545

<210> 6

<211> 1824

<212> DNA

<213> Solanum tuberosum

<400> 6
atggcaagca tcacagcttc acaccacttt gtgtcaagaa gccaaacttc actagacacc 60
aaatcaacct tgtcacagat aggactcagg aaccatactc tgactcacaa tggtttaagg 120
gctgttaaca agcttgatgg gctccaatca agaactaata ctaaggtaac acccaagatg 180
gcatccagaa ctgagaccaa gagacctgga tgctcagcta ccattgtttg tggaaaggga 240
atgaacttga tctttgtggg tactgaggtt ggtccttgga gcaaaactgg tggactaggt 300
gatgttcttg gtggactacc accagccctt gcagcccgcg gacatcgggt aatgacaata 360
tccccccgtt atgaccaata caaagatgct tgggatacta gcgttgcggt tgaggtcaaa 420
gttggagaca gcattgaaat tgttcgtttc tttcactgct ataaacgtgg ggttgatcgt 480
gtttttgttg accacccaat gttcttggag aaagtttggg gcaaaactgg ttcaaaaatc 540
tatggcccca aagctggact agattatctg gacaatgaac ttaggttcag cttgttgtgt 600

caagcagccc tagaggcacc taaagttttg aatttgaaca gtagcaacta cttctcagga 660

ccatatggag aggatgttct cttcattgcc aatgattggc acacagctct cattccttgc 720

tacttgaagt caatgtacca gtccagagga atctatttga atgccaaggt cgctttctgc 780

atccataaca ttgcctacca aggccgattt tctttctctg acttccctct tctcaatctt 840

cctgatgaat tcaggggttc ttttgatttc attgatggat atgagaagcc tgttaagggt 900

aggaaaatca actggatgaa ggctgggata ttagaatcac atagggtggt tacagtgagc 960

ccatactatg cccaagaact tgtctctgct gttgacaagg gtgttgaatt ggacagtgtc 1020

cttcgtaaga cttgcataac tgggattgtg aatggcatgg atacacaaga gtggaaccca 1080

gcgactgaca aatacacaga tgtcaaatac gatataacca ctgtcatgga cgcaaaacct 1140

ttactaaagg aggctcttca agcagcagtt ggcttgcctg ttgacaagaa gatccctttg 1200

attggcttca tcggcagact tgaggagcag aaaggttcag atattcttgt tgctgcaatt 1260

cacaagttca tcggattgga tgttcaaatt gtagtccttg gaactggcaa aaaggagttt 1320

gagcaggaga ttgaacagct cgaagtgttg taccctaaca aagctaaagg agtggcaaaa 1380

ttcaatgtcc ctttggctca catgatcact gctggtgctg attttatgtt ggttccaagc 1440

agatttgaac cttgtggtct cattcagtta catgctatgc gatatggaac agtgccaatc 1500

tgtgcatcga ctggtggact tgttgacact gtgaaagaag gctatactgg attccatatg 1560

ggagccttca atgttgaatg cgatgttgtt gacccagctg atgtgcttaa gatagtaaca 1620

acagttgcta gagctcttgc agtctatggc accctcgcat ttgctgagat gataaaaaat 1680

tgcatgtcag aggaactctc ctggaaggaa cctgccaaga aatgggagac attgctattg 1740

ggcttaggag cttctggcag tgaacccggt gttgaagggg aagaaatcgc tccacttgcc 1800

aaggaaaatg tagccactcc ctaa 1824

<210> 7

<211> 607

<212> PRT

<213> Solanum tuberosum

<400> 7

Met Ala Ser Ile Thr Ala Ser His His Phe Val Ser Arg Ser Gln Thr
1 5 10 15

Ser Leu Asp Thr Lys Ser Thr Leu Ser Gln Ile Gly Leu Arg Asn His
20 25 30

Thr Leu Thr His Asn Gly Leu Arg Ala Val Asn Lys Leu Asp Gly Leu
35 40 45

Gln Ser Arg Thr Asn Thr Lys Val Thr Pro Lys Met Ala Ser Arg Thr
50 55 60

Glu Thr Lys Arg Pro Gly Cys Ser Ala Thr Ile Val Cys Gly Lys Gly
65 70 75 80

Met Asn Leu Ile Phe Val Gly Thr Glu Val Gly Pro Trp Ser Lys Thr
85 90 95

Gly Gly Leu Gly Asp Val Leu Gly Gly Leu Pro Pro Ala Leu Ala Ala
100 105 110

Arg Gly His Arg Val Met Thr Ile Ser Pro Arg Tyr Asp Gln Tyr Lys
115 120 125

Asp Ala Trp Asp Thr Ser Val Ala Val Glu Val Lys Val Gly Asp Ser
130 135 140

Ile Glu Ile Val Arg Phe Phe His Cys Tyr Lys Arg Gly Val Asp Arg
145 150 155 160

Val Phe Val Asp His Pro Met Phe Leu Glu Lys Val Trp Gly Lys Thr
165 170 175

Gly Ser Lys Ile Tyr Gly Pro Lys Ala Gly Leu Asp Tyr Leu Asp Asn
180 185 190

Glu Leu Arg Phe Ser Leu Leu Cys Gln Ala Ala Leu Glu Ala Pro Lys
195 200 205

Val Leu Asn Leu Asn Ser Ser Asn Tyr Phe Ser Gly Pro Tyr Gly Glu
210 215 220

Asp Val Leu Phe Ile Ala Asn Asp Trp His Thr Ala Leu Ile Pro Cys
225 230 235 240

Tyr Leu Lys Ser Met Tyr Gln Ser Arg Gly Ile Tyr Leu Asn Ala Lys
245 250 255

Val Ala Phe Cys Ile His Asn Ile Ala Tyr Gln Gly Arg Phe Ser Phe
260 265 270

Ser Asp Phe Pro Leu Leu Asn Leu Pro Asp Glu Phe Arg Gly Ser Phe
275 280 285

Asp Phe Ile Asp Gly Tyr Glu Lys Pro Val Lys Gly Arg Lys Ile Asn
290 295 300

Trp Met Lys Ala Gly Ile Leu Glu Ser His Arg Val Val Thr Val Ser
305 310 315 320

Pro Tyr Tyr Ala Gln Glu Leu Val Ser Ala Val Asp Lys Gly Val Glu
325 330 335

Leu Asp Ser Val Leu Arg Lys Thr Cys Ile Thr Gly Ile Val Asn Gly
340 345 350

Met Asp Thr Gln Glu Trp Asn Pro Ala Thr Asp Lys Tyr Thr Asp Val
355 360 365

Lys Tyr Asp Ile Thr Thr Val Met Asp Ala Lys Pro Leu Leu Lys Glu
370 375 380

Ala Leu Gln Ala Ala Val Gly Leu Pro Val Asp Lys Lys Ile Pro Leu
385 390 395 400

Ile Gly Phe Ile Gly Arg Leu Glu Glu Gln Lys Gly Ser Asp Ile Leu
405 410 415

Val Ala Ala Ile His Lys Phe Ile Gly Leu Asp Val Gln Ile Val Val
420 425 430

Leu Gly Thr Gly Lys Lys Glu Phe Glu Gln Glu Ile Glu Gln Leu Glu
435 440 445

Val Leu Tyr Pro Asn Lys Ala Lys Gly Val Ala Lys Phe Asn Val Pro
450 455 460

Leu Ala His Met Ile Thr Ala Gly Ala Asp Phe Met Leu Val Pro Ser
465 470 475 480

Arg Phe Glu Pro Cys Gly Leu Ile Gln Leu His Ala Met Arg Tyr Gly
485 490 495

Thr Val Pro Ile Cys Ala Ser Thr Gly Gly Leu Val Asp Thr Val Lys
500 505 510

Glu Gly Tyr Thr Gly Phe His Met Gly Ala Phe Asn Val Glu Cys Asp
515 520 525

Val Val Asp Pro Ala Asp Val Leu Lys Ile Val Thr Thr Val Ala Arg
530 535 540

Ala Leu Ala Val Tyr Gly Thr Leu Ala Phe Ala Glu Met Ile Lys Asn
545 550 555 560

Cys Met Ser Glu Glu Leu Ser Trp Lys Glu Pro Ala Lys Lys Trp Glu
565 570 575

Thr Leu Leu Leu Gly Leu Gly Ala Ser Gly Ser Glu Pro Gly Val Glu
580 585 590

```
Gly Glu Glu Ile Ala Pro Leu Ala Lys Glu Asn Val Ala Thr Pro
        595                 600                 605
```

<210> 8

<211> 31

<212> DNA

<213> synthetic

<400> 8
gatgggtacc agcacttcta cttggcagag g 31

<210> 9

<211> 1004

<212> DNA

<213> Solanum tuberosum

<400> 9
```
tcaaactagt cacaaccagt ccatttctgg aggtcgttcc ttcgcagaaa tactgattgg     60
taactccttg gggaaatcct ccatatcaca agagtcatta cttagaggct gctcgttaca    120
caagatgatc agattaatta catctacaat tggtggtcat gcatacctca acttcatggg    180
caatgaattt ggtcacccaa agagagtaga gtttccaatg tcaagcaaca atttctcctt    240
ttcactggct aaccgtcgct gggatctatt ggaagatgtt gtacattatc aattgttctc    300
atttgataag ggtatgatgg acttggataa aaatgggaga attttgtcca gaggtcttgc    360
caacattcac catgtcaatg atactaccat ggtgatttct tacttgagag gtcccaatct    420
ctttgtgttc aactttcatc ctgtcaattc atatgaaaga tacattatag gtgtggaaga    480
agctggagag tatcaagtca cattaaatac agatgaaaac aagtatggtg gtagaggact    540
acttggccat gatcagaata ttcaaagaac cattagtaga agagctgatg gaatgagatt    600
ttgcttggaa gtgcctctgc caagtagaag tgctcaggtc tacaagttga cccgaattct    660
aagagcatga tcactctagt aatcaaagtg cctcatatga tgacacaaaa ggaaaggttc    720
tacattgccc ttacactgat caatattgac acctttccga ggtgagtttc tgtgattctt    780
gagcagactg ttggctagtc aattatcatg aacttttgcc ttcagcatcc ggatagtcgc    840
ttctcctgtg caatgagggc atggacgaat ttttttttgg cttgtcatgg gggtcataag    900
catccgccag attaagattt cacaggcctc gagtaaaacc atcacttact ttaaggatac    960
acaaacacac caacggggtg caggctctga taccttctaa agtg                    1004
```

<210> 10

<211> 2096

<212> DNA

<213> Solanum tuberosum

<400> 10
aacaatgctc tctctgtcgg attcaattcg aatttcttca ccattgagcg attctcgtct 60

tagttttcta tctcaaaccg gaagcagaac cagtcgccag cttaaatttg ttcgcagccg 120

ccgggctcga gtttcgaggt gtagatgctc agcaacggag caaccgccac cgcaacgacg 180

gaagcaacga ccggagaagt acaaacagtc ggaggaaggg aaaggaatcg atcctgttgg 240

atttctcagc aaatacggca ttactcataa agcgtttgct caatttcttc gtgaaagata 300

taaatcattg aaggacttga aggatgaaat attgactcgt catttcagtc tcaaggagat 360

gtctactggg tatgaattaa tgggtatgca tcgcaacata caacatcgag tggatttctt 420

ggaatgggct ccaggtgctc gctactgtgc tctgattggt gacttcaatg ggtggtcaac 480

aactggtaac tgtgccagag agggtcattt tggtcatgac gattatgggt attggtttat 540

tattcttgaa gataaattac gtgaaggaga agaacctgat aaattgtatt ttcaacagta 600

caattatgcg gaggactatg gtaaaggtga cacgggtatt accgtcgagg aaatctttaa 660

aaaagcaaat gatgagtatt gggaacctgg agaagatcgc ttcattaaat cacgttatga 720

ggtggcagca aagttatatg aggaaatgtt cggaccaaat ggacctcaaa cagaagagga 780

actagaagca atgcctgatg cagctacacg atacaaaact tggaaagagc aacaaaaaga 840

ggatccggca agcaatttgc catcgtatga tgtggtagat agtggaaaag aatatgatat 900

ttacaatatt ataggtgatc ctgaatcgtt taagaaattt cgtatgaaac agcctcctat 960

tgcttactgg ttagaaacta aaaagggaag gaaaggctgg ttacagaaat atatgcctgc 1020

tttacctcat ggaagcaaat acagggtgta ttttaacaca ccaaatgggc ctcttgaacg 1080

agttcctgcg tgggccaatt ttgtcattcc agatgcaggc gggatggcat tagcagtcca 1140

ttgggaacca cctcctgaat atgcttataa atggaaacac aagctaccag tcaagcctaa 1200

gtccttgcgc atatatgaat gtcatgttgg catctctggc caggaaccaa aagtttcatc 1260

tttcaatgat tttattagca aggtccttcc gcatgtaaaa gaagctggat acaatgcaat 1320

acaaattatt ggagttgttg agcacaagga ttatttcact gttggatata gagtgaccaa 1380

tttttatgct gttagtagcc gttatggcac accggatgac ttcaagcgct tggttgatga 1440

agcacatggg cttggactgc ttgtcttttt ggagattgtg cactcttatg cagcagcaga 1500

tgaaatggtt gggttatctc tttttgatgg agcaaatgat tgctatttcc acactggtaa 1560

acgtggacac cacaaattct ggggcacacg gatgttcaaa tatggagatc ttgatgttct 1620

gcactttctt ctttcaaatc tgaactggtg ggtggaggag tatcatgtcg atggcttcca 1680

ttttcattcg ctctcgtcca tgttgtatac gcataatgga tttgcttcat ttactggtga 1740

catggatgaa tactgtaacc aatatgttga caaggaggcc ttattgtacc tcatattagc 1800

aaatgaagta ttacatgctc ttcatcctaa tgtgatcacg attgctgagg atgcaactct 1860

```
gtatcctgga ctctgcgatc caacatctca aggtggactg ggctttgatt attttgccaa    1920
tctttctgcc tcagagatgt ggcttgcatt acttgaaaat actcctgatc atgaatggtg    1980
catgagtaag attgttagca cattagtggg cgatagacaa aatactgata aaatgctttt    2040
gtatgcagaa aatcacaacc agtccatttc tggaggtcgt tccttcgcag aaatac        2096

<210>  11
<211>  3204
<212>  DNA
<213>  Solanum tuberosum


<220>
<221>  CDS
<222>  (99)..(2804)
<223>


<400>  11
gaattgtaat acgactcact atagggcgaa ttgggccctc tagatgcatg ctcgagcggc      60

cgccagtgtg atggatatct gcagaattcg gcttaaca atg ctc tct ctg tcg gat     116
                                          Met Leu Ser Leu Ser Asp
                                          1               5

tca att cga att tct tca cca ttg agc gat tct cgt ctt agt ttt cta       164
Ser Ile Arg Ile Ser Ser Pro Leu Ser Asp Ser Arg Leu Ser Phe Leu
            10                  15                  20

tct caa acc gga agc aga acc agt cgc cag ctt aaa ttt gtt cgc agc       212
Ser Gln Thr Gly Ser Arg Thr Ser Arg Gln Leu Lys Phe Val Arg Ser
        25                  30                  35

cgc cgg gct cga gtt tcg agg tgt aga tgc tca gca acg gag caa ccg       260
Arg Arg Ala Arg Val Ser Arg Cys Arg Cys Ser Ala Thr Glu Gln Pro
    40                  45                  50

cca ccg caa cga cgg aag caa cga ccg gag aag tac aaa cag tcg gag       308
Pro Pro Gln Arg Arg Lys Gln Arg Pro Glu Lys Tyr Lys Gln Ser Glu
55                  60                  65                  70

gaa gag aaa gga atc gat cct gtt gga ttt ctc agc aaa tac ggc att       356
Glu Glu Lys Gly Ile Asp Pro Val Gly Phe Leu Ser Lys Tyr Gly Ile
                75                  80                  85

act cat aaa gcg ttt gct caa ttt ctt cgt gaa aga tat aaa tca ttg       404
Thr His Lys Ala Phe Ala Gln Phe Leu Arg Glu Arg Tyr Lys Ser Leu
            90                  95                  100

aag gac ttg aag gat gaa ata ttg act cgt cat ttc agt ctc aag gag       452
Lys Asp Leu Lys Asp Glu Ile Leu Thr Arg His Phe Ser Leu Lys Glu
        105                 110                 115

atg tct act ggg tat gaa tta atg ggt atg cat cgc aac ata caa cat       500
Met Ser Thr Gly Tyr Glu Leu Met Gly Met His Arg Asn Ile Gln His
    120                 125                 130
```

```
cga gtg gat ttc ttg gaa tgg gct cca ggt gct cgc tac tgt gct ctg      548
Arg Val Asp Phe Leu Glu Trp Ala Pro Gly Ala Arg Tyr Cys Ala Leu
135                 140                 145                 150

att ggt gac ttc aat ggg tgg tca aca act ggt aac tgt gcc aga gag      596
Ile Gly Asp Phe Asn Gly Trp Ser Thr Thr Gly Asn Cys Ala Arg Glu
                155                 160                 165

ggt cat ttt ggt cat gac gat tat ggg tat tgg ttt att att ctt gaa      644
Gly His Phe Gly His Asp Asp Tyr Gly Tyr Trp Phe Ile Ile Leu Glu
            170                 175                 180

gat aaa tta cgt gaa gga gaa gaa cct gat aaa ttg tat ttt caa cag      692
Asp Lys Leu Arg Glu Gly Glu Glu Pro Asp Lys Leu Tyr Phe Gln Gln
        185                 190                 195

tac aat tat gcg gag gac tat gat aaa ggt gac acg ggt att acc gtc      740
Tyr Asn Tyr Ala Glu Asp Tyr Asp Lys Gly Asp Thr Gly Ile Thr Val
    200                 205                 210

gag gaa atc ttt aaa aaa gca aat gat gag tat tgg gaa cct gga gaa      788
Glu Glu Ile Phe Lys Lys Ala Asn Asp Glu Tyr Trp Glu Pro Gly Glu
215                 220                 225                 230

gat cgc ttc att aaa tca cgt tat gag gtg gca gca aag tta tat gag      836
Asp Arg Phe Ile Lys Ser Arg Tyr Glu Val Ala Ala Lys Leu Tyr Glu
                235                 240                 245

gaa atg ttc gga cca aat gga cct caa aca gaa gag gaa cta gaa gca      884
Glu Met Phe Gly Pro Asn Gly Pro Gln Thr Glu Glu Glu Leu Glu Ala
            250                 255                 260

atg cct gat gca gct aca cga tac aaa act tgg aaa gag caa caa aaa      932
Met Pro Asp Ala Ala Thr Arg Tyr Lys Thr Trp Lys Glu Gln Gln Lys
        265                 270                 275

aag gat ccg gca agc aat ttg cca tcg tat gat gtg gta gat agt gga      980
Lys Asp Pro Ala Ser Asn Leu Pro Ser Tyr Asp Val Val Asp Ser Gly
    280                 285                 290

aaa gaa tat gat att tac aat att ata ggt gat cct gaa tcg ttt aag     1028
Lys Glu Tyr Asp Ile Tyr Asn Ile Ile Gly Asp Pro Glu Ser Phe Lys
295                 300                 305                 310

aaa ttt cgt atg aaa cag cct cct att gct tac tgg tta gaa act aaa     1076
Lys Phe Arg Met Lys Gln Pro Pro Ile Ala Tyr Trp Leu Glu Thr Lys
                315                 320                 325

aag gga agg aaa ggc tgg tta cag aaa tat atg cct gct tta cct cat     1124
Lys Gly Arg Lys Gly Trp Leu Gln Lys Tyr Met Pro Ala Leu Pro His
            330                 335                 340

gga agc aaa cac agg gtg tat ttt aac aca cca aat ggg cct ctt gaa     1172
Gly Ser Lys His Arg Val Tyr Phe Asn Thr Pro Asn Gly Pro Leu Glu
        345                 350                 355

cga gtt cct gcg tgg gcc aat ttt gtc att cca gat gca gac ggg atg     1220
Arg Val Pro Ala Trp Ala Asn Phe Val Ile Pro Asp Ala Asp Gly Met
    360                 365                 370

gca tta gca gtc cat tgg gaa cca cct cct gaa tat gct tat aaa tgg     1268
Ala Leu Ala Val His Trp Glu Pro Pro Pro Glu Tyr Ala Tyr Lys Trp
375                 380                 385                 390

aaa cac aag cta cca gtc aag cct aag tcc ttg cgc ata tat gaa tgt     1316
Lys His Lys Leu Pro Val Lys Pro Lys Ser Leu Arg Ile Tyr Glu Cys
                395                 400                 405
```

```
cat gtt ggc atc tct ggc cag gaa cca aaa gtt tca tct ttc aat gat      1364
His Val Gly Ile Ser Gly Gln Glu Pro Lys Val Ser Ser Phe Asn Asp
            410                 415                 420

ttt att agc aag gtc ctt ccg cat gta aaa gaa gct gga tac aat gca      1412
Phe Ile Ser Lys Val Leu Pro His Val Lys Glu Ala Gly Tyr Asn Ala
        425                 430                 435

acg caa att att gga gtt gtt gag cac aag gat tat ttc act gtt gga      1460
Thr Gln Ile Ile Gly Val Val Glu His Lys Asp Tyr Phe Thr Val Gly
    440                 445                 450

tat aga gtg acc aat ttt tat gct gtt agt agc cgt tat ggc aca ccg      1508
Tyr Arg Val Thr Asn Phe Tyr Ala Val Ser Ser Arg Tyr Gly Thr Pro
455                 460                 465                 470

gat gac ttc aag cgc ttg gtt gat gaa gca cat ggg ctt gga ctg ctt      1556
Asp Asp Phe Lys Arg Leu Val Asp Glu Ala His Gly Leu Gly Leu Leu
                475                 480                 485

gtc ttt ttg gag att gtg cac tcc tat gca gca gca gat gaa atg gtt      1604
Val Phe Leu Glu Ile Val His Ser Tyr Ala Ala Ala Asp Glu Met Val
            490                 495                 500

ggg tta tct ctt ttt gat gga gca aat gat tgc tat ttc cac act ggt      1652
Gly Leu Ser Leu Phe Asp Gly Ala Asn Asp Cys Tyr Phe His Thr Gly
        505                 510                 515

aaa cgt gga cac cac aaa ttc tgg ggc aca cgg atg ttc aaa tat gga      1700
Lys Arg Gly His His Lys Phe Trp Gly Thr Arg Met Phe Lys Tyr Gly
    520                 525                 530

gat cct gat gtt ctg cac ttt ctt ctt tca aat ctg aac tgg tgg gtg      1748
Asp Pro Asp Val Leu His Phe Leu Leu Ser Asn Leu Asn Trp Trp Val
535                 540                 545                 550

gag gag tat cat gtc gat ggc ttc cat ttt cat tcg ctc tcg tcc atg      1796
Glu Glu Tyr His Val Asp Gly Phe His Phe His Ser Leu Ser Ser Met
                555                 560                 565

ttg tat acg cat aat gga ttt gct tca ttt act ggt gac atg gat gaa      1844
Leu Tyr Thr His Asn Gly Phe Ala Ser Phe Thr Gly Asp Met Asp Glu
            570                 575                 580

tac tgt aac caa tat gtt gac aag gag gcc tta ttg tac ctc ata tta      1892
Tyr Cys Asn Gln Tyr Val Asp Lys Glu Ala Leu Leu Tyr Leu Ile Leu
        585                 590                 595

gca aat gaa gta tta cat gct ctt cat cct aat gtg atc acg att gct      1940
Ala Asn Glu Val Leu His Ala Leu His Pro Asn Val Ile Thr Ile Ala
    600                 605                 610

gtg gat gca act ctg tat cct gga ctc tgc gat cca aca tct caa ggt      1988
Val Asp Ala Thr Leu Tyr Pro Gly Leu Cys Asp Pro Thr Ser Gln Gly
615                 620                 625                 630

gga ctg ggc ttt gat tat ttt gcc aat ctt tct gcc tca gag atg tgg      2036
Gly Leu Gly Phe Asp Tyr Phe Ala Asn Leu Ser Ala Ser Glu Met Trp
                635                 640                 645

ctt gca tta ctt gaa aat act cct gat cat gaa tgg tgc atg agt aag      2084
Leu Ala Leu Leu Glu Asn Thr Pro Asp His Glu Trp Cys Met Ser Lys
            650                 655                 660

att gtt agc aca tta gtg ggc gat aga caa aat act gat aaa atg ctt      2132
Ile Val Ser Thr Leu Val Gly Asp Arg Gln Asn Thr Asp Lys Met Leu
        665                 670                 675
```

```
ttg tat gca gaa aat cac aac cag tcc att tct gga ggt cgt tcc ttc      2180
Leu Tyr Ala Glu Asn His Asn Gln Ser Ile Ser Gly Gly Arg Ser Phe
    680                 685                 690

gca gaa ata ctg att ggt aac tcc ttg ggg aaa tct tcc ata tca caa      2228
Ala Glu Ile Leu Ile Gly Asn Ser Leu Gly Lys Ser Ser Ile Ser Gln
695                 700                 705                 710

gag tca tta ctt aga ggc tgc tcg tta cac aag atg atc aga tta att      2276
Glu Ser Leu Leu Arg Gly Cys Ser Leu His Lys Met Ile Arg Leu Ile
                715                 720                 725

aca tct aca att ggt ggt cat gca tac ctc aac ttc atg ggc aat gaa      2324
Thr Ser Thr Ile Gly Gly His Ala Tyr Leu Asn Phe Met Gly Asn Glu
            730                 735                 740

ttt ggt cac cca aag aga gta gag ttt cca atg tca agc aac aat ttc      2372
Phe Gly His Pro Lys Arg Val Glu Phe Pro Met Ser Ser Asn Asn Phe
        745                 750                 755

tcc ttt tca ctg gct aac cgt cgc tgg gat cta ttg gaa gat gtt gta      2420
Ser Phe Ser Leu Ala Asn Arg Arg Trp Asp Leu Leu Glu Asp Val Val
    760                 765                 770

cat tat caa tta ttc tca ttt gat aag gat atg atg gac ttg gat aaa      2468
His Tyr Gln Leu Phe Ser Phe Asp Lys Asp Met Met Asp Leu Asp Lys
775                 780                 785                 790

aat ggg aga att ttg tcc aga ggt ctt gcc aac att cac cat gtc aat      2516
Asn Gly Arg Ile Leu Ser Arg Gly Leu Ala Asn Ile His His Val Asn
                795                 800                 805

gat act acc atg gtg att tct tac ttg aga ggt ccc aat ctc ttt gtg      2564
Asp Thr Thr Met Val Ile Ser Tyr Leu Arg Gly Pro Asn Leu Phe Val
            810                 815                 820

ttc aac ttt cat cct gtc aat tca tat gaa aga tac att ata ggt gtg      2612
Phe Asn Phe His Pro Val Asn Ser Tyr Glu Arg Tyr Ile Ile Gly Val
        825                 830                 835

gaa gaa gct gga gag tat caa gtc aca tta aat aca gat gaa aac aag      2660
Glu Glu Ala Gly Glu Tyr Gln Val Thr Leu Asn Thr Asp Glu Asn Lys
    840                 845                 850

tat ggt ggt aga gga cta ctt ggc cat gat cag aat act caa aga acc      2708
Tyr Gly Gly Arg Gly Leu Leu Gly His Asp Gln Asn Thr Gln Arg Thr
855                 860                 865                 870

att agt aga aga gct gat gga atg aga ttt tgc ttg gaa gta cct ctg      2756
Ile Ser Arg Arg Ala Asp Gly Met Arg Phe Cys Leu Glu Val Pro Leu
                875                 880                 885

cca agt aga agt gct cag gtc tac aag ttg acc cga att cta aga gca      2804
Pro Ser Arg Ser Ala Gln Val Tyr Lys Leu Thr Arg Ile Leu Arg Ala
            890                 895                 900

tgatcactct agcaatcaaa gtgcctcata tgatcacaca aaagggaagg ttctacattg    2864

cccttatact gaccaatatt gtggcctttc cgaggtgagt ttctgtgatt cttgagcaca    2924

ggctgttggc tagtcagtta tcatgaactt ttgccttcag catctggata agcgcttctc    2984

ctgtgcaatg agggcatgga cgaaattttt ttggttcgtc atgggagtca aaagcatctg    3044

ccagattaag atttcacagg cctcgagtaa aaccatcact tacttaggat acacaaacac    3104

atcaacgggg tgcaggctct gataccttct aaagtgaagc cgaattccag cacactggcg    3164
```

gccgttacta gtggatccga gctcggtacc aagcttggcg 3204

<210> 12

<211> 902

<212> PRT

<213> Solanum tuberosum

<400> 12

```
Met Leu Ser Leu Ser Asp Ser Ile Arg Ile Ser Ser Pro Leu Ser Asp
1               5                   10                  15

Ser Arg Leu Ser Phe Leu Ser Gln Thr Gly Ser Arg Thr Ser Arg Gln
            20                  25                  30

Leu Lys Phe Val Arg Ser Arg Arg Ala Arg Val Ser Arg Cys Arg Cys
        35                  40                  45

Ser Ala Thr Glu Gln Pro Pro Pro Gln Arg Arg Lys Gln Arg Pro Glu
    50                  55                  60

Lys Tyr Lys Gln Ser Glu Glu Glu Lys Gly Ile Asp Pro Val Gly Phe
65                  70                  75                  80

Leu Ser Lys Tyr Gly Ile Thr His Lys Ala Phe Ala Gln Phe Leu Arg
                85                  90                  95

Glu Arg Tyr Lys Ser Leu Lys Asp Leu Lys Asp Glu Ile Leu Thr Arg
            100                 105                 110

His Phe Ser Leu Lys Glu Met Ser Thr Gly Tyr Glu Leu Met Gly Met
        115                 120                 125

His Arg Asn Ile Gln His Arg Val Asp Phe Leu Glu Trp Ala Pro Gly
    130                 135                 140

Ala Arg Tyr Cys Ala Leu Ile Gly Asp Phe Asn Gly Trp Ser Thr Thr
145                 150                 155                 160

Gly Asn Cys Ala Arg Glu Gly His Phe Gly His Asp Asp Tyr Gly Tyr
                165                 170                 175

Trp Phe Ile Ile Leu Glu Asp Lys Leu Arg Glu Gly Glu Glu Pro Asp
            180                 185                 190

Lys Leu Tyr Phe Gln Gln Tyr Asn Tyr Ala Glu Asp Tyr Asp Lys Gly
        195                 200                 205
```

```
Asp Thr Gly Ile Thr Val Glu Glu Ile Phe Lys Lys Ala Asn Asp Glu
    210                 215                 220

Tyr Trp Glu Pro Gly Glu Asp Arg Phe Ile Lys Ser Arg Tyr Glu Val
225                 230                 235                 240

Ala Ala Lys Leu Tyr Glu Glu Met Phe Gly Pro Asn Gly Pro Gln Thr
                245                 250                 255

Glu Glu Glu Leu Glu Ala Met Pro Asp Ala Ala Thr Arg Tyr Lys Thr
            260                 265                 270

Trp Lys Glu Gln Gln Lys Lys Asp Pro Ala Ser Asn Leu Pro Ser Tyr
        275                 280                 285

Asp Val Val Asp Ser Gly Lys Glu Tyr Asp Ile Tyr Asn Ile Ile Gly
    290                 295                 300

Asp Pro Glu Ser Phe Lys Lys Phe Arg Met Lys Gln Pro Pro Ile Ala
305                 310                 315                 320

Tyr Trp Leu Glu Thr Lys Lys Gly Arg Lys Gly Trp Leu Gln Lys Tyr
                325                 330                 335

Met Pro Ala Leu Pro His Gly Ser Lys His Arg Val Tyr Phe Asn Thr
            340                 345                 350

Pro Asn Gly Pro Leu Glu Arg Val Pro Ala Trp Ala Asn Phe Val Ile
        355                 360                 365

Pro Asp Ala Asp Gly Met Ala Leu Ala Val His Trp Glu Pro Pro Pro
    370                 375                 380

Glu Tyr Ala Tyr Lys Trp Lys His Lys Leu Pro Val Lys Pro Lys Ser
385                 390                 395                 400

Leu Arg Ile Tyr Glu Cys His Val Gly Ile Ser Gly Gln Glu Pro Lys
                405                 410                 415

Val Ser Ser Phe Asn Asp Phe Ile Ser Lys Val Leu Pro His Val Lys
            420                 425                 430

Glu Ala Gly Tyr Asn Ala Thr Gln Ile Ile Gly Val Val Glu His Lys
        435                 440                 445

Asp Tyr Phe Thr Val Gly Tyr Arg Val Thr Asn Phe Tyr Ala Val Ser
    450                 455                 460

Ser Arg Tyr Gly Thr Pro Asp Asp Phe Lys Arg Leu Val Asp Glu Ala
465                 470                 475                 480
```

His Gly Leu Gly Leu Leu Val Phe Leu Glu Ile Val His Ser Tyr Ala
485 490 495

Ala Ala Asp Glu Met Val Gly Leu Ser Leu Phe Asp Gly Ala Asn Asp
500 505 510

Cys Tyr Phe His Thr Gly Lys Arg Gly His His Lys Phe Trp Gly Thr
515 520 525

Arg Met Phe Lys Tyr Gly Asp Pro Asp Val Leu His Phe Leu Leu Ser
530 535 540

Asn Leu Asn Trp Trp Val Glu Glu Tyr His Val Asp Gly Phe His Phe
545 550 555 560

His Ser Leu Ser Ser Met Leu Tyr Thr His Asn Gly Phe Ala Ser Phe
565 570 575

Thr Gly Asp Met Asp Glu Tyr Cys Asn Gln Tyr Val Asp Lys Glu Ala
580 585 590

Leu Leu Tyr Leu Ile Leu Ala Asn Glu Val Leu His Ala Leu His Pro
595 600 605

Asn Val Ile Thr Ile Ala Val Asp Ala Thr Leu Tyr Pro Gly Leu Cys
610 615 620

Asp Pro Thr Ser Gln Gly Gly Leu Gly Phe Asp Tyr Phe Ala Asn Leu
625 630 635 640

Ser Ala Ser Glu Met Trp Leu Ala Leu Leu Glu Asn Thr Pro Asp His
645 650 655

Glu Trp Cys Met Ser Lys Ile Val Ser Thr Leu Val Gly Asp Arg Gln
660 665 670

Asn Thr Asp Lys Met Leu Leu Tyr Ala Glu Asn His Asn Gln Ser Ile
675 680 685

Ser Gly Gly Arg Ser Phe Ala Glu Ile Leu Ile Gly Asn Ser Leu Gly
690 695 700

Lys Ser Ser Ile Ser Gln Glu Ser Leu Leu Arg Gly Cys Ser Leu His
705 710 715 720

Lys Met Ile Arg Leu Ile Thr Ser Thr Ile Gly Gly His Ala Tyr Leu
725 730 735

Asn Phe Met Gly Asn Glu Phe Gly His Pro Lys Arg Val Glu Phe Pro
740 745 750

```
Met Ser Ser Asn Asn Phe Ser Phe Ser Leu Ala Asn Arg Arg Trp Asp
        755                 760                 765

Leu Leu Glu Asp Val Val His Tyr Gln Leu Phe Ser Phe Asp Lys Asp
    770                 775                 780

Met Met Asp Leu Asp Lys Asn Gly Arg Ile Leu Ser Arg Gly Leu Ala
785                 790                 795                 800

Asn Ile His His Val Asn Asp Thr Thr Met Val Ile Ser Tyr Leu Arg
                805                 810                 815

Gly Pro Asn Leu Phe Val Phe Asn Phe His Pro Val Asn Ser Tyr Glu
            820                 825                 830

Arg Tyr Ile Ile Gly Val Glu Glu Ala Gly Glu Tyr Gln Val Thr Leu
        835                 840                 845

Asn Thr Asp Glu Asn Lys Tyr Gly Gly Arg Gly Leu Leu Gly His Asp
    850                 855                 860

Gln Asn Thr Gln Arg Thr Ile Ser Arg Arg Ala Asp Gly Met Arg Phe
865                 870                 875                 880

Cys Leu Glu Val Pro Leu Pro Ser Arg Ser Ala Gln Val Tyr Lys Leu
                885                 890                 895

Thr Arg Ile Leu Arg Ala
            900
```

<210> 13

<211> 3047

<212> DNA

<213> Solanum tuberosum

<220>

<221> CDS

<222> (5)..(2710)

<223>

<400> 13

```
aaca atg ctc tct ctg tcg gat tca att cga att tct tca cca ttg agc      49
     Met Leu Ser Leu Ser Asp Ser Ile Arg Ile Ser Ser Pro Leu Ser
     1               5                   10                  15

gat tct cgt ctt agt ttt cta tct caa acc gga agc aga acc agt cgc      97
Asp Ser Arg Leu Ser Phe Leu Ser Gln Thr Gly Ser Arg Thr Ser Arg
                20                  25                  30
```

```
cag ctt aaa ttt gtt cgc agc cgc cgg gct cga gtt tcg agg tgt aga      145
Gln Leu Lys Phe Val Arg Ser Arg Arg Ala Arg Val Ser Arg Cys Arg
            35                  40                  45

tgc tca gca acg gag caa ccg cca ccg caa cga cgg aag caa cga ccg      193
Cys Ser Ala Thr Glu Gln Pro Pro Pro Gln Arg Arg Lys Gln Arg Pro
        50                  55                  60

gag aag tac aaa cag tcg gag gaa ggg aaa gga atc gat cct gtt gga      241
Glu Lys Tyr Lys Gln Ser Glu Glu Gly Lys Gly Ile Asp Pro Val Gly
    65                  70                  75

ttt ctc agc aaa tac ggc att act cat aaa gcg ttt gct caa ttt ctt      289
Phe Leu Ser Lys Tyr Gly Ile Thr His Lys Ala Phe Ala Gln Phe Leu
80                  85                  90                  95

cgt gaa aga tat aaa tca ttg aag gac ttg aag gat gaa ata ttg act      337
Arg Glu Arg Tyr Lys Ser Leu Lys Asp Leu Lys Asp Glu Ile Leu Thr
                100                 105                 110

cgt cat ttc agt ctc aag gag atg tct act ggg tat gaa tta atg ggt      385
Arg His Phe Ser Leu Lys Glu Met Ser Thr Gly Tyr Glu Leu Met Gly
            115                 120                 125

atg cat cgc aac ata caa cat cga gtg gat ttc ttg gaa tgg gct cca      433
Met His Arg Asn Ile Gln His Arg Val Asp Phe Leu Glu Trp Ala Pro
        130                 135                 140

ggt gct cgc tac tgt gct ctg att ggt gac ttc aat ggg tgg tca aca      481
Gly Ala Arg Tyr Cys Ala Leu Ile Gly Asp Phe Asn Gly Trp Ser Thr
    145                 150                 155

act ggt aac tgt gcc aga gag ggt cat ttt ggt cat gac gat tat ggg      529
Thr Gly Asn Cys Ala Arg Glu Gly His Phe Gly His Asp Asp Tyr Gly
160                 165                 170                 175

tat tgg ttt att att ctt gaa gat aaa tta cgt gaa gga gaa gaa cct      577
Tyr Trp Phe Ile Ile Leu Glu Asp Lys Leu Arg Glu Gly Glu Glu Pro
                180                 185                 190

gat aaa ttg tat ttt caa cag tac aat tat gcg gag gac tat ggt aaa      625
Asp Lys Leu Tyr Phe Gln Gln Tyr Asn Tyr Ala Glu Asp Tyr Gly Lys
            195                 200                 205

ggt gac acg ggt att acc gtc gag gaa atc ttt aaa aaa gca aat gat      673
Gly Asp Thr Gly Ile Thr Val Glu Glu Ile Phe Lys Lys Ala Asn Asp
        210                 215                 220

gag tat tgg gaa cct gga gaa gat cgc ttc att aaa tca cgt tat gag      721
Glu Tyr Trp Glu Pro Gly Glu Asp Arg Phe Ile Lys Ser Arg Tyr Glu
    225                 230                 235

gtg gca gca aag tta tat gag gaa atg ttc gga cca aat gga cct caa      769
Val Ala Ala Lys Leu Tyr Glu Glu Met Phe Gly Pro Asn Gly Pro Gln
240                 245                 250                 255

aca gaa gag gaa cta gaa gca atg cct gat gca gct aca cga tac aaa      817
Thr Glu Glu Glu Leu Glu Ala Met Pro Asp Ala Ala Thr Arg Tyr Lys
                260                 265                 270

act tgg aaa gag caa caa aaa gag gat ccg gca agc aat ttg cca tcg      865
Thr Trp Lys Glu Gln Gln Lys Glu Asp Pro Ala Ser Asn Leu Pro Ser
            275                 280                 285

tat gat gtg gta gat agt gga aaa gaa tat gat att tac aat att ata      913
Tyr Asp Val Val Asp Ser Gly Lys Glu Tyr Asp Ile Tyr Asn Ile Ile
        290                 295                 300
```

```
ggt gat cct gaa tcg ttt aag aaa ttt cgt atg aaa cag cct cct att      961
Gly Asp Pro Glu Ser Phe Lys Lys Phe Arg Met Lys Gln Pro Pro Ile
    305                 310                 315

gct tac tgg tta gaa act aaa aag gga agg aaa ggc tgg tta cag aaa     1009
Ala Tyr Trp Leu Glu Thr Lys Lys Gly Arg Lys Gly Trp Leu Gln Lys
320                 325                 330                 335

tat atg cct gct tta cct cat gga agc aaa tac agg gtg tat ttt aac     1057
Tyr Met Pro Ala Leu Pro His Gly Ser Lys Tyr Arg Val Tyr Phe Asn
                340                 345                 350

aca cca aat ggg cct ctt gaa cga gtt cct gcg tgg gcc aat ttt gtc     1105
Thr Pro Asn Gly Pro Leu Glu Arg Val Pro Ala Trp Ala Asn Phe Val
            355                 360                 365

att cca gat gca ggc ggg atg gca tta gca gtc cat tgg gaa cca cct     1153
Ile Pro Asp Ala Gly Gly Met Ala Leu Ala Val His Trp Glu Pro Pro
        370                 375                 380

cct gaa tat gct tat aaa tgg aaa cac aag cta cca gtc aag cct aag     1201
Pro Glu Tyr Ala Tyr Lys Trp Lys His Lys Leu Pro Val Lys Pro Lys
    385                 390                 395

tcc ttg cgc ata tat gaa tgt cat gtt ggc atc tct ggc cag gaa cca     1249
Ser Leu Arg Ile Tyr Glu Cys His Val Gly Ile Ser Gly Gln Glu Pro
400                 405                 410                 415

aaa gtt tca tct ttc aat gat ttt att agc aag gtc ctt ccg cat gta     1297
Lys Val Ser Ser Phe Asn Asp Phe Ile Ser Lys Val Leu Pro His Val
                420                 425                 430

aaa gaa gct gga tac aat gca ata caa att att gga gtt gtt gag cac     1345
Lys Glu Ala Gly Tyr Asn Ala Ile Gln Ile Ile Gly Val Val Glu His
            435                 440                 445

aag gat tat ttc act gtt gga tat aga gtg acc aat ttt tat gct gtt     1393
Lys Asp Tyr Phe Thr Val Gly Tyr Arg Val Thr Asn Phe Tyr Ala Val
        450                 455                 460

agt agc cgt tat ggc aca ccg gat gac ttc aag cgc ttg gtt gat gaa     1441
Ser Ser Arg Tyr Gly Thr Pro Asp Asp Phe Lys Arg Leu Val Asp Glu
    465                 470                 475

gca cat ggg ctt gga ctg ctt gtc ttt ttg gag att gtg cac tct tat     1489
Ala His Gly Leu Gly Leu Leu Val Phe Leu Glu Ile Val His Ser Tyr
480                 485                 490                 495

gca gca gca gat gaa atg gtt ggg tta tct ctt ttt gat gga gca aat     1537
Ala Ala Ala Asp Glu Met Val Gly Leu Ser Leu Phe Asp Gly Ala Asn
                500                 505                 510

gat tgc tat ttc cac act ggt aaa cgt gga cac cac aaa ttc tgg ggc     1585
Asp Cys Tyr Phe His Thr Gly Lys Arg Gly His His Lys Phe Trp Gly
            515                 520                 525

aca cgg atg ttc aaa tat gga gat ctt gat gtt ctg cac ttt ctt ctt     1633
Thr Arg Met Phe Lys Tyr Gly Asp Leu Asp Val Leu His Phe Leu Leu
        530                 535                 540

tca aat ctg aac tgg tgg gtg gag gag tat cat gtc gat ggc ttc cat     1681
Ser Asn Leu Asn Trp Trp Val Glu Glu Tyr His Val Asp Gly Phe His
    545                 550                 555

ttt cat tcg ctc tcg tcc atg ttg tat acg cat aat gga ttt gct tca     1729
Phe His Ser Leu Ser Ser Met Leu Tyr Thr His Asn Gly Phe Ala Ser
560                 565                 570                 575
```

```
ttt act ggt gac atg gat gaa tac tgt aac caa tat gtt gac aag gag      1777
Phe Thr Gly Asp Met Asp Glu Tyr Cys Asn Gln Tyr Val Asp Lys Glu
                580                 585                 590

gcc tta ttg tac ctc ata tta gca aat gaa gta tta cat gct ctt cat      1825
Ala Leu Leu Tyr Leu Ile Leu Ala Asn Glu Val Leu His Ala Leu His
            595                 600                 605

cct aat gtg atc acg att gct gag gat gca act ctg tat cct gga ctc      1873
Pro Asn Val Ile Thr Ile Ala Glu Asp Ala Thr Leu Tyr Pro Gly Leu
        610                 615                 620

tgc gat cca aca tct caa ggt gga ctg ggc ttt gat tat ttt gcc aat      1921
Cys Asp Pro Thr Ser Gln Gly Gly Leu Gly Phe Asp Tyr Phe Ala Asn
    625                 630                 635

ctt tct gcc tca gag atg tgg ctt gca tta ctt gaa aat act cct gat      1969
Leu Ser Ala Ser Glu Met Trp Leu Ala Leu Leu Glu Asn Thr Pro Asp
640                 645                 650                 655

cat gaa tgg tgc atg agt aag att gtt agc aca tta gtg ggc gat aga      2017
His Glu Trp Cys Met Ser Lys Ile Val Ser Thr Leu Val Gly Asp Arg
                660                 665                 670

caa aat act gat aaa atg ctt ttg tat gca gaa aat cac aac cag tcc      2065
Gln Asn Thr Asp Lys Met Leu Leu Tyr Ala Glu Asn His Asn Gln Ser
            675                 680                 685

att tct gga ggt cgt tcc ttc gca gaa ata ctg att ggt aac tcc ttg      2113
Ile Ser Gly Gly Arg Ser Phe Ala Glu Ile Leu Ile Gly Asn Ser Leu
        690                 695                 700

ggg aaa tcc tcc ata tca caa gag tca tta ctt aga ggc tgc tcg tta      2161
Gly Lys Ser Ser Ile Ser Gln Glu Ser Leu Leu Arg Gly Cys Ser Leu
    705                 710                 715

cac aag atg atc aga tta att aca tct aca att ggt ggt cat gca tac      2209
His Lys Met Ile Arg Leu Ile Thr Ser Thr Ile Gly Gly His Ala Tyr
720                 725                 730                 735

ctc aac ttc atg ggc aat gaa ttt ggt cac cca aag aga gta gag ttt      2257
Leu Asn Phe Met Gly Asn Glu Phe Gly His Pro Lys Arg Val Glu Phe
                740                 745                 750

cca atg tca agc aac aat ttc tcc ttt tca ctg gct aac cgt cgc tgg      2305
Pro Met Ser Ser Asn Asn Phe Ser Phe Ser Leu Ala Asn Arg Arg Trp
            755                 760                 765

gat cta ttg gaa gat gtt gta cat tat caa ttg ttc tca ttt gat aag      2353
Asp Leu Leu Glu Asp Val Val His Tyr Gln Leu Phe Ser Phe Asp Lys
        770                 775                 780

ggt atg atg gac ttg gat aaa aat ggg aga att ttg tcc aga ggt ctt      2401
Gly Met Met Asp Leu Asp Lys Asn Gly Arg Ile Leu Ser Arg Gly Leu
    785                 790                 795

gcc aac att cac cat gtc aat gat act acc atg gtg att tct tac ttg      2449
Ala Asn Ile His His Val Asn Asp Thr Thr Met Val Ile Ser Tyr Leu
800                 805                 810                 815

aga ggt ccc aat ctc ttt gtg ttc aac ttt cat cct gtc aat tca tat      2497
Arg Gly Pro Asn Leu Phe Val Phe Asn Phe His Pro Val Asn Ser Tyr
                820                 825                 830

gaa aga tac att ata ggt gtg gaa gaa gct gga gag tat caa gtc aca      2545
Glu Arg Tyr Ile Ile Gly Val Glu Glu Ala Gly Glu Tyr Gln Val Thr
            835                 840                 845
```

```
tta aat aca gat gaa aac aag tat ggt ggt aga gga cta ctt ggc cat      2593
Leu Asn Thr Asp Glu Asn Lys Tyr Gly Gly Arg Gly Leu Leu Gly His
        850                 855                 860

gat cag aat att caa aga acc att agt aga aga gct gat gga atg aga      2641
Asp Gln Asn Ile Gln Arg Thr Ile Ser Arg Arg Ala Asp Gly Met Arg
    865                 870                 875

ttt tgc ttg gaa gtg cct ctg cca agt aga agt gct cag gtc tac aag      2689
Phe Cys Leu Glu Val Pro Leu Pro Ser Arg Ser Ala Gln Val Tyr Lys
880                 885                 890                 895

ttg acc cga att cta aga gca tgatcactct agtaatcaaa gtgcctcata        2740
Leu Thr Arg Ile Leu Arg Ala
                900

tgatgacaca aaaggaaagg ttctacattg cccttacact gatcaatatt gacacctttc   2800

cgaggtgagt ttctgtgatt cttgagcaga ctgttggcta gtcaattatc atgaactttt   2860

gccttcagca tccggatagt cgcttctcct gtgcaatgag ggcatggacg aatttttttt   2920

tggcttgtca tgggggtcat aagcatccgc cagattaaga tttcacaggc ctcgagtaaa   2980

accatcactt actttaagga tacacaaaca caccaacggg gtgcaggctc tgataccttc   3040

taaagtg                                                               3047
```

<210> 14

<211> 902

<212> PRT

<213> Solanum tuberosum

<400> 14

```
Met Leu Ser Leu Ser Asp Ser Ile Arg Ile Ser Ser Pro Leu Ser Asp
1               5                   10                  15

Ser Arg Leu Ser Phe Leu Ser Gln Thr Gly Ser Arg Thr Ser Arg Gln
            20                  25                  30

Leu Lys Phe Val Arg Ser Arg Arg Ala Arg Val Ser Arg Cys Arg Cys
        35                  40                  45

Ser Ala Thr Glu Gln Pro Pro Pro Gln Arg Arg Lys Gln Arg Pro Glu
    50                  55                  60

Lys Tyr Lys Gln Ser Glu Glu Gly Lys Gly Ile Asp Pro Val Gly Phe
65                  70                  75                  80

Leu Ser Lys Tyr Gly Ile Thr His Lys Ala Phe Ala Gln Phe Leu Arg
                85                  90                  95

Glu Arg Tyr Lys Ser Leu Lys Asp Leu Lys Asp Glu Ile Leu Thr Arg
            100                 105                 110
```

His Phe Ser Leu Lys Glu Met Ser Thr Gly Tyr Glu Leu Met Gly Met
115 120 125

His Arg Asn Ile Gln His Arg Val Asp Phe Leu Glu Trp Ala Pro Gly
130 135 140

Ala Arg Tyr Cys Ala Leu Ile Gly Asp Phe Asn Gly Trp Ser Thr Thr
145 150 155 160

Gly Asn Cys Ala Arg Glu Gly His Phe Gly His Asp Asp Tyr Gly Tyr
165 170 175

Trp Phe Ile Ile Leu Glu Asp Lys Leu Arg Glu Gly Glu Glu Pro Asp
180 185 190

Lys Leu Tyr Phe Gln Gln Tyr Asn Tyr Ala Glu Asp Tyr Gly Lys Gly
195 200 205

Asp Thr Gly Ile Thr Val Glu Glu Ile Phe Lys Lys Ala Asn Asp Glu
210 215 220

Tyr Trp Glu Pro Gly Glu Asp Arg Phe Ile Lys Ser Arg Tyr Glu Val
225 230 235 240

Ala Ala Lys Leu Tyr Glu Glu Met Phe Gly Pro Asn Gly Pro Gln Thr
245 250 255

Glu Glu Glu Leu Glu Ala Met Pro Asp Ala Ala Thr Arg Tyr Lys Thr
260 265 270

Trp Lys Glu Gln Gln Lys Glu Asp Pro Ala Ser Asn Leu Pro Ser Tyr
275 280 285

Asp Val Val Asp Ser Gly Lys Glu Tyr Asp Ile Tyr Asn Ile Ile Gly
290 295 300

Asp Pro Glu Ser Phe Lys Lys Phe Arg Met Lys Gln Pro Pro Ile Ala
305 310 315 320

Tyr Trp Leu Glu Thr Lys Lys Gly Arg Lys Gly Trp Leu Gln Lys Tyr
325 330 335

Met Pro Ala Leu Pro His Gly Ser Lys Tyr Arg Val Tyr Phe Asn Thr
340 345 350

Pro Asn Gly Pro Leu Glu Arg Val Pro Ala Trp Ala Asn Phe Val Ile
355 360 365

Pro Asp Ala Gly Gly Met Ala Leu Ala Val His Trp Glu Pro Pro Pro
370 375 380

```
Glu Tyr Ala Tyr Lys Trp Lys His Lys Leu Pro Val Lys Pro Lys Ser
385                 390                 395                 400

Leu Arg Ile Tyr Glu Cys His Val Gly Ile Ser Gly Gln Glu Pro Lys
                405                 410                 415

Val Ser Ser Phe Asn Asp Phe Ile Ser Lys Val Leu Pro His Val Lys
            420                 425                 430

Glu Ala Gly Tyr Asn Ala Ile Gln Ile Ile Gly Val Val Glu His Lys
        435                 440                 445

Asp Tyr Phe Thr Val Gly Tyr Arg Val Thr Asn Phe Tyr Ala Val Ser
    450                 455                 460

Ser Arg Tyr Gly Thr Pro Asp Asp Phe Lys Arg Leu Val Asp Glu Ala
465                 470                 475                 480

His Gly Leu Gly Leu Leu Val Phe Leu Glu Ile Val His Ser Tyr Ala
                485                 490                 495

Ala Ala Asp Glu Met Val Gly Leu Ser Leu Phe Asp Gly Ala Asn Asp
            500                 505                 510

Cys Tyr Phe His Thr Gly Lys Arg Gly His His Lys Phe Trp Gly Thr
        515                 520                 525

Arg Met Phe Lys Tyr Gly Asp Leu Asp Val Leu His Phe Leu Leu Ser
    530                 535                 540

Asn Leu Asn Trp Trp Val Glu Glu Tyr His Val Asp Gly Phe His Phe
545                 550                 555                 560

His Ser Leu Ser Ser Met Leu Tyr Thr His Asn Gly Phe Ala Ser Phe
                565                 570                 575

Thr Gly Asp Met Asp Glu Tyr Cys Asn Gln Tyr Val Asp Lys Glu Ala
            580                 585                 590

Leu Leu Tyr Leu Ile Leu Ala Asn Glu Val Leu His Ala Leu His Pro
        595                 600                 605

Asn Val Ile Thr Ile Ala Glu Asp Ala Thr Leu Tyr Pro Gly Leu Cys
    610                 615                 620

Asp Pro Thr Ser Gln Gly Gly Leu Gly Phe Asp Tyr Phe Ala Asn Leu
625                 630                 635                 640

Ser Ala Ser Glu Met Trp Leu Ala Leu Leu Glu Asn Thr Pro Asp His
                645                 650                 655
```

Glu Trp Cys Met Ser Lys Ile Val Ser Thr Leu Val Gly Asp Arg Gln
660 665 670

Asn Thr Asp Lys Met Leu Leu Tyr Ala Glu Asn His Asn Gln Ser Ile
675 680 685

Ser Gly Gly Arg Ser Phe Ala Glu Ile Leu Ile Gly Asn Ser Leu Gly
690 695 700

Lys Ser Ser Ile Ser Gln Glu Ser Leu Leu Arg Gly Cys Ser Leu His
705 710 715 720

Lys Met Ile Arg Leu Ile Thr Ser Thr Ile Gly Gly His Ala Tyr Leu
725 730 735

Asn Phe Met Gly Asn Glu Phe Gly His Pro Lys Arg Val Glu Phe Pro
740 745 750

Met Ser Ser Asn Asn Phe Ser Phe Ser Leu Ala Asn Arg Arg Trp Asp
755 760 765

Leu Leu Glu Asp Val Val His Tyr Gln Leu Phe Ser Phe Asp Lys Gly
770 775 780

Met Met Asp Leu Asp Lys Asn Gly Arg Ile Leu Ser Arg Gly Leu Ala
785 790 795 800

Asn Ile His His Val Asn Asp Thr Thr Met Val Ile Ser Tyr Leu Arg
805 810 815

Gly Pro Asn Leu Phe Val Phe Asn Phe His Pro Val Asn Ser Tyr Glu
820 825 830

Arg Tyr Ile Ile Gly Val Glu Glu Ala Gly Glu Tyr Gln Val Thr Leu
835 840 845

Asn Thr Asp Glu Asn Lys Tyr Gly Gly Arg Gly Leu Leu Gly His Asp
850 855 860

Gln Asn Ile Gln Arg Thr Ile Ser Arg Arg Ala Asp Gly Met Arg Phe
865 870 875 880

Cys Leu Glu Val Pro Leu Pro Ser Arg Ser Ala Gln Val Tyr Lys Leu
885 890 895

Thr Arg Ile Leu Arg Ala
900

<210> 15

<211> 30

<212> DNA

<213> synthetic

<400> 15
tcaagtcgac cacaaccagt ccatttctgg 30

<210> 16

<211> 30

<212> DNA

<213> synthetic

<400> 16
tcaaactagt cacaaccagt ccatttctgg 30

<210> 17

<211> 21

<212> DNA

<213> synthetic

<400> 17
cactttagaa ggtatcagag c 21

<210> 18

<211> 22

<212> DNA

<213> synthetic

<400> 18
gtatttctgc gaaggaacga cc 22

<210> 19

<211> 20

<212> DNA

<213> synthetic

<400> 19
aacaatgctc tctctgtcgg 20

<210> 20

<211> 30

<212> DNA

<213> synthetic

<400> 20
gcttgtcgac gggagaattt tgtccagagg 30

<210> 21

<211> 31

<212> DNA

<213> synthetic

<400> 21
gatcgtcgac agcacttcta cttggcagag g 31